(19) Europäisches Patentamt
European Patent Office
Office européen des brevets

(11) EP 1 910 532 B1

(12) # EUROPÄISCHE PATENTSCHRIFT

(45) Veröffentlichungstag und Bekanntmachung des Hinweises auf die Patenterteilung:
**20.07.2011 Patentblatt 2011/29**

(21) Anmeldenummer: **06776589.1**

(22) Anmeldetag: **03.08.2006**

(51) Int Cl.:
***C12N 9/18*** *(2006.01)*

(86) Internationale Anmeldenummer:
**PCT/EP2006/007693**

(87) Internationale Veröffentlichungsnummer:
**WO 2007/017181 (15.02.2007 Gazette 2007/07)**

(54) **VERWENDUNG VON ESTERASEN ZUR SPALTUNG VON KUNSTSTOFFEN**

USE OF ESTERASES FOR SEPARATING PLASTICS

UTILISATION D'ESTERASES POUR LA SEPARATION DE MATIERES SYNTHETIQUES

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **05.08.2005 DE 102005037659**

(43) Veröffentlichungstag der Anmeldung:
**16.04.2008 Patentblatt 2008/16**

(73) Patentinhaber: **Henkel AG & Co. KGaA**
**40589 Düsseldorf (DE)**

(72) Erfinder:
- **MICHELS, Andreas**
**40327 Düsseldorf (DE)**
- **PÜTZ, André**
**40591 Düsseldorf (DE)**
- **MAURER, Karl-Heinz**
**40699 Erkrath (DE)**
- **EGGERT, Thorsten**
**45259 Essen (DE)**
- **JÄGER, Karl-Erich**
**45479 Mülheim (DE)**

(56) Entgegenhaltungen:
**EP-A2- 0 551 750 WO-A-00/66696**
**WO-A-2004/016669**

- **ZOCK J ET AL: "The Bacillus subtilis pnbA gene encoding p-nitrobenzyl esterase: cloning, sequence and high-level expression in Escherichia coli" GENE, ELSEVIER, AMSTERDAM, NL, Bd. 151, Nr. 1, 30. Dezember 1994 (1994-12-30), Seiten 37-43, XP004042611 ISSN: 0378-1119**
- **SPILLER BEN ET AL: "A structural view of evolutionary divergence" PROCEEDINGS OF THE NATIONAL ACADEMY OF SCIENCES OF THE UNITED STATES OF AMERICA, Bd. 96, Nr. 22, 26. Oktober 1999 (1999-10-26), Seiten 12305-12310, XP002421542 ISSN: 0027-8424 -& DATABASE PDB 21. Februar 2000 (2000-02-21), XP002421545 Database accession no. 1C7J -& DATABASE PDB 12. Juli 1999 (1999-07-12), XP002421546 Database accession no. 1QE3**
- **VEITH B ET AL: "THE COMPLETE GENOME SEQUENCE OF BACILLUS LICHENIFORMIS DSM13, AN ORGANISM WITH GREAT INDUSTRIAL POTENTIAL" JOURNAL OF MOLECULAR MICROBIOLOGY AND BIOTECHNOLOGY, HORIZON SCIENTIFIC PRESS, WYMONDHAM,, GB, Bd. 7, Nr. 4, 2004, Seiten 204-211, XP009047713 ISSN: 1464-1801 -& DATABASE EMBL 25. Januar 2005 (2005-01-25), XP002432701 Database accession no. Q65MY7**
- **FABRET C. ET AL: 'Integrated mapping and sequencing of a 115 kb DNA fragment from Bacillus subtilis: Sequence analysis of a 21 kb segment containing the sigL locus', 1996, MICROBIOLOGY (READING), VOL. 142, NR. 11, PAGE(S) 3089-3096, ISSN 1350-0872**
- **DATABASE NCBI [Online] 18 April 2005 Database accession no. Z71928**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

SEQUENCE LISTING

<110> Henkel KGaA

<1.20> H 06819 PCT

<130> Esterase

<150> DE 10 2005 037 659.2 <151> 05.08.2005

<160> 27

<170> PatentIn version 3.1

<210> 1
<211> 489
<212> PRT
<213> Bacillus subtilis

<400> 1

Met Thr His Gln Ile Val Thr Thr Gln Tyr Gly Lys Val Lys Gly Thr
1               5                   10                  15

Thr Glu Asn Gly Val His Lys Trp Lys Gly Ile Pro Tyr Ala Lys Pro
            20                  25                  30

Pro Val Gly Gln Trp Arg Phe Lys Ala Pro Glu Pro Pro Glu Val Trp
        35                  40                  45

Glu Asp Val Leu Asp Ala Thr Ala Tyr Gly Pro Ile Cys Pro Gln Pro
    50                  55                  60

Ser Asp Leu Leu Ser Leu Ser Tyr Thr Glu Leu Pro Arg Gln Ser Glu
65                  70                  75                  80

Asp Cys Leu Tyr Val Asn Val Phe Ala Pro Asp Thr Pro Ser Gln Asn
                85                  90                  95

Leu Pro Val Met Val Trp Ile His Gly Gly Ala Phe Tyr Leu Gly Ala
            100                 105                 110

Gly Ser Glu Pro Leu Tyr Asp Gly Ser Lys Leu Ala Ala Gln Gly Glu
        115                 120                 125

Val Ile Val Val Thr Leu Asn Tyr Arg Leu Gly Pro Phe Gly Phe Leu
    130                 135                 140

His Leu Ser Ser Phe Asp Glu Ala Tyr Ser Asp Asn Leu Gly Leu Leu
145                 150                 155                 160

Asp Gln Ala Ala Ala Leu Lys Trp Val Arg Glu Asn Ile Ser Ala Phe

165 170 175

Gly Gly Asp Pro Asp Asn Val Thr Val Phe Gly Glu Ser Ala Gly Gly
180 185 190

Met Ser Ile Ala Ala Leu Leu Ala Met Pro Ala Ala Lys Gly Leu Phe
195 200 205

Gln Lys Ala Ile Met Glu Ser Gly Ala Ser Arg Thr Met Thr Lys Glu
210 215 220

Gln Ala Ala Ser Thr Ser Ala Ala Phe Leu Gln Val Leu Gly Ile Asn
225 230 235 240

Glu Gly Gln Leu Asp Lys Leu His Thr Val Ser Ala Glu Asp Leu Leu
245 250 255

Lys Ala Ala Asp Gln Leu Arg Ile Ala Glu Lys Glu Asn Ile Phe Gln
260 265 270

Leu Phe Phe Gln Pro Ala Leu Asp Pro Lys Thr Leu Pro Ala Glu Pro
275 280 285

Glu Lys Ala Ile Ser Glu Gly Ala Ala Ser Gly Ile Pro Leu Leu Ile
290 295 300

Gly Thr Thr Arg Asp Glu Gly Tyr Leu Phe Phe Thr Pro Asp Ser Asp
305 310 315 320

Val His Ser Gln Glu Thr Leu Asp Ala Ala Leu Glu Tyr Leu Leu Gly
325 330 335

Lys Pro Leu Ala Glu Lys Ala Ala Asp Leu Tyr Pro Arg Ser Leu Glu
340 345 350

Ser Gln Ile His Met Met Thr Asp Leu Leu Phe Trp Arg Pro Ala Val
355 360 365

Ala Tyr Ala Ser Ala Gln Ser His Tyr Ala Pro Val Trp Met Tyr Arg
370 375 380

Phe Asp Trp His Pro Lys Lys Pro Pro Tyr Asn Lys Ala Phe His Ala
385 390 395 400

Leu Glu Leu Pro Phe Val Phe Gly Asn Leu Asp Gly Leu Glu Arg Met
405 410 415

Ala Lys Ala Glu Ile Thr Asp Glu Val Lys Gln Leu Ser His Thr Ile
420 425 430

Gln Ser Ala Trp Ile Thr Phe Ala Lys Thr Gly Asn Pro Ser Thr Glu
435 440 445

Ala Val Asn Trp Pro Thr Tyr His Glu Glu Thr Arg Glu Thr Leu Ile
450 455 460

Leu Asp Ser Glu Ile Thr Ile Glu Asn Asp Pro Glu Ser Glu Lys Arg
465 470 475 480

Gln Lys Leu Phe Pro Ser Lys Gly Glu
485

<210> 2
<211> 489
<212> PRT
<213> Bacillus licheniformis

<400> 2

Met Thr His Gln Ile Val Thr Thr Gln Tyr Gly Lys Val Lys Gly Thr
1 5 10 15

Thr Glu Asn Gly Val His Lys Trp Lys Gly Ile Pro Tyr Ala Lys Pro
20 25 30

Pro Val Gly Gln Trp Arg Phe Lys Ala Pro Glu Pro Pro Glu Val Trp
35 40 45

Glu Asp Val Leu Asp Ala Thr Ala Tyr Gly Pro Ile Cys Pro Gln Pro
50 55 60

Ser Asp Leu Leu Ser Leu Ser Tyr Thr Glu Leu Pro Arg Gln Ser Glu
65 70 75 80

Asp Cys Leu Tyr Val Asn Val Phe Ala Pro Asp Thr Pro Ser Gln Asn
85 90 95

Leu Pro Val Met Val Trp Ile His Gly Gly Ala Phe Tyr Leu Gly Ala
100 105 110

Gly Ser Glu Pro Leu Tyr Asp Gly Ser Lys Leu Ala Ala Gln Gly Glu
115 120 125

Val Ile Val Val Thr Leu Asn Tyr Arg Leu Gly Pro Phe Gly Phe Leu
130 135 140

His Leu Ser Ser Phe Asp Glu Ala Tyr Ser Asp Asn Leu Gly Leu Leu
145 150 155 160

Asp Gln Ala Ala Ala Leu Lys Trp Val Arg Glu Asn Ile Ser Ala Phe
165 170 175

Gly Gly Asp Pro Asp Asn Val Thr Val Phe Gly Glu Ser Ala Gly Gly
180 185 190

Met Ser Ile Ala Ala Leu Leu Ala Met Pro Ala Ala Lys Gly Leu Phe
195 200 205

Gln Lys Ala Ile Met Glu Ser Gly Ala Ser Arg Thr Met Thr Lys Glu
210 215 220

Gln Ala Ala Ser Thr Ser Ala Ala Phe Leu Gln Val Leu Gly Ile Asn
225 230 235 240

Glu Gly Gln Leu Asp Lys Leu His Thr Val Ser Ala Glu Asp Leu Leu
245 250 255

Lys Ala Ala Asp Gln Leu Arg Ile Ala Glu Lys Glu Asn Ile Phe Gln
260 265 270

Leu Phe Phe Gln Pro Ala Leu Asp Pro Lys Thr Leu Pro Ala Glu Pro
275 280 285

Glu Lys Ala Ile Ser Glu Gly Ala Ala Ser Gly Ile Pro Leu Leu Ile
290 295 300

Gly Thr Thr Arg Asp Glu Gly Tyr Leu Phe Phe Thr Pro Asp Ser Asp
305 310 315 320

Val His Ser Gln Glu Thr Leu Asp Ala Ala Leu Glu Tyr Leu Leu Gly
325 330 335

Lys Pro Leu Ala Glu Lys Ala Ala Asp Leu Tyr Pro Arg Ser Leu Glu
340 345 350

Ser Gln Ile His Met Met Thr Asp Leu Leu Phe Trp Arg Pro Ala Val
355 360 365

Ala Tyr Ala Ser Ala Gln Ser His Tyr Ala Pro Val Trp Met Tyr Arg
370 375 380

Phe Asp Trp His Pro Lys Lys Pro Pro Tyr Asn Lys Ala Phe His Ala
385 390 395 400

```
Leu Glu Leu Pro Phe Val Phe Gly Asn Leu Asp Gly Leu Glu Arg Met
                405                 410                 415

Ala Lys Ala Glu Ile Thr Asp Glu Val Lys Gln Leu Ser His Thr Ile
            420                 425                 430

Gln Ser Ala Trp Ile Thr Phe Ala Lys Thr Gly Asn Pro Ser Thr Glu
        435                 440                 445

Ala Val Asn Trp Pro Thr Tyr His Glu Glu Thr Arg Glu Thr Leu Ile
    450                 455                 460

Leu Asp Ser Glu Ile Thr Ile Glu Asn Asp Pro Glu Ser Glu Lys Arg
465                 470                 475                 480

Gln Lys Leu Phe Pro Ser Lys Gly Glu
                485
```

<210> 3
<211> 1470
<212> DNA
<213> Bacillus subtilis

<400> 3

```
atgactcatc aaatagtaac gactcaatac ggcaaagtaa aaggcacaac ggaaaacggc     60
gtacataagt ggaaaggcat cccctatgcc aagccgcctg tcggacaatg gcgttttaaa    120
gcacctgagc cgcctgaagt gtgggaagat gtccttgatg ccacagcgta cggccctatt    180
tgcccgcagc cgtctgattt gctctcactg tcgtatacag agctgccccg ccagtccgag    240
gattgcttgt atgtcaatgt atttgcgcct gacaccccaa gtcaaaatct tcctgtcatg    300
gtgtggattc acggaggcgc tttttatctt ggagcgggca gtgagccatt gtatgacgga    360
tcaaaacttg cggcacaggg agaagtcatt gtcgttacat tgaactatcg gctggggccg    420
tttggctttt tgcacttgtc ttcgtttgat gaggcgtatt ccgataacct tgggctttta    480
gaccaagccg ccgcactgaa atgggtgcgg gagaatattt cagcgtttgg cggtgatccc    540
gataacgtaa cagtatttgg agaatccgcc ggcgggatga gcattgccgc gcttctcgct    600
atgcctgcgg caaaaggcct gttccagaaa gcaatcatgg aaagcggcgc ttctcgaacg    660
atgacgaaag aacaagcggc gagcacctcg gcagcctttt tacaggtcct tgggattaac    720
gagggccaat tggataaatt gcatacggtt tctgcagaag atttgctaaa agcggctgat    780
cagcttcgga ttgcagaaaa agaaaatatc tttcagctgt tcttccagcc cgcccttgat    840
ccaaaaacgc tgcctgctga accagaaaaa gcgatctcag aaggggctgc ttccggcatt    900
cctctattga ttggaacaac ccgtgatgaa ggatatttat ttttcacccc ggattcagac    960

gttcattctc aggaaacgct tgatgcagca ctcgagtatt tactagggaa gccgctggca   1020
gagaaagctg ccgatttgta tccgcgttct ctggaaagcc aaattcatat gatgactgat   1080
ttattatttt ggcgccctgc cgtcgcctat gcatccgcac agtctcatta cgcccctgtc   1140
tggatgtaca ggttcgattg gcacccgaag aagccgccgt acaataaagc gtttcacgca   1200
ttagagcttc cttttgtctt tggaaatctg gacggattgg aacgaatggc aaaagcggag   1260
attacggatg aggtgaaaca gctttctcac acgatacaat cagcgtggat cacgttcgct   1320
aaaacaggaa acccaagcac cgaagctgtg aattggccga cgtatcatga agaaacgaga   1380
gagacgctga ttttagattc agagattacg atcgaaaacg atcccgaatc tgaaaaaagg   1440
cagaagctat tcccttcaaa aggagaataa                                     1470
```

<210> 4
<211> 1470
<212> DNA
<213> Bacillus licheniformis

<400> 4

```
atgtctcata aaacagtaac aactcaatac ggcaaagtaa aaggcacaac agaaaacggc     60
gtacataaat ggaaaggcat cccctatgcc aaacctcctg tcgggccatt gcgttttaaa    120
gcaccggaac ctcctgaagc gtgggagaac gaactggacg caacagcata cggctctatt    180
tgcccgcagc cgtctgattt gctgtcactt tcgtatactg agctgccccg ccagtctgag    240
gattgcttgt atatcaatgt atttgcgcct gatactccaa gtcaaaacct gcctgtcatg    300
gtatggattc acggcggcgc tttttatctt ggagcgggca gtgagccatt atatgatggg    360
tcaagacttg cggcgcaggg agaagtcatt gtcgttacac tgaattatcg tctggggccg    420
tttggatttt tacatttgtc ttcgtttgaa gagacgtatt ccgataacct tgggcttttg    480
gaccaagccg ccgcactgaa atgggtgcga gacaatatct cagcatttgg cggtgatccg    540
gataacgtaa cagtatttgg agaatcagca ggcggcatga gcattgccgc gctgctcgca    600
atgcctgcgg caaaaggcct gttccagaaa gcaatcatgg aaagtggcgc ttccagaacg    660
atgacaaaag aaaaagcggc tagcaccgcg gcagcctttt tagaggtcct tgggattgac    720
gagagccaat tggacaggtt gcatactgta tctgcggaag atttgcttaa agcggccgat    780
cagcttcgga aagcagaaaa tgaaaatctc tttcagctgt tcttccagcc cgcccttgat    840
ccgaaaacgc tgcctgctga accagaaaaa gcgatcgcag agggtgctgc tgccggcatt    900
ccgctgttaa tcggaacaaa ccgcgatgaa ggatatttat ttttcacccc ggactcagac    960
gttcattctc aggaaacgtt tgatgccgcg cttgtgtatt tattagggca gccgctggca   1020
gagaaagccg ccgatctgta tccgcgttcg ctggaaagcc aaattcatat gatgactgat   1080
ttgttatttt ggcgcccggc cgtcgcctgt gcctccgcac agtcccatta cgcgcctgtc   1140
tggatgtacc gattcgattg gcactctgat aagccgccgt ataataaagc gtttcacgca   1200
ttagagcttc cttttgtttt cggaaatctg gacgggttag aacggatggc aaaagcagag   1260
attacggatg aagtgaaaca gctctctcac accatacaat cagcatggat cacgttcgcc   1320
aaaacaggga acccaagcac tgaagatgta aaatggccgg cgtatcatga ggaaacaaga   1380
gagacgctga ttttaaattc agagattgcg attgaaaacg accctgaagc tgaaaaaagg   1440
cagaaactat tcccttcaca aggagaataa                                     1470
```

<210> 5
<211> 518
<212> PRT
<213> Bacillus subtilis

<400> 5

```
Met Met Arg Lys Lys Ser Phe Trp Leu Gly Met Leu Thr Ala Phe Met
1               5                   10                  15

Leu Val Phe Thr Met Ala Phe Ser Asp Ser Ala Ser Ala Met Thr His
            20                  25                  30

Gln Ile Val Thr Thr Gln Tyr Gly Lys Val Lys Gly Thr Thr Glu Asn
        35                  40                  45

Gly Val His Lys Trp Lys Gly Ile Pro Tyr Ala Lys Pro Pro Val Gly
    50                  55                  60

Gln Trp Arg Phe Lys Ala Pro Glu Pro Pro Glu Val Trp Glu Asp Val
65                  70                  75                  80

Leu Asp Ala Thr Ala Tyr Gly Pro Ile Cys Pro Gln Pro Ser Asp Leu
                85                  90                  95

Leu Ser Leu Ser Tyr Thr Glu Leu Pro Arg Gln Ser Glu Asp Cys Leu
            100                 105                 110

Tyr Val Asn Val Phe Ala Pro Asp Thr Pro Ser Gln Asn Leu Pro Val
        115                 120                 125

Met Val Trp Ile His Gly Gly Ala Phe Tyr Leu Gly Ala Gly Ser Glu
    130                 135                 140

Pro Leu Tyr Asp Gly Ser Lys Leu Ala Ala Gln Gly Glu Val Ile Val
145                 150                 155                 160

Val Thr Leu Asn Tyr Arg Leu Gly Pro Phe Gly Phe Leu His Leu Ser
                165                 170                 175
```

Ser Phe Asp Glu Ala Tyr Ser Asp Asn Leu Gly Leu Leu Asp Gln Ala
180 185 190

Ala Ala Leu Lys Trp Val Arg Glu Asn Ile Ser Ala Phe Gly Gly Asp
195 200 205

Pro Asp Asn Val Thr Val Phe Gly Glu Ser Ala Gly Gly Met Ser Ile
210 215 220

Ala Ala Leu Leu Ala Met Pro Ala Ala Lys Gly Leu Phe Gln Lys Ala
225 230 235 240

Ile Met Glu Ser Gly Ala Ser Arg Thr Met Thr Lys Glu Gln Ala Ala
245 250 255

Ser Thr Ser Ala Ala Phe Leu Gln Val Leu Gly Ile Asn Glu Gly Gln
260 265 270

Leu Asp Lys Leu His Thr Val Ser Ala Glu Asp Leu Leu Lys Ala Ala
275 280 285

Asp Gln Leu Arg Ile Ala Glu Lys Glu Asn Ile Phe Gln Leu Phe Phe
290 295 300

Gln Pro Ala Leu Asp Pro Lys Thr Leu Pro Ala Glu Pro Glu Lys Ala
305 310 315 320

Ile Ser Glu Gly Ala Ala Ser Gly Ile Pro Leu Leu Ile Gly Thr Thr
325 330 335

Arg Asp Glu Gly Tyr Leu Phe Phe Thr Pro Asp Ser Asp Val His Ser
340 345 350

Gln Glu Thr Leu Asp Ala Ala Leu Glu Tyr Leu Leu Gly Lys Pro Leu
355 360 365

Ala Glu Lys Ala Ala Asp Leu Tyr Pro Arg Ser Leu Glu Ser Gln Ile
370 375 380

His Met Met Thr Asp Leu Leu Phe Trp Arg Pro Ala Val Ala Tyr Ala
385 390 395 400

Ser Ala Gln Ser His Tyr Ala Pro Val Trp Met Tyr Arg Phe Asp Trp
405 410 415

His Pro Lys Lys Pro Pro Tyr Asn Lys Ala Phe His Ala Leu Glu Leu
420 425 430

```
Pro Phe Val Phe Gly Asn Leu Asp Gly Leu Glu Arg Met Ala Lys Ala
        435                     440                     445

Glu Ile Thr Asp Glu Val Lys Gln Leu Ser His Thr Ile Gln Ser Ala
    450                 455                     460

Trp Ile Thr Phe Ala Lys Thr Gly Asn Pro Ser Thr Glu Ala Val Asn
465                 470                 475                     480

Trp Pro Thr Tyr His Glu Glu Thr Arg Glu Thr Leu Ile Leu Asp Ser
                485                 490                     495

Glu Ile Thr Ile Glu Asn Asp Pro Glu Ser Glu Lys Arg Gln Lys Leu
            500                 505                     510

Phe Pro Ser Lys Gly Glu
        515
```

<210> 6
<211> 518
<212> PRT
<213> Bacillus licheniformis

<400> 6

```
Met Met Arg Lys Lys Ser Phe Trp Leu Gly Met Leu Thr Ala Phe Met
1               5                   10                  15

Leu Val Phe Thr Met Ala Phe Ser Asp Ser Ala Ser Ala Met Thr His
            20                  25                  30

Gln Ile Val Thr Thr Gln Tyr Gly Lys Val Lys Gly Thr Thr Glu Asn
        35                  40                  45

Gly Val His Lys Trp Lys Gly Ile Pro Tyr Ala Lys Pro Pro Val Gly
    50                  55                  60

Gln Trp Arg Phe Lys Ala Pro Glu Pro Pro Glu Val Trp Glu Asp Val
65                  70                  75                  80

Leu Asp Ala Thr Ala Tyr Gly Pro Ile Cys Pro Gln Pro Ser Asp Leu
                85                  90                  95

Leu Ser Leu Ser Tyr Thr Glu Leu Pro Arg Gln Ser Glu Asp Cys Leu
            100                 105                 110

Tyr Val Asn Val Phe Ala Pro Asp Thr Pro Ser Gln Asn Leu Pro Val
        115                 120                 125
```

Met Val Trp Ile His Gly Gly Ala Phe Tyr Leu Gly Ala Gly Ser Glu
    130                 135                 140

Pro Leu Tyr Asp Gly Ser Lys Leu Ala Ala Gln Gly Glu Val Ile Val
145                 150                 155                 160

Val Thr Leu Asn Tyr Arg Leu Gly Pro Phe Gly Phe Leu His Leu Ser
                165                 170                 175

Ser Phe Asp Glu Ala Tyr Ser Asp Asn Leu Gly Leu Leu Asp Gln Ala
            180                 185                 190

Ala Ala Leu Lys Trp Val Arg Glu Asn Ile Ser Ala Phe Gly Gly Asp
        195                 200                 205

Pro Asp Asn Val Thr Val Phe Gly Glu Ser Ala Gly Gly Met Ser Ile
    210                 215                 220

Ala Ala Leu Leu Ala Met Pro Ala Ala Lys Gly Leu Phe Gln Lys Ala
225                 230                 235                 240

Ile Met Glu Ser Gly Ala Ser Arg Thr Met Thr Lys Glu Gln Ala Ala
                245                 250                 255

Ser Thr Ser Ala Ala Phe Leu Gln Val Leu Gly Ile Asn Glu Gly Gln
            260                 265                 270

Leu Asp Lys Leu His Thr Val Ser Ala Glu Asp Leu Leu Lys Ala Ala
        275                 280                 285

Asp Gln Leu Arg Ile Ala Glu Lys Glu Asn Ile Phe Gln Leu Phe Phe
    290                 295                 300

Gln Pro Ala Leu Asp Pro Lys Thr Leu Pro Ala Glu Pro Glu Lys Ala
305                 310                 315                 320

Ile Ser Glu Gly Ala Ala Ser Gly Ile Pro Leu Leu Ile Gly Thr Thr
                325                 330                 335

Arg Asp Glu Gly Tyr Leu Phe Phe Thr Pro Asp Ser Asp Val His Ser
            340                 345                 350

Gln Glu Thr Leu Asp Ala Ala Leu Glu Tyr Leu Leu Gly Lys Pro Leu
        355                 360                 365

Ala Glu Lys Ala Ala Asp Leu Tyr Pro Arg Ser Leu Glu Ser Gln Ile

370 375 380

His Met Met Thr Asp Leu Leu Phe Trp Arg Pro Ala Val Ala Tyr Ala
385 390 395 400

Ser Ala Gln Ser His Tyr Ala Pro Val Trp Met Tyr Arg Phe Asp Trp
405 410 415

His Pro Lys Lys Pro Pro Tyr Asn Lys Ala Phe His Ala Leu Glu Leu
420 425 430

Pro Phe Val Phe Gly Asn Leu Asp Gly Leu Glu Arg Met Ala Lys Ala
435 440 445

Glu Ile Thr Asp Glu Val Lys Gln Leu Ser His Thr Ile Gln Ser Ala
450 455 460

Trp Ile Thr Phe Ala Lys Thr Gly Asn Pro Ser Thr Glu Ala Val Asn
465 470 475 480

Trp Pro Thr Tyr His Glu Glu Thr Arg Glu Thr Leu Ile Leu Asp Ser
485 490 495

Glu Ile Thr Ile Glu Asn Asp Pro Glu Ser Glu Lys Arg Gln Lys Leu
500 505 510

Phe Pro Ser Lys Gly Glu
515

<210> 7
<211> 1557
<212> DNA
<213> Bacillus subtilis

<400> 7

```
atgatgagga aaaagagttt ttggcttggg atgctgacgg ccttcatgct cgtgttcacg      60
atggcattca gcgattccgc ttctgctatg actcatcaaa tagtaacgac tcaatacggc     120
aaagtaaaag gcacaacgga aaacggcgta cataagtgga aaggcatccc ctatgccaag     180
ccgcctgtcg gacaatggcg ttttaaagca cctgagccgc ctgaagtgtg ggaagatgtc     240
cttgatgcca cagcgtacgg ccctatttgc ccgcagccgt ctgatttgct ctcactgtcg     300
tatacagagc tgccccgcca gtccgaggat tgcttgtatg tcaatgtatt tgcgcctgac     360
accccaagtc aaaatcttcc tgtcatggtg tggattcacg gaggcgcttt ttatcttgga     420
gcgggcagtg agccattgta tgacggatca aaacttgcgg cacagggaga agtcattgtc     480
gttacattga actatcggct ggggccgttt ggctttttgc acttgtcttc gtttgatgag     540

gcgtattccg ataaccttgg gcttttagac caagccgccg cactgaaatg ggtgcgggag     600
aatatttcag cgtttggcgg tgatcccgat aacgtaacag tatttggaga atccgccggc     660
gggatgagca ttgccgcgct tctcgctatg cctgcggcaa aaggcctgtt ccagaaagca     720
atcatggaaa gcggcgcttc tcgaacgatg acgaaagaac aagcggcgag cacctcggca     780
gcctttttac aggtccttgg gattaacgag ggccaattgg ataaattgca tacggtttct     840
gcagaagatt tgctaaaagc ggctgatcag cttcggattg cagaaaaaga aaatatcttt     900
cagctgttct tccagcccgc ccttgatcca aaaacgctgc ctgctgaacc agaaaaagcg     960
atctcagaag ggctgcttc cggcattcct ctattgattg gaacaacccg tgatgaagga     1020
tatttatttt tcaccccgga ttcagacgtt cattctcagg aaacgcttga tgcagcactc    1080
gagtatttac tagggaagcc gctggcagag aaagctgccg atttgtatcc gcgttctctg    1140
gaaagccaaa ttcatatgat gactgattta ttattttggc gccctgccgt cgcctatgca    1200
tccgcacagt ctcattacgc ccctgtctgg atgtacaggt tcgattggca cccgaagaag    1260
ccgccgtaca ataaagcgtt tcacgcatta gagcttcctt ttgtctttgg aaatctggac    1320
ggattggaac gaatggcaaa agcggagatt acggatgagg tgaaacagct ttctcacacg    1380
atacaatcag cgtggatcac gttcgctaaa acaggaaacc caagcaccga agctgtgaat    1440
tggccgacgt atcatgaaga aacgagagag acgctgattt tagattcaga gattacgatc    1500
gaaaacgatc ccgaatctga aaaaaggcag aagctattcc cttcaaaagg agaataa        1557
```

<210> 8
<211> 1557
<212> DNA
<213> Bacillus licheniformis

<400> 8

```
atgatgagga aaaagagttt ttggcttggg atgctgacgg ccttcatgct cgtgttcacg      60
atggcattca gcgattccgc ttctgctatg tctcataaaa cagtaacaac tcaatacggc     120
aaagtaaaag gcacaacaga aaacggcgta cataaatgga aaggcatccc ctatgccaaa     180
cctcctgtcg ggccattgcg ttttaaagca ccggaacctc ctgaagcgtg ggagaacgaa     240
ctggacgcaa cagcatacgg ctctatttgc ccgcagccgt ctgatttgct gtcactttcg     300
tatactgagc tgccccgcca gtctgaggat tgcttgtata tcaatgtatt tgcgcctgat     360
actccaagtc aaaacctgcc tgtcatggta tggattcacg gcggcgcttt ttatcttgga     420
gcgggcagtg agccattata tgatgggtca agacttgcgg cgcagggaga agtcattgtc     480
gttacactga attatcgtct ggggccgttt ggatttttac atttgtcttc gtttgaagag     540
acgtattccg ataaccttgg gcttttggac caagccgccg cactgaaatg ggtgcgagac     600
aatatctcag catttggcgg tgatccggat aacgtaacag tatttggaga atcagcaggc     660

ggcatgagca ttgccgcgct gctcgcaatg cctgcggcaa aaggcctgtt ccagaaagca     720
atcatggaaa gtggcgcttc cagaacgatg acaaaagaaa aagcggctag caccgcggca     780
gcctttttag aggtccttgg gattgacgag agccaattgg acaggttgca tactgtatct     840
gcggaagatt tgcttaaagc ggccgatcag cttcggaaag cagaaaatga aaatctcttt     900
cagctgttct tccagcccgc ccttgatccg aaaacgctgc ctgctgaacc agaaaaagcg     960
atcgcagagg gtgctgctgc cggcattccg ctgttaatcg gaacaaaccg cgatgaagga    1020
tatttatttt tcaccccgga ctcagacgtt cattctcagg aaacgtttga tgccgcgctt    1080
gtgtatttat tagggcagcc gctggcagag aaagccgccg atctgtatcc gcgttcgctg    1140
gaaagccaaa ttcatatgat gactgatttg ttattttggc gcccggccgt cgcctgtgcc    1200
tccgcacagt cccattacgc gcctgtctgg atgtaccgat tcgattggca ctctgataag    1260
ccgccgtata ataaagcgtt tcacgcatta gagcttcctt ttgttttcgg aaatctggac    1320
gggttagaac ggatggcaaa agcagagatt acggatgaag tgaaacagct ctctcacacc    1380
atacaatcag catggatcac gttcgccaaa acagggaacc caagcactga agatgtaaaa    1440
tggccggcgt atcatgagga aacaagagag acgctgattt taaattcaga gattgcgatt    1500
gaaaacgacc ctgaagctga aaaaaggcag aaactattcc cttcacaagg agaataa       1557
```

<210> 9
<211> 29
<212> PRT

<213> Artificial
<220> SIGNAL
<223>

<400> 9

```
Met Met Arg Lys Lys Ser Phe Trp Leu Gly Met Leu Thr Ala Phe Met
1               5                   10                  15

Leu Val Phe Thr Met Ala Phe Ser Asp Ser Ala Ser Ala
            20                  25
```

<210> 10
<211> 87
<212> DNA
<213> Artificial
<220> sig_peptide
<223>

<400> 10

```
atgatgagga aaaagagttt ttggcttggg atgctgacgg ccttcatgct cgtgttcacg      60

atggcattca gcgattccgc ttctgct                                          87
```

<210> 11
<211> 489
<212> PRT
<213> Bacillus subtilis

<400> 11

Met Thr His Gln Ile Val Thr Thr Gln Tyr Gly Lys Val Lys Gly Thr
1 5 10 15

Thr Glu Asn Gly Val His Lys Trp Lys Gly Ile Pro Tyr Ala Lys Pro
20 25 30

Pro Val Gly Gln Trp Arg Phe Lys Ala Pro Glu Pro Pro Glu Val Trp
35 40 45

Glu Asp Val Leu Asp Ala Thr Ala Tyr Gly Ser Ile Cys Pro Gln Pro
50 55 60

Ser Asp Leu Leu Ser Leu Ser Tyr Thr Glu Leu Pro Arg Gln Ser Glu
65 70 75 80

Asp Cys Leu Tyr Val Asn Val Phe Ala Pro Asp Thr Pro Ser Lys Asn
85 90 95

Leu Pro Val Met Val Trp Ile His Gly Gly Ala Phe Tyr Leu Gly Ala
100 105 110

Gly Ser Glu Pro Leu Tyr Asp Gly Ser Lys Leu Ala Ala Gln Gly Glu
115 120 125

Val Ile Val Val Thr Leu Asn Tyr Arg Leu Gly Pro Phe Gly Phe Leu
130 135 140

His Leu Ser Ser Phe Asn Glu Ala Tyr Ser Asp Asn Leu Gly Leu Leu
145 150 155 160

Asp Gln Ala Ala Ala Leu Lys Trp Val Arg Glu Asn Ile Ser Ala Phe
165 170 175

Gly Gly Asp Pro Asp Asn Val Thr Val Phe Gly Glu Ser Ala Gly Gly
180 185 190

Met Ser Ile Ala Ala Leu Leu Ala Met Pro Ala Ala Lys Gly Leu Phe
195 200 205

Gln Lys Ala Ile Met Glu Ser Gly Ala Ser Arg Thr Met Thr Lys Glu
210 215 220

Gln Ala Ala Ser Thr Ser Ala Ala Phe Leu Gln Val Leu Gly Ile Asn
225 230 235 240

```
Glu Gly Gln Leu Asp Lys Leu His Thr Val Ser Ala Glu Asp Leu Leu
                245                 250                 255

Lys Ala Ala Asp Gln Leu Arg Ile Ala Glu Lys Glu Asn Ile Phe Gln
            260                 265                 270

Leu Phe Phe Gln Pro Ala Leu Asp Pro Lys Thr Leu Pro Glu Glu Pro
        275                 280                 285

Glu Lys Ala Ile Ala Glu Gly Ala Ala Ser Gly Ile Pro Leu Leu Ile
    290                 295                 300

Gly Thr Thr Arg Asp Glu Gly Tyr Leu Phe Phe Thr Pro Asp Ser Asp
305                 310                 315                 320

Val His Ser Gln Glu Thr Leu Asp Ala Ala Leu Glu Tyr Leu Leu Gly
                325                 330                 335

Lys Pro Leu Ala Glu Lys Val Ala Asp Leu Tyr Pro Arg Ser Leu Glu
            340                 345                 350

Ser Gln Ile His Met Met Thr Asp Leu Leu Phe Trp Arg Pro Ala Val
        355                 360                 365

Ala Tyr Ala Ser Ala Gln Ser His Tyr Ala Pro Val Trp Met Tyr Arg
    370                 375                 380

Phe Asp Trp His Pro Lys Lys Pro Pro Tyr Asn Lys Ala Phe His Ala
385                 390                 395                 400

Leu Glu Leu Pro Phe Val Phe Gly Asn Leu Asp Gly Leu Glu Arg Met
                405                 410                 415

Ala Lys Ala Glu Ile Thr Asp Glu Val Lys Gln Leu Ser His Thr Ile
            420                 425                 430

Gln Ser Ala Trp Ile Thr Phe Ala Lys Thr Gly Asn Pro Ser Thr Glu
        435                 440                 445

Ala Val Asn Trp Pro Ala Tyr His Glu Glu Thr Arg Glu Thr Leu Ile
    450                 455                 460

Leu Asp Ser Glu Ile Thr Ile Glu Asn Asp Pro Glu Ser Glu Lys Arg
465                 470                 475                 480

Gln Lys Leu Phe Pro Ser Lys Gly Glu
```

485

<210> 12
<211> 490
<212> PRT
<213> Bacillus licheniformis

<400> 12

Met Tyr Asp Thr Thr Val Glu Thr Arg Phe Gly Lys Leu Lys Gly Arg
1 5 10 15

Ala Glu Asn Gly Val Arg Ile Phe Lys Gly Val Pro Tyr Ala Lys Pro
20 25 30

Pro Val Gly Asp Leu Arg Phe Arg Glu Pro Gln Arg Met Glu Ala Trp
35 40 45

Glu Gly Glu Leu Asp Ala Phe Gln Phe Gly Pro Val Cys Pro Gln Pro
50 55 60

Asp Gly Val Leu Pro Glu Ser Ala Gly Val Gln Lys Ser Glu Asp Cys
65 70 75 80

Leu Tyr Leu Asn Val Tyr Ala Pro Glu Glu Ala Asp Gly Asp Leu Pro
85 90 95

Val Met Val Trp Ile His Gly Gly Ala Phe Tyr Arg Gly Ala Gly Ser
100 105 110

Glu Pro Leu Tyr Asp Gly Thr Gln Leu Ala Lys Gln Gly Lys Val Ile
115 120 125

Val Val Thr Ile Asn Tyr Arg Leu Gly Pro Phe Gly Phe Leu His Leu
130 135 140

Ser Ser Ile Asp Asp Ser Tyr Ser Ser Asn Leu Gly Leu Leu Asp Gln
145 150 155 160

Ile Ala Ala Leu Glu Trp Val Lys Asp Asn Ile Ala Phe Phe Gly Gly
165 170 175

Asp Arg His His Ile Thr Val Phe Gly Glu Ser Ala Gly Ser Met Ser
180 185 190

Ile Ala Ser Leu Leu Ala Met Pro Lys Ala Lys Gly Leu Phe Gln Gln
195 200 205

Ala Ile Met Glu Ser Gly Ala Ser Ala Thr Met Ser Asp Lys Leu Ala

210 215 220

Lys Ala Ala Ala Glu Arg Phe Leu Arg Ile Leu Asp Ile Asp His His
225 230 235 240

His Leu Glu Arg Leu His Asp Val Ser Asp Gln Glu Leu Leu Glu Ala
245 250 255

Ala Asp Gln Leu Arg Thr Leu Met Gly Glu Asn Ile Phe Glu Leu Ile
260 265 270

Phe Leu Pro Ala Leu Asp Glu Lys Thr Leu Pro Leu Lys Pro Glu Val
275 280 285

Ala Val Ala Lys Gly Ala Ala Lys Glu Ile Asn Leu Leu Ile Gly Thr
290 295 300

Asn Arg Asp Glu Gly Val Leu Phe Phe Pro Ser Asp Ser Asp Leu Leu
305 310 315 320

Pro Glu Ser Lys Ile Asn Glu Ile Leu Glu Glu Tyr Met Gly Lys Glu
325 330 335

Ala Ala Glu Ala Ala Ser Ser Leu Tyr Pro Arg Ser Leu Glu Gly His
340 345 350

Val Asp Met Met Thr Asp Leu Ile Phe Trp His Pro Ser Val Val Phe
355 360 365

Ala Ser Ala Gln Ser Arg Tyr Ala Ser Val Phe Met Tyr Arg Phe Asp
370 375 380

Trp His Ala Asp Ser Glu Gln Pro Pro Phe Asn Lys Ala Ala His Gly
385 390 395 400

Leu Glu Ile Pro Phe Val Phe Gly Asn Met Asp Ile Leu Glu Gln Leu
405 410 415

Thr Gly Thr Lys Ala Gly Glu Glu Ala Gln Leu Leu Ala Glu Gln Ile
420 425 430

Gln Ala Ala Trp Val Ser Phe Ala Arg Ser Gly Asn Pro Ser Thr Asp
435 440 445

Asp Val Ser Trp Pro Asp Tyr Asp Glu Asp Ser Arg Lys Thr Leu Ile
450 455 460

Phe Asp Gln Glu Val Ala Val Glu Ser Asp Pro Tyr Ser Asp Lys Arg
465 470 475 480

Lys Met Leu Thr Ala Pro Asn Pro Gln Ile
485 490

<210> 13
<211> 489
<212> PRT
<213> bacillus subtilis

<400> 13

Met Thr His Gln Ile Val Thr Thr Gln Tyr Gly Lys Val Lys Gly Thr
1               5                   10                  15

Thr Glu Asn Gly Val His Lys Trp Lys Gly Ile Pro Tyr Ala Lys Pro
            20                  25                  30

Pro Val Gly Gln Trp Arg Phe Lys Ala Pro Glu Pro Pro Glu Val Trp
        35                  40                  45

Glu Asp Val Leu Asp Ala Thr Ala Tyr Gly Pro Val Cys Pro Gln Pro
    50                  55                  60

Ser Asp Leu Leu Ser Leu Ser Tyr Thr Glu Leu Pro Arg Gln Ser Glu
65                  70                  75                  80

Asp Cys Leu Tyr Val Asn Val Phe Ala Pro Asp Thr Pro Ser Gln Asn
                85                  90                  95

Leu Pro Val Met Val Trp Ile His Gly Gly Ala Phe Tyr Leu Gly Ala
            100                 105                 110

Gly Ser Glu Pro Leu Tyr Asp Gly Ser Lys Leu Ala Ala Gln Gly Glu
        115                 120                 125

Val Ile Val Val Thr Leu Asn Tyr Arg Leu Gly Pro Phe Gly Phe Met
    130                 135                 140

His Leu Ser Ser Phe Asp Glu Ala Tyr Ser Asp Asn Leu Gly Leu Leu
145                 150                 155                 160

Asp Gln Ala Ala Ala Leu Lys Trp Val Arg Glu Asn Ile Ser Ala Phe
                165                 170                 175

Gly Gly Asp Pro Asp Asn Val Thr Val Phe Gly Glu Ser Ala Gly Gly
            180                 185                 190

```
Met Ser Ile Ala Ala Leu Leu Ala Met Pro Ala Ala Lys Gly Leu Phe
        195                 200                 205

Gln Lys Ala Ile Met Glu Ser Gly Ala Ser Arg Thr Met Thr Lys Glu
    210                 215                 220

Gln Ala Ala Ser Thr Ala Ala Ala Phe Leu Gln Val Leu Gly Ile Asn
225                 230                 235                 240

Glu Ser Gln Leu Asp Arg Leu His Thr Val Ala Ala Glu Asp Leu Leu
                245                 250                 255

Lys Ala Ala Asp Gln Leu Arg Ile Ala Glu Lys Glu Asn Ile Phe Gln
            260                 265                 270

Leu Phe Phe Gln Pro Ala Leu Asp Pro Lys Thr Leu Pro Glu Glu Pro
        275                 280                 285

Glu Lys Ser Ile Ala Glu Gly Ala Ala Ser Gly Ile Pro Leu Leu Ile
    290                 295                 300

Gly Thr Thr Arg Asp Glu Gly Tyr Leu Phe Phe Thr Ser Asp Ser Asp
305                 310                 315                 320

Val Arg Ser Gln Glu Thr Leu Asp Ala Ala Leu Glu Tyr Ser Leu Gly
                325                 330                 335

Lys Pro Leu Ala Glu Lys Ala Ala Asp Leu Tyr Pro Arg Ser Leu Glu
            340                 345                 350

Ser Gln Ile His Met Val Thr Asp Leu Leu Phe Trp Arg Pro Ala Val
        355                 360                 365

Ala Phe Ala Ser Ala Gln Ser His Tyr Ala Pro Val Trp Met Tyr Arg
    370                 375                 380

Phe Asp Trp His Pro Glu Lys Pro Pro Tyr Asn Lys Ala Phe His Ala
385                 390                 395                 400

Leu Glu Leu Pro Phe Val Phe Gly Asn Leu Asp Gly Leu Glu Arg Met
                405                 410                 415

Ala Lys Ala Glu Ile Thr Asp Glu Val Lys Gln Leu Ser His Thr Ile
            420                 425                 430

Gln Ser Ala Trp Ile Thr Phe Ala Lys Thr Gly Asn Pro Ser Thr Glu
        435                 440                 445
```

Ala Val Asn Trp Pro Ala Tyr His Glu Glu Thr Arg Glu Thr Val Ile
450 455 460

Leu Asp Ser Glu Ile Thr Ile Glu Asn Asp Pro Glu Ser Glu Lys Arg
465 470 475 480

Gln Lys Leu Phe Pro Ser Lys Gly Glu
485

<210> 14
<211> 489
<212> PRT
<213> Bacillus subtilis

<400> 14

```
Met Thr His Gln Ile Val Thr Thr Gln Tyr Gly Lys Val Lys Gly Thr
1               5                   10                  15

Thr Glu Asn Gly Val His Lys Trp Lys Gly Ile Pro Tyr Ala Lys Pro
            20                  25                  30

Pro Val Gly Gln Trp Arg Phe Lys Ala Pro Glu Pro Pro Glu Val Trp
        35                  40                  45

Glu Asp Val Leu Asp Ala Thr Ala Tyr Gly Pro Ile Cys Pro Gln Pro
    50                  55                  60

Ser Asp Leu Leu Ser Leu Ser Tyr Thr Glu Leu Pro Arg Gln Ser Glu
65                  70                  75                  80

Asp Cys Leu Tyr Val Asn Val Phe Ala Pro Asp Thr Pro Ser Gln Asn
                85                  90                  95

Leu Pro Val Met Val Trp Ile His Gly Gly Ala Phe Tyr Leu Gly Ala
            100                 105                 110

Gly Ser Glu Pro Leu Tyr Asp Gly Ser Lys Leu Ala Ala Gln Gly Glu
        115                 120                 125

Val Ile Val Val Thr Leu Asn Tyr Arg Leu Gly Pro Phe Gly Phe Leu
    130                 135                 140

His Leu Ser Ser Phe Asp Glu Ala Tyr Ser Asp Asn Leu Gly Leu Leu
145                 150                 155                 160

Asp Gln Ala Ala Ala Leu Lys Trp Val Arg Glu Asn Ile Ser Ala Phe
                165                 170                 175
```

```
Gly Gly Asp Pro Asp Asn Val Thr Val Phe Gly Glu Ser Ala Gly Gly
            180                 185                 190

Met Ser Ile Ala Ala Leu Leu Ala Met Pro Ala Ala Lys Gly Leu Phe
        195                 200                 205

Gln Lys Ala Ile Met Glu Ser Gly Ala Ser Arg Thr Met Thr Lys Glu
    210                 215                 220

Gln Ala Ala Ser Thr Ala Ala Ala Phe Leu Gln Val Leu Gly Ile Asn
225                 230                 235                 240

Glu Ser Gln Leu Asp Arg Leu His Thr Val Ala Ala Glu Asp Leu Leu
                245                 250                 255

Lys Ala Ala Asp Gln Leu Arg Ile Ala Glu Lys Glu Asn Ile Phe Gln
            260                 265                 270

Leu Phe Phe Gln Pro Ala Leu Asp Pro Lys Thr Leu Pro Glu Glu Pro
        275                 280                 285

Glu Lys Ser Ile Ala Glu Gly Ala Ala Ser Gly Ile Pro Leu Leu Ile
    290                 295                 300

Gly Thr Thr Arg Asp Glu Gly Tyr Leu Phe Phe Thr Pro Asp Ser Asp
305                 310                 315                 320

Val His Ser Gln Glu Thr Leu Asp Ala Ala Leu Glu Tyr Leu Leu Gly
                325                 330                 335

Lys Pro Leu Ala Glu Lys Ala Ala Asp Leu Tyr Pro Arg Ser Leu Glu
            340                 345                 350

Ser Gln Ile His Met Met Thr Asp Leu Leu Phe Trp Arg Pro Ala Val
        355                 360                 365

Ala Tyr Ala Ser Ala Gln Ser His Tyr Ala Pro Val Trp Met Tyr Arg
    370                 375                 380

Phe Asp Trp His Pro Glu Lys Pro Pro Tyr Asn Lys Ala Phe His Ala
385                 390                 395                 400

Leu Glu Leu Pro Phe Val Phe Gly Asn Leu Asp Gly Leu Glu Arg Met
                405                 410                 415

Ala Lys Ala Glu Ile Thr Asp Glu Val Lys Gln Leu Ser His Thr Ile
            420                 425                 430
```

```
Gln Ser Ala Trp Ile Thr Phe Ala Lys Thr Gly Asn Pro Ser Thr Glu
        435                 440                 445

Ala Val Asn Trp Pro Ala Tyr His Glu Glu Thr Arg Glu Thr Val Ile
    450                 455                 460

Leu Asp Ser Glu Ile Thr Ile Glu Asn Asp Pro Glu Ser Glu Lys Arg
465                 470                 475                 480

Gln Lys Leu Phe Pro Ser Lys Gly Glu
                485
```

<210> 15
<211> 489
<212> PRT
<213> Bacillus subtilis

<400> 15

Met Thr His Gln Ile Val Thr Thr Gln Tyr Gly Lys Val Lys Gly Thr
1 5 10 15

Thr Glu Asn Gly Val His Lys Trp Lys Gly Ile Pro Tyr Ala Lys Pro
20 25 30

Pro Val Gly Gln Trp Arg Phe Lys Ala Pro Glu Pro Pro Glu Val Trp
35 40 45

Glu Asp Val Leu Asp Ala Thr Ala Tyr Gly Ser Ile Cys Pro Gln Pro
50 55 60

Ser Asp Leu Leu Ser Leu Ser Tyr Thr Glu Leu Pro Arg Gln Ser Glu
65 70 75 80

Asp Cys Leu Tyr Val Asn Val Phe Ala Pro Asp Thr Pro Ser Lys Asn
85 90 95

Leu Pro Val Met Val Trp Ile His Gly Gly Ala Phe Tyr Leu Gly Ala
100 105 110

Gly Ser Glu Pro Leu Tyr Asp Gly Ser Lys Leu Ala Ala Gln Gly Glu
115 120 125

Val Ile Val Val Thr Leu Asn Tyr Arg Leu Gly Pro Phe Gly Phe Leu
130 135 140

His Leu Ser Ser Phe Asn Glu Ala Tyr Ser Asp Asn Leu Gly Leu Leu
145 150 155 160

Asp Gln Ala Ala Ala Leu Lys Trp Val Arg Glu Asn Ile Ser Ala Phe
165 170 175

Gly Gly Asp Pro Asp Asn Val Thr Val Phe Gly Glu Ser Ala Gly Gly
180 185 190

Met Ser Ile Ala Ala Leu Leu Ala Met Pro Ala Ala Lys Gly Leu Phe
195 200 205

Gln Lys Ala Ile Met Glu Ser Gly Ala Ser Arg Thr Met Thr Lys Glu
210 215 220

Gln Ala Ala Ser Thr Ser Ala Ala Phe Leu Gln Val Leu Gly Ile Asn
225 230 235 240

Glu Gly Gln Leu Asp Lys Leu His Thr Val Ser Ala Glu Asp Leu Leu
245 250 255

Lys Ala Ala Asp Gln Leu Arg Ile Ala Glu Lys Glu Asn Ile Phe Gln
260 265 270

Leu Phe Phe Gln Pro Ala Leu Asp Pro Lys Thr Leu Pro Glu Glu Pro
275 280 285

Glu Lys Ala Ile Ala Glu Gly Ala Ala Ser Gly Ile Pro Leu Leu Ile
290 295 300

Gly Thr Thr Arg Asp Glu Gly Tyr Leu Phe Phe Thr Pro Asp Ser Asp
305 310 315 320

Val His Ser Gln Glu Thr Leu Asp Ala Ala Leu Glu Tyr Leu Leu Gly
325 330 335

Lys Pro Leu Ala Glu Lys Val Ala Asp Leu Tyr Pro Arg Ser Leu Glu
340 345 350

Ser Gln Ile His Met Met Thr Asp Leu Leu Phe Trp Arg Pro Ala Val
355 360 365

Ala Tyr Ala Ser Ala Gln Ser His Tyr Ala Pro Val Trp Met Tyr Arg
370 375 380

Phe Asp Trp His Pro Lys Lys Pro Pro Tyr Asn Lys Ala Phe His Ala
385 390 395 400

Leu Glu Leu Pro Phe Val Phe Gly Asn Leu Asp Gly Leu Glu Arg Met

405 410 415

Ala Lys Ala Glu Ile Thr Asp Glu Val Lys Gln Leu Ser His Thr Ile
420 425 430

Gln Ser Ala Trp Ile Thr Phe Ala Lys Thr Gly Asn Pro Ser Thr Glu
435 440 445

Ala Val Asn Trp Pro Ala Tyr His Glu Glu Thr Arg Glu Thr Leu Ile
450 455 460

Leu Asp Ser Glu Ile Thr Ile Glu Asn Asp Pro Glu Ser Glu Lys Arg
465 470 475 480

Gln Lys Leu Phe Pro Ser Lys Gly Glu
485

<210> 16
<211> 489
<212> PRT
<213> Bacillus subtilis

<400> 16

```
Met Thr His Gln Ile Val Thr Thr Gln Tyr Gly Lys Val Lys Gly Thr
1               5                   10                  15

Thr Glu Asn Gly Val His Lys Trp Lys Gly Ile Pro Tyr Ala Lys Pro
            20                  25                  30

Pro Val Gly Gln Trp Arg Phe Lys Ala Pro Glu Pro Pro Glu Val Trp
        35                  40                  45

Glu Asp Val Leu Asp Ala Thr Ala Tyr Gly Ser Ile Cys Pro Gln Pro
    50                  55                  60

Ser Asp Leu Leu Ser Leu Ser Tyr Thr Glu Leu Pro Arg Gln Ser Glu
65                  70                  75                  80

Asp Cys Leu Tyr Val Asn Val Phe Ala Pro Asp Thr Pro Ser Lys Asn
                85                  90                  95

Leu Pro Val Met Val Trp Ile His Gly Gly Ala Phe Tyr Leu Gly Ala
            100                 105                 110

Gly Ser Glu Pro Leu Tyr Asp Gly Ser Lys Leu Ala Ala Gln Gly Glu
        115                 120                 125

Val Ile Val Val Thr Leu Asn Tyr Arg Leu Gly Pro Phe Gly Phe Leu
```

130 135 140

His Leu Ser Ser Phe Asn Glu Ala Tyr Ser Asp Asn Leu Gly Leu Leu
145 150 155 160

Asp Gln Ala Ala Ala Leu Lys Trp Val Arg Glu Asn Ile Ser Ala Phe
165 170 175

Gly Gly Asp Pro Asp Asn Val Thr Val Phe Gly Glu Ser Ala Gly Gly
180 185 190

Met Ser Ile Ala Ala Leu Leu Ala Met Pro Ala Ala Lys Gly Leu Phe
195 200 205

Gln Lys Ala Ile Met Glu Ser Gly Ala Ser Arg Thr Met Thr Lys Glu
210 215 220

Gln Ala Ala Ser Thr Ser Ala Ala Phe Leu Gln Val Leu Gly Ile Asn
225 230 235 240

Glu Gly Gln Leu Asp Lys Leu His Thr Val Ser Ala Glu Asp Leu Leu
245 250 255

Lys Ala Ala Asp Gln Leu Arg Ile Ala Glu Lys Glu Asn Ile Phe Gln
260 265 270

Leu Phe Phe Gln Pro Ala Leu Asp Pro Lys Thr Leu Pro Glu Glu Pro
275 280 285

Glu Lys Ala Ile Ala Glu Gly Ala Ala Ser Gly Ile Pro Leu Leu Ile
290 295 300

Gly Thr Thr Arg Asp Glu Gly Tyr Leu Phe Phe Thr Pro Asp Ser Asp
305 310 315 320

Val His Ser Gln Glu Thr Leu Asp Ala Ala Leu Glu Tyr Leu Leu Gly
325 330 335

Lys Pro Leu Ala Glu Lys Val Ala Asp Leu Tyr Pro Arg Ser Leu Glu
340 345 350

Ser Gln Ile His Met Met Thr Asp Leu Leu Phe Trp Arg Pro Ala Val
355 360 365

Ala Tyr Ala Ser Ala Gln Ser His Tyr Ala Pro Val Trp Met Tyr Arg
370 375 380

Phe Asp Trp His Pro Lys Lys Pro Pro Tyr Asn Lys Ala Phe His Ala
385 390 395 400

Leu Glu Leu Pro Phe Val Phe Gly Asn Leu Asp Gly Leu Glu Arg Met
405 410 415

Ala Lys Ala Glu Ile Thr Asp Glu Val Lys Gln Leu Ser His Thr Ile
420 425 430

Gln Ser Ala Trp Ile Thr Phe Ala Lys Thr Gly Asn Pro Ser Thr Glu
435 440 445

Ala Val Asn Trp Pro Ala Tyr His Glu Glu Thr Arg Glu Thr Leu Ile
450 455 460

Leu Asp Ser Glu Ile Thr Ile Glu Asn Asp Pro Glu Ser Glu Lys Arg
465 470 475 480

Gln Lys Leu Phe Pro Ser Lys Gly Glu
485

<210> 17
<211> 489
<212> PRT
<213> Bacillus subtilis

<400> 17

Met Thr His Gln Ile Val Thr Thr Gln Tyr Gly Lys Val Lys Gly Thr
1 5 10 15

Thr Glu Asn Gly Val His Lys Trp Lys Gly Ile Pro Tyr Ala Lys Pro
20 25 30

Pro Val Gly Gln Trp Arg Phe Lys Ala Pro Glu Pro Pro Glu Val Trp
35 40 45

Glu Asp Val Leu Asp Ala Thr Ala Tyr Gly Ser Ile Cys Pro Gln Pro
50 55 60

Ser Asp Leu Leu Ser Leu Ser Tyr Thr Glu Leu Pro Arg Gln Ser Glu
65 70 75 80

Asp Cys Leu Tyr Val Asn Val Phe Ala Pro Asp Thr Pro Ser Lys Asn
85 90 95

Leu Pro Val Met Val Trp Ile His Gly Gly Ala Phe Tyr Leu Gly Ala
100 105 110

```
Gly Ser Glu Pro Leu Tyr Asp Gly Ser Lys Leu Ala Ala Gln Gly Glu
        115                 120                 125

Val Ile Val Val Thr Leu Asn Tyr Arg Leu Gly Pro Phe Gly Phe Leu
    130                 135                 140

His Leu Ser Ser Phe Asn Glu Ala Tyr Ser Asp Asn Leu Gly Leu Leu
145                 150                 155                 160

Asp Gln Ala Ala Ala Leu Lys Trp Val Arg Glu Asn Ile Ser Ala Phe
                165                 170                 175

Gly Gly Asp Pro Asp Asn Val Thr Val Phe Gly Glu Ser Ala Gly Gly
            180                 185                 190

Met Ser Ile Ala Ala Leu Leu Ala Met Pro Ala Ala Lys Gly Leu Phe
        195                 200                 205

Gln Lys Ala Ile Met Glu Ser Gly Ala Ser Arg Thr Met Thr Lys Glu
    210                 215                 220

Gln Ala Ala Ser Thr Ser Ala Ala Phe Leu Gln Val Leu Gly Ile Asn
225                 230                 235                 240

Glu Gly Gln Leu Asp Lys Leu His Thr Val Ser Ala Glu Asp Leu Leu
                245                 250                 255

Lys Ala Ala Asp Gln Leu Arg Ile Ala Glu Lys Glu Asn Ile Phe Gln
            260                 265                 270

Leu Phe Phe Gln Pro Ala Leu Asp Pro Lys Thr Leu Pro Glu Glu Pro
        275                 280                 285

Glu Lys Ala Ile Ala Glu Gly Ala Ala Ser Gly Ile Pro Leu Leu Ile
    290                 295                 300

Gly Thr Thr Arg Asp Glu Gly Tyr Leu Phe Phe Thr Pro Asp Ser Asp
305                 310                 315                 320

Val His Ser Gln Glu Thr Leu Asp Ala Ala Leu Glu Tyr Leu Leu Gly
                325                 330                 335

Lys Pro Leu Ala Glu Lys Val Ala Asp Leu Tyr Pro Arg Ser Leu Glu
            340                 345                 350

Ser Gln Ile His Met Met Thr Asp Leu Leu Phe Trp Arg Pro Ala Val
        355                 360                 365
```

Ala Tyr Ala Ser Ala Gln Ser His Tyr Ala Pro Val Trp Met Tyr Arg
370 375 380

Phe Asp Trp His Pro Lys Lys Pro Pro Tyr Asn Lys Ala Phe His Ala
385 390 395 400

Leu Glu Leu Pro Phe Val Phe Gly Asn Leu Asp Gly Leu Glu Arg Met
405 410 415

Ala Lys Ala Glu Ile Thr Asp Glu Val Lys Gln Leu Ser His Thr Ile
420 425 430

Gln Ser Ala Trp Ile Thr Phe Ala Lys Thr Gly Asn Pro Ser Thr Glu
435 440 445

Ala Val Asn Trp Pro Ala Tyr His Glu Glu Thr Arg Glu Thr Leu Ile
450 455 460

Leu Asp Ser Glu Ile Thr Ile Glu Asn Asp Pro Glu Ser Glu Lys Arg
465 470 475 480

Gln Lys Leu Phe Pro Ser Lys Gly Glu
485

<210> 18
<211> 489
<212> PRT
<213> Bacillus subtilis

<400> 18

```
Met Thr His Gln Ile Val Thr Thr Gln Tyr Gly Lys Val Lys Gly Thr
1               5                   10                  15

Thr Glu Asn Gly Val His Lys Trp Lys Gly Ile Pro Tyr Ala Lys Pro
            20                  25                  30

Pro Val Gly Gln Trp Arg Phe Lys Ala Pro Glu Pro Pro Glu Val Trp
        35                  40                  45

Glu Asp Val Leu Asp Ala Thr Ala Tyr Gly Ser Ile Cys Pro Gln Pro
    50                  55                  60

Ser Asp Leu Leu Ser Leu Ser Tyr Thr Glu Leu Pro Arg Gln Ser Glu
65                  70                  75                  80

Asp Cys Leu Tyr Val Asn Val Phe Ala Pro Asp Thr Pro Ser Lys Asn
                85                  90                  95
```

Leu Pro Val Met Val Trp Ile His Gly Gly Ala Phe Tyr Leu Gly Ala
100 105 110

Gly Ser Glu Pro Leu Tyr Asp Gly Ser Lys Leu Ala Ala Gln Gly Glu
115 120 125

Val Ile Val Val Thr Leu Asn Tyr Arg Leu Gly Pro Phe Gly Phe Leu
130 135 140

His Leu Ser Ser Phe Asn Glu Ala Tyr Ser Asp Asn Leu Gly Leu Leu
145 150 155 160

Asp Gln Ala Ala Ala Leu Lys Trp Val Arg Glu Asn Ile Ser Ala Phe
165 170 175

Gly Gly Asp Pro Asp Asn Val Thr Val Phe Gly Glu Ser Ala Gly Gly
180 185 190

Met Ser Ile Ala Ala Leu Leu Ala Met Pro Ala Ala Lys Gly Leu Phe
195 200 205

Gln Lys Ala Ile Met Glu Ser Gly Ala Ser Arg Thr Met Thr Lys Glu
210 215 220

Gln Ala Ala Ser Thr Ser Ala Ala Phe Leu Gln Val Leu Gly Ile Asn
225 230 235 240

Glu Gly Gln Leu Asp Lys Leu His Thr Val Ser Ala Glu Asp Leu Leu
245 250 255

Lys Ala Ala Asp Gln Leu Arg Ile Ala Glu Lys Glu Asn Ile Phe Gln
260 265 270

Leu Phe Phe Gln Pro Ala Leu Asp Pro Lys Thr Leu Pro Glu Glu Pro
275 280 285

Glu Lys Ala Ile Ala Glu Gly Ala Ala Ser Gly Ile Pro Leu Leu Ile
290 295 300

Gly Thr Thr Arg Asp Glu Gly Tyr Leu Phe Phe Thr Pro Asp Ser Asp
305 310 315 320

Val His Ser Gln Glu Thr Leu Asp Ala Ala Leu Glu Tyr Leu Leu Gly
325 330 335

Lys Pro Leu Ala Glu Lys Val Ala Asp Leu Tyr Pro Arg Ser Leu Glu
340 345 350

```
Ser Gln Ile His Met Met Thr Asp Leu Leu Phe Trp Arg Pro Ala Val
        355                 360                 365

Ala Tyr Ala Ser Ala Gln Ser His Tyr Ala Pro Val Trp Met Tyr Arg
    370                 375                 380

Phe Asp Trp His Pro Lys Lys Pro Pro Tyr Asn Lys Ala Phe His Ala
385                 390                 395                 400

Leu Glu Leu Pro Phe Val Phe Gly Asn Leu Asp Gly Leu Glu Arg Met
                405                 410                 415

Ala Lys Ala Glu Ile Thr Asp Glu Val Lys Gln Leu Ser His Thr Ile
            420                 425                 430

Gln Ser Ala Trp Ile Thr Phe Ala Lys Thr Gly Asn Pro Ser Thr Glu
        435                 440                 445

Ala Val Asn Trp Pro Ala Tyr His Glu Glu Thr Arg Glu Thr Leu Ile
    450                 455                 460

Leu Asp Ser Glu Ile Thr Ile Glu Asn Asp Pro Glu Ser Glu Lys Arg
465                 470                 475                 480

Gln Lys Leu Phe Pro Ser Lys Gly Glu
                485
```

<210> 19
<211> 490
<212> PRT
<213> Bacillus licheniformis

<400> 19

Met Tyr Asp Thr Thr Val Glu Thr Arg Phe Gly Lys Leu Lys Gly Arg
1 5 10 15

Ala Glu Asn Gly Val Arg Ile Phe Lys Gly Val Pro Tyr Ala Lys Pro
20 25 30

Pro Val Gly Asp Leu Arg Phe Arg Glu Pro Gln Arg Met Glu Ala Trp
35 40 45

Glu Gly Glu Leu Asp Ala Phe Gln Phe Gly Pro Val Cys Pro Gln Pro
50 55 60

Asp Gly Val Leu Pro Glu Ser Ala Gly Val Gln Lys Ser Glu Asp Cys
65 70 75 80

Leu Tyr Leu Asn Val Tyr Ala Pro Glu Glu Ala Asp Gly Asp Leu Pro
85 90 95

Val Met Val Trp Ile His Gly Gly Ala Phe Tyr Arg Gly Ala Gly Ser
100 105 110

Glu Pro Leu Tyr Asp Gly Thr Gln Leu Ala Lys Gln Gly Lys Val Ile
115 120 125

Val Val Thr Ile Asn Tyr Arg Leu Gly Pro Phe Gly Phe Leu His Leu
130 135 140

Ser Ser Ile Asp Asp Ser Tyr Ser Ser Asn Leu Gly Leu Leu Asp Gln
145 150 155 160

Ile Ala Ala Leu Glu Trp Val Lys Asp Asn Ile Ala Phe Phe Gly Gly
165 170 175

Asp Arg His His Ile Thr Val Phe Gly Glu Ser Ala Gly Ser Met Ser
180 185 190

Ile Ala Ser Leu Leu Ala Met Pro Lys Ala Lys Gly Leu Phe Gln Gln
195 200 205

Ala Ile Met Glu Ser Gly Ala Ser Ala Thr Met Ser Asp Lys Leu Ala
210 215 220

Lys Ala Ala Ala Glu Arg Phe Leu Arg Ile Leu Asp Ile Asp His His
225 230 235 240

His Leu Glu Arg Leu His Asp Val Ser Asp Gln Glu Leu Leu Glu Ala
245 250 255

Ala Asp Gln Leu Arg Thr Leu Met Gly Glu Asn Ile Phe Glu Leu Ile
260 265 270

Phe Leu Pro Ala Leu Asp Glu Lys Thr Leu Pro Leu Lys Pro Glu Val
275 280 285

Ala Val Ala Lys Gly Ala Ala Lys Glu Ile Asn Leu Leu Ile Gly Thr
290 295 300

Asn Arg Asp Glu Gly Val Leu Phe Phe Pro Ser Asp Ser Asp Leu Leu
305 310 315 320

Pro Glu Ser Lys Ile Asn Glu Ile Leu Glu Glu Tyr Met Gly Lys Glu

325 330 335

Ala Ala Glu Ala Ala Ser Ser Leu Tyr Pro Arg Ser Leu Glu Gly His
340 345 350

Val Asp Met Met Thr Asp Leu Ile Phe Trp His Pro Ser Val Val Phe
355 360 365

Ala Ser Ala Gln Ser Arg Tyr Ala Ser Val Phe Met Tyr Arg Phe Asp
370 375 380

Trp His Ala Asp Ser Glu Gln Pro Pro Phe Asn Lys Ala Ala His Gly
385 390 395 400

Leu Glu Ile Pro Phe Val Phe Gly Asn Met Asp Ile Leu Glu Gln Leu
405 410 415

Thr Gly Thr Lys Ala Gly Glu Glu Ala Gln Leu Leu Ala Glu Gln Ile
420 425 430

Gln Ala Ala Trp Val Ser Phe Ala Arg Ser Gly Asn Pro Ser Thr Asp
435 440 445

Asp Val Ser Trp Pro Asp Tyr Asp Glu Asp Ser Arg Lys Thr Leu Ile
450 455 460

Phe Asp Gln Glu Val Ala Val Glu Ser Asp Pro Tyr Ser Asp Lys Arg
465 470 475 480

Lys Met Leu Thr Ala Pro Asn Pro Gln Ile
485 490

<210> 20
<211> 455
<212> PRT
<213> Staphylococcus epidermidis

<400> 20

```
Met Cys Ser Asn Met Val Gln Val Lys Ile Gly Asn Cys Thr Ile Asn
1               5                   10                  15

Gly Leu His Lys Lys Asn Ile Asp Val Phe Leu Gly Ile Pro Tyr Ala
            20                  25                  30

Lys Ser Phe Asn Lys Ile Ser Arg Phe Gln His Ser Lys Leu Met Glu
        35                  40                  45

Leu Ser Lys Pro Met Ile Asp Ala Thr His Ile Gln Ser Ile Pro Pro
```

50 55 60

Gln Pro Tyr Asn Ser Leu Glu Asp Phe Phe Ser Met Thr Asp Ser Ser
65 70 75 80

Phe Asn Ser Phe Lys Gln Asn Asp Tyr Cys Leu Phe Leu Asn Ile Trp
85 90 95

Lys Pro Ser Ser Asn Gln Asn His Leu Pro Val Val Ile Tyr Phe Tyr
100 105 110

Gly Gly Ser Phe Leu Gln Gly His Gly Thr Ala Glu Leu Tyr Cys Pro
115 120 125

Glu His Ile Val Glu Gln Glu Asn Ile Ile Val Val Thr Phe Asn Tyr
130 135 140

Arg Leu Gly Ala Leu Gly Tyr Leu Asp Trp Ser Tyr Phe Asn Gln His
145 150 155 160

Leu Asn Tyr Asn Asn Gly Ile Ser Asp Gln Ile Asn Val Leu Arg Trp
165 170 175

Val His Gln Tyr Ile Glu His Phe Gly Gly Asp Ser Asn Asn Val Thr
180 185 190

Leu Met Gly Gln Ser Ala Gly Ser Met Ser Ile Met Thr Leu Met Gln
195 200 205

Met Pro Glu Leu Asp Asp Tyr Tyr His Lys Val Met Leu Leu Ser Gly
210 215 220

Thr Leu Thr Thr Asp Thr Pro Leu Asn Ala His Thr Lys Val Gln His
225 230 235 240

Phe Ser Gln Leu Met Arg His Tyr Phe Pro Asn Lys Thr Leu Lys Thr
245 250 255

Leu Thr Ser Asp Asp Ile Leu Tyr Leu Met Glu Ser Gln Lys Ile Glu
260 265 270

Arg Gly Arg Ser Arg Gly Leu Asp Leu Ile Tyr Gln Pro Ile Lys Asp
275 280 285

His His Met Ser Arg Ser Ile Lys Lys Phe Pro Lys Pro Thr Phe Met
290 295 300

Ser Tyr Thr His Asp Glu Gly Asp Ile Tyr Ile Glu Asp Ala Thr Arg
305 310 315 320

Thr Leu Pro Ser Glu Arg Phe Ile His Leu Met Ser Gln Tyr Gly Thr
325 330 335

His Val Glu Lys Asn Asp Ala Leu Thr Met Lys Gln Gln Arg Asn Leu
340 345 350

Ile Thr Glu Tyr Cys Phe Val Arg Pro Ile Tyr Leu Phe Leu Asn Lys
355 360 365

Met Asn Ser Cys Asp Thr Trp Leu Ala Arg Phe Asp Trp His Gln Pro
370 375 380

His Thr Ser Tyr Phe Lys Ser Ala Tyr His Ile Leu Asp Leu Val Phe
385 390 395 400

Trp Phe Gly His Leu Ser Ile Leu Thr Lys Asn His Tyr Ser Ile Thr
405 410 415

Gln His Asp Met Asn Leu Ser Arg Asn Met Ile Ser Asp Leu Ala Tyr
420 425 430

Phe Ala Arg Lys Gly Lys Met Pro Trp Lys Cys Tyr Glu Pro Gln His
435 440 445

Gln Ala Leu His Ile Tyr Arg
450 455

<210> 21
<211> 450
<212> PRT
<213> Staphylococcus aureus

<400> 21

```
Met Lys Ile Asn Thr Thr Gly Gly Gln Ile His Gly Ile Thr Gln Asp
1               5                   10                  15

Gly Leu Asp Ile Phe Leu Gly Ile Pro Tyr Ala Glu Pro Pro Val His
            20                  25                  30

Asp Asn Arg Phe Lys His Ser Thr Leu Lys Thr Gln Trp Ser Glu Pro
        35                  40                  45

Ile Asp Ala Thr Glu Ile Gln Pro Ile Pro Pro Gln Pro Asp Asn Lys
    50                  55                  60
```

Leu Glu Asp Phe Phe Ser Ser Gln Ser Thr Thr Phe Thr Glu His Glu
65 70 75 80

Asp Cys Leu Tyr Leu Asn Ile Trp Lys Gln His Asn Asp Gln Thr Lys
85 90 95

Lys Pro Val Ile Ile Tyr Phe Tyr Gly Gly Ser Phe Glu Asn Gly His
100 105 110

Gly Lys Ala Glu Leu Tyr Gln Pro Ala His Leu Val Gln Asn Asn Asp
115 120 125

Ile Ile Val Ile Thr Cys Asn Tyr Arg Leu Gly Ala Leu Gly Tyr Leu
130 135 140

Asp Trp Ser Tyr Phe Asn Lys Asp Phe His Ser Asn Asn Gly Leu Ser
145 150 155 160

Asp Gln Ile Asn Val Ile Lys Trp Val His Gln Phe Ile Glu Ser Phe
165 170 175

Gly Gly Asp Ala Asn Asn Ile Thr Leu Met Gly Gln Ser Ala Gly Ser
180 185 190

Met Ser Ile Leu Thr Leu Leu Lys Ile Pro Asp Ile Glu Pro Tyr Phe
195 200 205

His Lys Val Val Leu Leu Ser Gly Ala Leu Arg Leu Asp Thr Leu Glu
210 215 220

Ser Ala Arg Asn Lys Ala Gln His Phe Gln Lys Met Met Leu Asp Tyr
225 230 235 240

Leu Asp Thr Asp Asp Val Thr Ser Leu Ser Thr Asn Asp Ile Leu Met
245 250 255

Leu Met Ala Lys Leu Lys Gln Ser Arg Gly Pro Ser Lys Gly Leu Asp
260 265 270

Leu Ile Tyr Ala Pro Ile Lys Thr Asp Tyr Ile Gln Asn Asn Tyr Pro
275 280 285

Thr Thr Lys Pro Ile Phe Ala Cys Tyr Thr Lys Asp Glu Gly Asp Ile
290 295 300

Tyr Ile Thr Ser Glu Gln Lys Lys Leu Ser Pro Gln Arg Phe Ile Asp
305 310 315 320

Ile Met Glu Leu Asn Asp Ile Pro Leu Lys Tyr Glu Asp Val Gln Thr
325 330 335

Ala Lys Gln Gln Ser Leu Ala Ile Thr His Cys Tyr Phe Lys Gln Pro
340 345 350

Met Lys Gln Phe Leu Gln Gln Leu Asn Ile Gln Asp Ser Asn Ala Gln
355 360 365

Leu Trp Leu Ala Glu Phe Ala Trp His Asp Thr Ser Ser Ala His Tyr
370 375 380

Arg Ser Ala Tyr His Ile Leu Asp Met Val Phe Trp Phe Gly Asn Leu
385 390 395 400

Gln Ile Leu Ala Ala His Gln Tyr Pro Thr Thr Ala His Leu Lys Phe
405 410 415

Leu Ser Arg Gln Met Gln Asn Asp Leu Ala Asn Phe Ala Lys Ser Gly
420 425 430

Lys Met Pro Trp Pro Met Tyr His Asn Glu Arg Arg Tyr Tyr Arg Thr
435 440 445

Tyr Gln
450

<210> 22
<211> 492
<212> PRT
<213> Streptomyces avermitilis

<400> 22

```
Met Arg Gly Arg Leu Glu Gly Gly Leu Ala Val Phe Arg Gly Val Pro
1               5                   10                  15

Phe Ala Glu Pro Pro Val Gly Asp Ala Arg Phe Ala Ala Pro Arg Pro
            20                  25                  30

Val Arg Ala Trp Asp Gly Thr Arg Asp Ala Phe Ala Phe Gly Pro Pro
        35                  40                  45

Pro Pro Gln Glu Thr Gly Ile Gln Gly Arg Ala Ala Leu Leu Asp Ala
    50                  55                  60

Pro Thr Gly Asp Asp Trp Leu Thr Val Asn Val Trp Thr Pro Asp Pro
65                  70                  75                  80
```

Asp Pro Gly Ala Arg Arg Pro Val Met Val Trp Ile Tyr Gly Gly Ala
85 90 95

Tyr Lys Leu Gly His Ser Gly Ser Pro Gly Tyr Asp Ala Arg Arg Ile
100 105 110

Ala Arg Asp Gly Asp Val Val Val Val Thr Leu Asn Tyr Arg Val Gly
115 120 125

Ile Glu Gly Phe Ala Arg Val Asp Gly Ala Pro Ala Asn Arg Gly Leu
130 135 140

Leu Asp Gln Val Ala Ala Leu Glu Trp Val Arg Glu Asn Ile Thr Ala
145 150 155 160

Phe Gly Gly Asp Pro Gly Arg Val Thr Val Phe Gly Glu Ser Ala Gly
165 170 175

Ala Gly Ser Ile Ala Ser Leu Leu Ala Met Pro Ser Ala Ser Gly Leu
180 185 190

Phe Arg Arg Ala Ile Ala Gln Ser Val Pro Gly Thr Tyr Phe Ser Asp
195 200 205

Glu Leu Ala Lys Asp Ile Ala Ala Ala Ile Ala Ala Glu Ala Gly Leu
210 215 220

Arg Pro Thr Ala Ala Asp Leu Ser Thr Val Asp Pro Arg Gln Leu Pro
225 230 235 240

Ala Ala Gly Glu Ala Leu Ala Ala Thr Met Arg Gln Tyr Glu Asp Arg
245 250 255

Trp Gly Pro Val Val His Thr Leu Thr Pro Phe Ser Pro Val Val Asp
260 265 270

Gly Glu Val Leu Pro Thr Thr Pro Trp Gln Ala Leu Ala Ala Gly Thr
275 280 285

Ala Arg Asp Val Glu Leu Ile Val Gly His Asn Ser Glu Glu Phe Arg
290 295 300

Leu Phe Val Leu Leu Ser Gly Gln Leu Gly Lys Ile Thr Asp Gly Glu
305 310 315 320

Ala Arg Ala Ala Leu Arg Arg Phe Gly Pro Gly Pro Asp Ala Glu Gln
325 330 335

```
Ala Tyr Arg Thr Gly Phe Pro Asp Ala Ser Pro Gly Glu Leu Tyr Glu
                340                 345                 350

Arg Val Met Ser Asp Trp Leu Phe His Met Pro Ser Leu His Leu Ala
            355                 360                 365

Glu Ala Gln Leu Thr Gly Gly Gly Arg Ala His Val Tyr Glu Leu Thr
    370                 375                 380

Trp Pro Ala Pro Gly Asn Gly Gly Val Leu Gly Ala Cys His Gly Leu
385                 390                 395                 400

Asp Ile Pro Leu Leu Phe Gly Thr Phe Asp Ala Asp Leu Gly Ser Leu
                405                 410                 415

Leu Phe Ala Gly Thr Glu Pro Ser Pro Glu Ala Glu Ala Leu Ser Ser
            420                 425                 430

Arg Phe Arg Ala Ser Trp Thr Ala Phe Ala Arg Thr Gly Asp Pro Gly
        435                 440                 445

Trp Pro Thr Tyr Asp Thr Glu Arg Arg Leu Val Gln Val Leu Asp Ala
    450                 455                 460

Ala Pro Glu Val Ile Pro Tyr Pro Glu Glu Thr Ser Arg Arg Leu Trp
465                 470                 475                 480

Glu Arg His Thr Phe Pro Ala Leu Pro Leu Ile Gln
                485                 490
```

<210> 23
<211> 515
<212> PRT
<213> Caulobacter crescentus

<400> 23

```
Met Gly Phe Thr Ala Glu Ala Arg Ser Pro Val Val Ala Thr Thr Asn
1               5                   10                  15

Gly Lys Val Arg Gly Tyr Leu Asp Gly Glu Val Ser Val Phe Lys Gly
            20                  25                  30

Leu Arg Tyr Gly Ala Asp Thr Gly Gly Ala Arg Arg Phe Met Pro Pro
        35                  40                  45

Val Lys Pro Glu Pro Trp Thr Glu Val Lys Asp Ala Leu Ala Tyr Gly
    50                  55                  60
```

Pro Ala Ser Met Gln Thr Gly Lys Gly Glu Glu Gly Glu Thr Leu Ser
65 70 75 80

Glu Asp Cys Leu Phe Leu Asn Val Trp Thr Pro Ala Arg Ala Ser Arg
85 90 95

Lys Thr Gly Leu Ala Asp Gly Ala Lys Arg Pro Val Met Phe Tyr Ile
100 105 110

His Gly Gly Ala Tyr Asn Gly Gly Ser Gly Ala Ser Pro Trp Tyr Glu
115 120 125

Gly Thr Lys Leu Ala Lys Arg Gly Asp Val Val Val Val Thr Val Asn
130 135 140

His Arg Leu Asn Ala Phe Gly Tyr Leu Tyr Leu Ala Arg Leu Phe Asn
145 150 155 160

Ala Pro Ser Val Ala Asp Ser Gly Asn Val Gly Gln Leu Asp Leu Val
165 170 175

Leu Ala Leu Gln Trp Val Arg Asp Asn Ile Ala Arg Phe Gly Gly Asp
180 185 190

Pro Asp Cys Val Met Leu Phe Gly Gln Ser Gly Gly Gly Ala Lys Ile
195 200 205

Ala Thr Leu Met Ala Met Pro Ser Ala Lys Gly Leu Phe His Arg Ala
210 215 220

Ala Thr Met Ser Gly Gln Gln Val Thr Val Gly Gly Pro Phe Asn Ala
225 230 235 240

Thr Arg Arg Ala Lys Ala Phe Leu Asp Lys Leu Gly Val Lys Asp Leu
245 250 255

Ala Ala Leu Arg Ala Leu Pro Ala Ala Glu Met Leu Ala Gly Leu Lys
260 265 270

Ala Val Asp Pro Ile Ala Gly Ser Gly Gly Val Tyr Val Gly Pro Val
275 280 285

Leu Asp Gln Arg Ser Leu Leu Arg His Pro Phe Phe Pro Asp Ala Ala
290 295 300

Pro Gln Ser Leu Ser Ile Pro Met Met Val Gly Asn Thr His Asp Glu

305 310 315 320

Thr Lys Gly Phe Ile Gly Trp Asp Ala Lys Ala Phe Pro Gln Thr Trp
325 330 335

Asp Glu Val Ile Ala Arg Leu Pro Gly Gln Phe Ala Ala Arg Ile Asp
340 345 350

Ile Asp Pro Glu Thr Val Val Ala Phe Tyr Arg Gln Thr Tyr Pro Asn
355 360 365

Tyr Ser Pro Ala Asp Val Phe Phe Ala Ala Ser Thr Ala Gly Arg Ser
370 375 380

Trp Lys Ala Ala Ile Ile Gln Asp Glu Glu Arg Ala Lys Ala Gly Ala
385 390 395 400

Pro Ala Phe Ala Tyr Gln Val Asn Trp Arg Ser Pro Ile Gln Gly Gly
405 410 415

Ile Phe Gly Ala Pro His Thr Ile Asp Ile Gly Leu Val Phe Gly Thr
420 425 430

Leu Asp Ala Lys Gly Ser Ile Val Gly Thr Gly Pro Asp Ser Val Ala
435 440 445

Met Ser Asn Thr Met Ser Asp Ala Phe Ile Ala Phe Ala Arg Thr Gly
450 455 460

Asp Pro Asn Gly Gly Ala Leu Pro Lys Trp Glu Pro Tyr Thr Leu Pro
465 470 475 480

Arg Arg Gln Thr Met Val Phe Asp Thr Val Ser Arg Leu Glu Asp Asp
485 490 495

Pro Arg Gly Val Glu Arg Glu Phe Phe Asn Arg Val Pro Phe Thr Gln
500 505 510

Phe Gly Thr
515

<210> 24
<211> 497
<212> PRT
<213> Clostridium acetobuylicum

<400> 24

Met Gly Thr Ile Ala Glu Thr Lys Tyr Gly Lys Leu Glu Gly Ile Lys

1 5 10 15

Glu Asn Gly Ile Asn Lys Trp Leu Gly Ile Pro Tyr Ala Lys Pro Pro
20 25 30

Val Gly Glu Leu Arg Phe Lys Arg Thr Val Glu Cys Glu Pro Trp Asn
35 40 45

Gly Val Arg Tyr Ala Lys Lys His Gly Ser Lys Pro His Gln Phe Ala
50 55 60

Asn Thr Ser Glu Glu Val Gly Ile Glu Ser Glu Asp Cys Leu Tyr Met
65 70 75 80

Asn Ile Trp Ala Pro Glu Asn Ala Lys Asn Ser Pro Val Phe Val Trp
85 90 95

Ile Tyr Gly Gly Ala Tyr Ala Met Gly Ser Cys Ser Glu Ala Tyr Tyr
100 105 110

Asp Gly Thr Asn Phe Ala Lys Glu Gly Ile Val Tyr Val Ala Phe Asn
115 120 125

Tyr Arg Leu Gly Val Leu Gly Phe Tyr Asp Phe Thr Met Tyr Asp Asp
130 135 140

Ser Phe Asp Ser Asn Cys Gly Val Ser Asp Gln Ile Met Ala Leu Lys
145 150 155 160

Trp Val Lys Glu Asn Ile Glu Ala Phe Gly Gly Asp Pro Asn Asn Ile
165 170 175

Thr Ile Ala Gly Glu Ser Ala Gly Ala Ala Ser Val Thr Asn Met Leu
180 185 190

Ala Val Pro Lys Ala Lys Gly Leu Phe Asn Lys Ala Ile Ala Glu Ser
195 200 205

Pro Leu Pro Gly Cys Val Thr Ser His Asn Thr Ala Arg Leu Ile Thr
210 215 220

Asp Ile Tyr Leu Lys Arg Leu Gly Leu Glu Ala Ser Glu Val His Lys
225 230 235 240

Leu Lys Thr Met Glu Leu Glu Asp Ile Lys Lys Ala Ala Leu Tyr Val
245 250 255

```
Ile Asp Asp Thr Cys Ser Ser Tyr Pro Gly Met Tyr Ile Pro Gly Pro
            260                 265                 270

Val Leu Asp Asp Leu Ile Pro Arg Leu Pro Trp Glu Gly Ile Ala Leu
        275                 280                 285

Gly Ser Ser Lys Gly Val Lys Leu Ile Ile Gly Thr Asn His Asp Glu
    290                 295                 300

Gly Thr Leu Phe Ile Asn Lys Asn Lys Ser Met Leu Pro Gly Gly Trp
305                 310                 315                 320

Lys Asp Val Glu Arg Met Leu Arg Met Asn Lys Cys Phe Asp Ser Leu
                325                 330                 335

Pro Lys Ile His Lys Leu Tyr Asp Lys Phe Ser Glu Glu Met Ile Gln
            340                 345                 350

Ile Gln Glu Ile Met Lys Asp Arg Thr Phe Leu Val Asp Ser Ile Lys
        355                 360                 365

Val Ala Asp Ala Gln Ser Glu Lys Asn Asp Thr Trp Met Tyr Arg Phe
    370                 375                 380

Asp Tyr Ala Pro Ile Ser Ala Lys Leu Asn Gly Leu Gly Ala Thr His
385                 390                 395                 400

Ala Val Glu Val Ser Val Ala Leu Asn Asn Thr Lys Gly Glu Gly Ile
                405                 410                 415

Ala Tyr Ser Phe Trp Arg Asp Thr Pro Glu Asp Ile Ile Lys Lys Phe
            420                 425                 430

Ile Glu Asn Met His Met Ser Trp Val Asn Phe Ala Lys Thr Gly Asp
        435                 440                 445

Pro Asn Gly Asn Leu Asp Ile Glu Trp Lys Lys Tyr Asp Ser Lys Ser
    450                 455                 460

Arg Thr Thr Phe Val Phe Asp Glu Glu Asn Lys Val Glu Asn Asn Pro
465                 470                 475                 480

Ala Lys Asp Ile Tyr Glu Thr Trp Arg Asp Ile Lys Leu Tyr Thr Asp
                485                 490                 495

Ile
```

<210> 25
<211> 489
<212> PRT
<213> artificial

<220>
<223> protein of unknown origin, database entry gi 7546320

<400> 25

```
Met Thr His Gln Ile Val Thr Thr Gln Tyr Gly Lys Val Lys Gly Thr
1               5                   10                  15

Thr Glu Asn Gly Val His Lys Trp Lys Gly Ile Pro Tyr Ala Lys Pro
            20                  25                  30

Pro Val Gly Gln Trp Arg Phe Lys Ala Pro Glu Pro Pro Glu Val Trp
        35                  40                  45

Glu Asp Val Leu Asp Ala Thr Val Tyr Gly Pro Val Cys Pro Gln Pro
    50                  55                  60

Ser Asp Leu Leu Ser Leu Ser Tyr Lys Glu Leu Pro Arg Gln Ser Glu
65                  70                  75                  80

Asp Cys Leu Tyr Val Asn Val Phe Ala Pro Asp Thr Pro Ser Gln Asn
                85                  90                  95

Leu Pro Val Met Val Trp Ile His Gly Gly Ala Phe Tyr Leu Gly Ala
            100                 105                 110

Gly Ser Glu Pro Leu Tyr Asp Gly Ser Lys Leu Ala Ala Gln Gly Glu
        115                 120                 125

Val Ile Val Val Thr Leu Asn Tyr Arg Leu Gly Pro Phe Gly Phe Met
    130                 135                 140

His Leu Ser Ser Phe Asp Glu Ala Tyr Ser Asp Asn Leu Gly Leu Leu
145                 150                 155                 160

Asp Gln Ala Ala Ala Leu Lys Trp Val Arg Glu Asn Ile Ser Ala Phe
                165                 170                 175

Gly Gly Asp Pro Asp Asn Val Thr Val Phe Gly Glu Ser Ala Gly Gly
            180                 185                 190

Met Ser Ile Ala Ala Leu Leu Ala Met Pro Ala Ala Lys Gly Leu Phe
        195                 200                 205
```

```
Gln Lys Ala Ile Met Glu Ser Gly Ala Ser Arg Thr Met Thr Lys Glu
    210                 215                 220

Gln Ala Ala Ser Thr Ala Ala Ala Phe Leu Gln Val Leu Gly Ile Asn
225                 230                 235                 240

Glu Ser Gln Leu Asp Arg Leu His Thr Val Ala Ala Glu Asp Leu Leu
                245                 250                 255

Lys Ala Ala Asp Gln Leu Arg Ile Ala Glu Lys Glu Asn Ile Phe Gln
            260                 265                 270

Leu Phe Phe Gln Pro Ala Leu Asp Pro Lys Thr Leu Pro Glu Glu Pro
        275                 280                 285

Glu Lys Ser Ile Ala Glu Gly Ala Ala Ser Gly Ile Pro Leu Leu Ile
    290                 295                 300

Gly Thr Thr Arg Asp Glu Gly Tyr Phe Phe Phe Thr Pro Asp Ser Asp
305                 310                 315                 320

Val Tyr Ser Gln Glu Thr Leu Asp Ala Ala Leu Glu Tyr Leu Leu Gly
                325                 330                 335

Lys Pro Leu Ala Glu Lys Val Ala Asp Leu Tyr Pro Arg Ser Leu Glu
            340                 345                 350

Ser Gln Ile His Met Val Thr Asp Leu Leu Phe Trp Arg Pro Ala Val
        355                 360                 365

Ala Phe Ala Ser Ala Gln Ser His Tyr Ala Pro Val Trp Met Tyr Arg
    370                 375                 380

Phe Asp Trp His Pro Glu Lys Pro Pro Tyr Asn Lys Ala Phe His Thr
385                 390                 395                 400

Leu Glu Leu Pro Phe Val Phe Gly Asn Leu Asp Glu Leu Glu Arg Met
                405                 410                 415

Ala Lys Ala Glu Ile Thr Asp Glu Val Lys Gln Leu Ser His Thr Ile
            420                 425                 430

Gln Ser Ala Trp Thr Thr Phe Ala Lys Thr Gly Asn Pro Ser Thr Glu
        435                 440                 445

Ala Val Asn Trp Pro Ala Tyr His Glu Glu Ser Arg Glu Thr Val Ile
    450                 455                 460
```

Leu Asp Ser Glu Ile Thr Ile Glu Asn Asp Pro Glu Ser Glu Lys Arg
465 470 475 480

Gln Lys Leu Phe Pro Ser Lys Gly Glu
485

<210> 26
<211> 320
<212> PRT
<213> burkholderia cepacia

<400> 26

```
Ala Asp Asn Tyr Ala Ala Thr Arg Tyr Pro Ile Ile Leu Val His Gly
1               5                   10                  15

Leu Thr Gly Thr Asp Lys Tyr Ala Gly Val Leu Glu Tyr Trp Tyr Gly
            20                  25                  30

Ile Gln Glu Asp Leu Gln Gln Arg Gly Ala Thr Val Tyr Val Ala Asn
        35                  40                  45

Leu Ser Gly Phe Gln Ser Asp Asp Gly Pro Asn Gly Arg Gly Glu Gln
    50                  55                  60

Leu Leu Ala Tyr Val Lys Thr Val Leu Ala Ala Thr Gly Ala Thr Lys
65                  70                  75                  80

Val Asn Leu Val Gly His Ser Gln Gly Gly Leu Thr Ser Arg Tyr Val
                85                  90                  95

Ala Ala Val Ala Pro Asp Leu Val Ala Ser Val Thr Thr Ile Gly Thr
            100                 105                 110

Pro His Arg Gly Ser Glu Phe Ala Asp Phe Val Gln Gly Val Leu Ala
        115                 120                 125

Tyr Asp Pro Thr Gly Leu Ser Ser Thr Val Ile Ala Ala Phe Val Asn
    130                 135                 140

Val Phe Gly Ile Leu Thr Ser Ser Ser Asn Asn Thr Asn Gln Asp Ala
145                 150                 155                 160

Leu Ala Ala Leu Lys Thr Leu Thr Thr Ala Gln Ala Ala Thr Tyr Asn
                165                 170                 175

Gln Asn Tyr Pro Ser Ala Gly Leu Gly Ala Pro Gly Ser Cys Gln Thr
            180                 185                 190
```

```
Gly Ala Pro Thr Glu Thr Val Gly Gly Asn Thr His Leu Leu Tyr Ser
        195                 200                 205

Trp Ala Gly Thr Ala Ile Gln Pro Thr Ile Ser Val Phe Gly Val Thr
    210                 215                 220

Gly Ala Thr Asp Thr Ser Thr Ile Pro Leu Val Asp Pro Ala Asn Ala
225                 230                 235                 240

Leu Asp Pro Ser Thr Leu Ala Leu Phe Gly Thr Gly Thr Val Met Val
                245                 250                 255

Asn Arg Gly Ser Gly Gln Asn Asp Gly Val Val Ser Lys Cys Ser Ala
            260                 265                 270

Leu Tyr Gly Gln Val Leu Ser Thr Ser Tyr Lys Trp Asn His Leu Asp
        275                 280                 285

Glu Ile Asn Gln Leu Leu Gly Val Arg Gly Ala Asn Ala Glu Asp Pro
    290                 295                 300

Val Ala Val Ile Arg Thr His Ala Asn Arg Leu Lys Leu Ala Gly Val
305                 310                 315                 320
```

<210> 27

<211> 1473

<212> DNA

<213> Bacillus licheniformis

<400> 27

```
atgtatgata caactgtcga aacacgcttc ggaaagctga aaggcagagc ggaaaacgga     60
gtccgtatct ttaaaggcgt tccatacgca aaacctcccg tcggcgacct aaggtttcgg    120
gaaccgcagc gaatggaggc ctgggaaggt gagctggatg cttttcaatt tggcccggtt    180
tgtccgcagc ctgatggggt attgcctgag tcagcggggg ttcaaaagtc tgaggattgc    240
ctttatttaa atgtgtacgc acccgaagag gcggacgggg atctgcctgt tatggtgtgg    300
attcatgggg gcgcttttta tcgcggcgcc ggaagtgaac cgctctatga cgggactcag    360
cttgcaaagc agggaaaggt gatcgtggtc accatcaatt atcgcctcgg tccgttcggt    420
tttttgcatc tatcctcaat tgatgattcc tacagcagca atcttggcct gctggatcaa    480
atcgcggctc tcgagtgggt gaaagacaat atcgctttct ttggcggaga ccgtcatcac    540

attacggttt ttggagagtc ggcgggatcg atgagcatcg cttcgctttt ggcgatgccg    600
aaagcaaagg ggctttttca acaggccatt atggaaagcg gggcttccgc aactatgtcc    660
gataagcttg cgaaagctgc agcagaaaga ttcttaagga ttctcgatat tgatcatcat    720
catctggagc gccttcatga tgtatctgat caagaacttc ttgaagccgc cgatcagctg    780
cgcactttaa tgggagaaaa tatttttgaa ttgatttttc tgcctgcgct tgacgaaaaa    840
accttgccgc tgaagccgga ggtcgccgtc gcaaaaggcg cggcaaaaga gatcaatcta    900
ttaatcggaa caaaccgtga tgaaggcgtc ttgtttttcc cctctgattc ggatcttttg    960
cctgagagca agatcaacga gattttagaa gaatacatgg gtaaagaggc cgccgaagcc   1020
gcctcctctc tgtatccgag gtcattggaa ggccatgttg atatgatgac agatctgatc   1080
ttttggcatc cgtctgttgt gttcgcttcg gctcaatcac gatatgcatc tgtctttatg   1140
taccggtttg actggcatgc ggattcagag cagccgccgt tcaacaaagc tgcgcacggc   1200
ttagagattc cgtttgtatt tggaaatatg gacattttgg aacagctgac aggtacgaag   1260
gccggtgaag aagcgcagct gcttgctgaa cagatccagg ctgcctgggt gtcttttgcc   1320
cgatccggaa atccgagcac cgatgatgtc agctggcctg attatgatga agattcacgg   1380
aaaacgctga tttttgatca agaggtcgca gttgaaagcg atccttattc agataagaga   1440
aagatgttga cagccccgaa cccgcagatt tag                                1473
```

## Beschreibung

Die vorliegende Erfindung betrifft Wasch- oder Reinigungsmittel enthaltend para-Nitrobenzylesterasen (pNB-Esterasen), sowie deren Verwendung zur Ausrüstung von Fasern, insbesondere Kunstfasern, und entsprechende Wasch- und Reinigungsverfahren sowie weitere technische Einsatzmöglichkeiten. Insbesondere betrifft sie die Verwendung von pNB-Esterasen zum Schutz vor oder zur Verminderung bzw. Verhinderung des Pillings, bevorzugt bei Textilien, insbesondere Kunstfasern, besonders bevorzugt Fasern aus Polyester, sowie die Verwendung von pNB-Esterasen zur Spaltung von Kunststoffen, insbesondere von Polyesterverbindungen.

Esterasen im Allgemeinen stellen eine Gruppe hydrolytischer Enzyme mit einer natürlicherweise vorkommenden breiten Vielfalt hinsichtlich der Substrate und des Reaktionstyps dar. Die Substratspezifität und die Aktivierung der Enzyme unterscheidet sich von denen der Lipasen. Von den Lipasen ist bekannt, dass sie durch die Lipid/Wasser-Grenzfläche aktiviert werden, bevor sie wasserunlösliche Substrate mit langkettigen Fettsäureestern hydrolysieren. Nach Arpigny (Arpigny, Jäger, 1999 Biochem. J. 343, S. 177-183)) sind die Esterasen (EC 3.1.1) in drei verschiedene Klassen: die echte Lipasen (EC 3.1.1.3), Carboxylesterasen (EC 3.1.1.1) und verschiedene Arten von Phospholipasen zu unterscheiden. Die physiologischen Funktionen vieler Esterasen, z.B. der para-Nitrobenzylesterasen (PNB-Esterasen), sind bislang allerdings noch unklar. Normalerweise zeichnen sich esterolytische Enzyme dadurch aus, dass sie konservierte Regionen besitzen, die die katalytische Triade beinhalten sowie durch ihre Fähigkeit eine große Bandbreite an Reaktionen zu katalysieren. Hierbei besitzt jede Hydrolase hinsichtlich eines vorgegebenen Substrats unter bestimmten Reaktionsbedingungen eine spezifische Stereopräferenz, die als ihr charakteristischer Fingerabdruck bezeichnet werden kann. Esterasen können verwendet werden für die enzymatische Hydrolyse von racemischen Carbonsäureestern in ihre korrespondierenden Carbonsäuren und Alkohole. Des weiteren können sie für Umesterungen und für die Synthese von Estern verwendet werden. Die Fähigkeit der Esterasen sowohl in wässrigen als auch in nichtwässrigen Systemen aktiv zu sein macht sie zu wichtigen Werkzeugen für die organische Synthese. Esterasen sind hierbei von besonderem Interesse für die Synthese von enantiomerenreinen Produkten.

Als Vorraussetzung für die kommerzielle Anwendung von Esterasen, ist es wünschenswert, mehr Informationen über die biokatalytischen Eigenschaften von Esterasen, die die Umsetzung von organischen Verbindungen katalysieren, zu erhalten.

Unter Pilling versteht man das durch Reiben bewirkte Herausziehen feiner Fäserchen aus Geweben odergewirken, die sich zu Faserkügelchen ("Pills", "Knötchen") zusammenrollen und dann nur noch durch wenige Einzelfasern mit der Oberfläche der Gewebe oder Gewirke verbundenen sind. Bei Kunstfasern haften die kleinen Faserkügelchen fest auf der Oberfläche der Gewebe. Es werden daher Lösungen gesucht, die einerseits die bereits entstandene Knötchenbildung vermindern (Depilling) und andererseits einen Schutz der Fasern vor dem Pilling bieten, so dass also die unschöne Knötchenbildung gar nicht erst entsteht.

Der Einsatz von Cellulasen für die Anti-Pilling-Ausrüstung von Baumwolle und anderen natürlichen Fasern bzw. Textilien ist bekannt. Allerdings sind die Cellulasen nur wenig zur Behandlung der Bildung von Knötchen oder Kügelchen bei Kunstfasern, wie beispielsweise Polyestern oder Polyacrylfasern, geeignet.

Der vorliegenden Erfindung lag daher die Aufgabe zugrunde, neue Wege aufzufinden, die geeignet sind, dem Pilling, insbesondere von Polyester- oder Polyacrylfasern, entgegenzuwirken und/oder die entstehenden knötchenartigen Strukturen zu aufzubrechen bzw. zu zersetzen. Eine weitere Aufgabe bestand darin, neue Esterasen, insbesondere zum Einsatz in Wasch- und/oder Reinigungsmitteln bereitzustellen.

Diese Aufgaben werden gelöst durch die Verwendung von pNB-Esterasen zur Ausrüstung von Fasern, insbesondere Kunstfasern, insbesondere für Anti-Pilling-Ausrüstung, Wasch- und Reinigungsmittel sowie Mittel zur Ausrüstung von Textilien, insbesondere Textilnach- und/oder -vorbehandlungsmittel enthaltend pNB-Esterasen, entsprechende Ausrüstungs-, Wasch- und Reinigungsverfahren und die Verwendung von pNB-Esterasen in Wasch- und Reinigungsmitteln sowie weitere technische Einsatzmöglichkeiten. Insbesondere betrifft sie die Verwendung von pNB-Esterasen zum Schutz vor oder zur Verminderung bzw. Verhinderung des Pillings, bevorzugt bei Textilien, insbesondere Kunstfasern, besonders bevorzugt Fasern aus Polyester, sowie die Verwendung von pNB-Esterasen zur Spaltung von Kunststoffen, insbesondere von Polyesterverbindungen.

Wenn das Pilling verhindert oder die Knötchen auf den Fasern, insbesondere bei Textilien, verringert werden, erhält die Kleidung einen höherer Tragekomfort, insbesondere durch verbesserte Weichheit, und erhält länger ein gutes bzw. neues Aussehen der Kleidung.

Ferner sollten entsprechende Wasch- und Reinigungsmittel, entsprechende Wasch- und Reinigungsverfahren sowie entsprechende Verwendungsmöglichkeiten für derartige Esterasen zur Verfügung gestellt werden.

Die Lösung der Aufgabe besteht in der Verwendung von para-Nitrobenzylesterasen (pNB-Esterasen), insbesondere solchen, die aus Mikroorganismen, insbesondere Bakterien, bevorzugt aus Bakterien der Gattung *Bacillus,* gewonnen werden können.

Besonders geeignet sind für den Einsatz in den erfindungsgemäßen Mitteln sowie für die auch im weiteren noch aufgezählten erfindungsgemäßen Verwendungen solche Esterasen, die gemäß der in den Beispielen unter 2.4 genannten Methode, eine spezifische Aktivität gegenüber dem Substrat Bis-(p-methylbenzoesäure)-ethylenglykolester von 0,1 bis 30, bevorzugt 0,6 bis 20, insbesondere 0,7 bis 15, ganz besonders bevorzugt 0,9 bis 10, noch stärker bevorzugt 1 bis 5, insbesondere 1,1 bis 4, ganz besonders bevorzugt 1,5 bis 3 (μmol freigesetzte Säure)/(min*mg Enzym) aufweisen. Diese Esterasen haben sich als besonders vorteilhaft für den Einsatz in den erfindungsgemäßen Mitteln bzw. Verwendungen und Verfahren erwiesen.

Insbesondere geeignet sind Esterasen mit Aminosäuresequenzen, die zu der im Sequenzprotokoll unter SEQ ID NO. 1,2,4,6,11-14, 20-26 angegebenen Aminosäuresequenzen mindestens zu 50 %, bevorzugt 60 %, inbesondere 70 %, bevorzugt mindestens zu 80 %, insbesondere bevorzugt zu mindestens 90 %, bevorzugt mindestens 95 % und ganz besonders bevorzugt zu 100 % identisch bzw. zu mindestens 80 %, bevorzugt mindestens zu 85 %, insbesondere bevorzugt zu mindestens 90 %, bevorzugt mindestens 95 % und ganz besonders bevorzugt zu 100 % homolog sind.

Es werden entsprechende Wasch- und Reinigungsmittel, entsprechende Wasch-und Reinigungsverfahren sowie entsprechende Verwendungsmöglichkeiten für derartige Esterasen zur Verfügung gestellt. Schließlich werden technische Einsatzmöglichkeiten für die gefundenen Esterasen definiert.

Insbesondere bevorzugt für den Einsatz in den erfindungsgemäßen Mitteln sowie für die auch im weiteren noch aufgezählten erfindungsgemäßen Verwendungen sind solche Esterasen, die zu der unter Seq. ID Nr. 12 angegebenen Proteinsequenz zu mindestens 50 %, mindestens 55 %, insbesondere mindestens 60 %, bevorzugt mindestens 65 %, insbesondere bevorzugt mindestens 70 %, vorzugsweise mindestens 75 %, ganz besonders bevorzugt mindestens 80 %, insbesondere bevorzugt mindestens 85 %, mindestens 90 %, mindestens 95 %, mindestens 99 %, insbesondere 100 % homolog sind.

Homologisierung ist der Vergleich einer Nukleinsäure- oder Aminosäuresequenz mit der von bekannten Genen oder Proteinen. Sie wird beispielsweise über ein Alignment vorgenommen. Das Maß für die Homologie ist ein Prozentsatz an Identität, wie er beispielsweise nach der von D. J. Lipman und W. R. Pearson in Science 227 (1985), S. 1435-1441 angegebenen Methode bestimmt werden kann. Diese Angabe kann sich auf das gesamte Protein oder auf den jeweils zuzuordnenden Bereich beziehen. Ein weiter gefaßter Homologie-Begriff, die Ähnlichkeit, bezieht auch konservierte Variationen, also Aminosäuren mit ähnlicher chemischer Aktivität in die Betrachtung mit ein, da diese innerhalb des Proteins meist ähnliche chemische Aktivitäten ausüben. Bei Nukleinsäuren kennt man nur den Prozentsatz an Identität.

Durch Homologisierung lassen sich aus der Aminosäure- oder Nukleotid-Sequenz die Funktionen einzelner Sequenzbereiche sowie die enzymatische Aktivität des betrachteten gesamten Enzyms folgern. Homologe Bereiche von verschiedenen Proteinen sind solche mit vergleichbaren Funktionen, die sich durch Identität oder konservierte Austausche in der primären Aminosäuresequenz erkennen lassen. Sie umfassen einzelne Aminosäuren, kleinste Bereiche, sogenannte Boxen, die wenige Aminosäuren lang sind, bis hin zu langen Bereichen in der primären Aminosäuresequenz. Unter den Funktionen der homologen Bereiche sind somit auch kleinste Teilfunktionen der vom gesamten Protein ausgeübten Funktion zu verstehen, wie beispielsweise die Ausbildung einzelner Wasserstoffbrückenbindungen zur Komplexierung eines Substrats oder Übergangskomplexes. Andere Bereiche des Proteins, die nicht an der eigentlichen enzymatischen Reaktion beteiligt sind, können sie qualitativ oder quantitativ modifizieren. Dies betrifft beispielsweise die Enzymstabilität, die Aktivität, die Reaktionsbedingungen oder die Substratspezifität.

Unter dem Begriff einer Esterase wird ein Enzym mit Esteraseaktivität oder dem einer Esterase verstanden, darüber hinaus sind deshalb über die Funktionen der wenigen Aminosäurereste des katalytisch aktiven Zentrums hinaus alle Funktionen zu verstehen, wie sie sich durch das Einwirken des gesamten übrigen Proteins oder eines Teils oder mehrerer Teile des übrigen Proteins auf die eigentlich katalytisch aktiven Bereiche ergeben. Auch allein solche modifizierenden Funktionen oder Teilaktivitäten werden, sofern sie eine Esterase-Reaktion unterstützen, im Sinne der Erfindung als esterolytische Aktivität angesehen. Zu solchen Hilfsfunktionen oder Teilaktivitäten gehören beispielsweise die Bindung eines Substrats, eines Zwischen- oder Endprodukts, die Aktivierung oder die Inhibierung oder Vermittlung eines regulierenden Einflusses auf die hydrolytische Aktivität. Dabei kann es sich beispielsweise auch um die Ausbildung eines Strukturelements handeln, das fern vom aktiven Zentrum liegt. Die zweite Voraussetzung dafür, dass es sich erfiridungsgemäß um ein Protein mit Esteraseaktivität handelt, ist allerdings, dass sich durch das chemische Verhalten der eigentlich aktiven Reste allein oder zusätzlich durch das Einwirken der modifizierenden Teile eine Hydrolyse von Ester-Bindungen ergibt. Es ist darüber hinaus möglich, dass auch die Aktivitäten anderer Esterasen durch einen oder mehrere Teile, beispielsweise des erfindungsgemäßen Proteins qualitativ oder quantitativ modifiziert werden. Diese Beeinflussung anderer Faktoren wird ebenfalls als Esteraseaktivität angesehen. Aktive Enzyme sind auch solche Esterasen, deren Aktivität zu einem gegebenen Zeitpunkt, etwa durch einen Inhibitor blockiert ist. Entscheidend ist ihre prinzipielle Eignung zur entsprechenden Esterase-Reaktion.

Para-Nitrobenzyl-Esterasen im Sinne der Erfindung sind solche Enzyme, die in der Lage sind, die Hydrolyse von para-Nitrophenylacetat zu katalysieren. Weiterhin sind alle als para-Nitrobenzylesterasen in Datenbanken bezeichnete Enzyme bevorzugt und unter die erfindungsgemäßen Para-Nitrobenzylesterasen zu fassen.

Besonders bevorzugt sind solche Esterasen, die gemäß der in den Beispielen unter 2.4 genannten Methode, eine spezifische Aktivität gegenüber dem Substrat Bis-(p-methylbenzoesäure)-ethylenglykolester von 0,1 bis 30, bevorzugt 0,6 bis 20, insbesondere 0,7 bis 15, ganz besonders bevorzugt 0,9 bis 10, noch stärker bevorzugt 1 bis 5, insbesondere 1,1 bis 4, ganz besonders bevorzugt 1,5 bis 3 μmol freigesetzte Säure/min*mg Enzym aufweisen. Diese Esterasen haben sich als besonders vorteilhaft für den Einsatz in den erfindungsgemäßen Mitteln bzw. Verwendungen und Verfahren erwiesen.

Insbesondere bevorzugt sind solche Esterasen, die zu der unter Seq. ID Nr. 12 angegebenen Proteinsequenz zu mindestens 50 %, mindestens 55 %, insbesondere mindestens 60 %, bevorzugt mindestens 65 %, insbesondere bevorzugt mindestens 70 %, vorzugsweise mindestens 75 %, ganz besonders bevorzugt mindestens 80 %, insbesondere bevorzugt mindestens 85 %, mindestens 90 %, mindestens 95 %, mindestens 99 % homolog sind. Ganz besonders bevorzugt ist die Esterase gemäß Seq ID Nr. 12 bzw. Fragmente dieser Esterase, insbesondere solche Fragmente, die eine Esteraseaktivität aufweisen. Diese Esterasen weisen insbesondere eine überraschend gute Stabilität gegenüber höheren Temperaturen und höheren pH-Werten auf. Es wurde gefunden, dass diese Esterasen bei alkalischen pH-Werten auch bei einer Temperatur größer bzw. gleich 60°C über längere Zeit stabil bzw. aktiv sind.

Die erfindungsgemäßen Esterasen sind daher insbesondere bei alkalischem pH zum Einsatz bei hohen Temperaturen, insbesondere in Vollwaschmitteln, die üblicherweise einen alkalischen pH-Wert aufweisen, und insbesondere für Kochwäsche (Waschtemperatur bei 95 °C) geeignet.

Im Sinne der vorliegenden Erfindung werden alle Enzyme, Proteine, Fragmente, Fusionsproteine und Derivate, sofern sie nicht explizit als solche angesprochen zu werden brauchen, unter dem Oberbegriff Proteine zusammengefasst.

Unter der Leistung eines Enzyms wird dessen Wirksamkeit im jeweils betrachteten technischen Bereich, vorzugsweise im Rahmen eines entsprechend ausgerichteten Mittels verstanden. Diese basiert auf der eigentlichen enzymatischen Aktivität, hängt darüber hinaus aber von weiteren, für den jeweiligen Prozess relevanten Faktoren ab. Dazu gehören beispielsweise Stabilität, Substratbindung, Wechselwirkung mit dem das Substrat tragenden Material oder Wechselwirkungen mit anderen Inhaltsstoffen, insbesondere Synergien.

Unter der Waschleistung oder der Reinigungsleistung eines Wasch-, beziehungsweise Reinigungsmittels ist im Sinne der vorliegenden Anmeldung der Effekt zu verstehen, den das betrachtete Mittel auf die verschmutzten Artikel, beispielsweise Textilien oder Gegenstände mit harten Oberflächen ausübt. Einzelne Komponenten solcher Mittel, beispielsweise einzelne Enzyme, werden hinsichtlich ihres Beitrags zur Wasch- oder Reinigungsleistung des gesamten Wasch-, beziehungsweise Reinigungsmittels beurteilt. Denn aus den enzymatischen Eigenschaften eines Enzyms kann nicht ohne weiteres auf seinen Beitrag zur Waschleistung eines Mittels geschlossen werden. Hier spielen als weitere Faktoren beispielsweise Stabilität, Substratbindung, Bindung an das Reinigungsgut oder Wechselwirkungen mit anderen Inhaltsstoffen der Wasch-oder Reinigungsmittel, insbesondere Synergien bei der Entfernung der Verschmutzungen eine Rolle.

Der vorliegenden Erfindung liegt die Erkenntnis zugrunde, dass p-Nitrobenzylesterasen, bevorzugt solche Enzyme, die natürlicherweise in Bakterien vorkommen, ganz besonders in Bakterien aus der Gattung *Bacillus,* insbesondere bevorzugt der Spezies *Bacillus licheniformis* und *subtilis,* geeignet sind, die Knötchenbildung (Pilling) auf Textilfasern und -geweben zu vermindern oder zu verhindern.

Polyester sind Polymere, deren Grundbausteine durch Ester-Bindungen zusammengehalten werden. Nach ihrem chemischen Aufbau lassen sich die sogenannten *Homopolyester* in zwei Gruppen einteilen, die Hydroxycarbonsäure-Typen (AB-Polyester) und die Dihydroxy-Dicarbonsäure-Typen (AA-BB-Polyester). Erstere werden aus nur einem einzigen Monomer durch z. B. Polykondensation einer w-Hydroxycarbonsäure od. durch Ringöffnungspolymerisation cycl. Ester (Lactone) hergestellt. Der Aufbau letzterer erfolgt dagegen durch Polykondensation zweier komplementärer Monomere, z. B. einem Diol und einer Dicarbonsäure. Verzweigte und vernetzte Polyestern werden bei der Polykondensation von drei- od. mehrwertigen Alkoholen mit polyfunktionellen Carbonsäuren erhalten. Zu den Polyestern werden allgemein auch die Polycarbonate (Polyester der Kohlensäure) gerechnet. AB-Typ-P. (I) sind unter anderem Polyglykolsäuren *(Polyglykolide,* R = $CH_2$), Polymilchsäuren (*Polylactide,* R = CH -$CH_3$), Poly(β-hydroxybuttersäure) [Poly(3-hydroxybuttersäure), R = $CH(CH_3)$ -$CH_2$], Poly(ε-caprolacton)e [R = $(CH_2)_5$] und *Polyhydroxybenzoesäuren* (R = $C_6H_4$).

Rein aliphat. AA-BB-Typ-Polyester (II) sind Polykondensate aus aliphat. Diolen und Dicarbonsäuren, die unter anderem als Produkte mit endständigen HydroxyGruppen (als Polydiole) für die Herst. von Polyesterpolyurethanen eingesetzt werden [z. B. Polytetramethylenadipat; $R^1$ = $R^2$ = $(CH_2)_4$]. Mengenmäßig größte techn. Bedeutung haben AA-BB-Typ-Polyester aus aliphat. Diolen und aromat. Dicarbonsäuren, insbes. die Polyalkylenterephthalate [$R^2$ = $C_6H_4$, mit Polyethylenterephthalat (PET) $R^1$ = $(CH_2)_2$, Polybutylenterephthalat (PBT) $R^1$ = $(CH_2)_4$ und Poly(1,4-cyclohexandimethylen-terephthalat)e (PCDT) $R^1$ = $CH_2$ -$C_6H_{10}$ -$CH_2$] als wichtigste Vertreter. Diese Typen von Polyestern können durch Mitverwenden anderer aromat. Dicarbonsäuren (z. B. Isophthalsäure) bzw. durch Einsatz von Diol-Gemischen bei der Polykondensation in ihren Eigenschaften breit variiert und unterschiedlichen Anwendungsgebieten angepasst werden.

Rein aromatische Polyester sind die Polyarylate, zu denen unter anderem die Poly(4-hydroxybenzoesäure) (Formel I, R = $C_6H_4$), Polykondensate aus Bisphenol A und Phthalsäuren (Formel II, $R^1$ = $C_6H_4$ $C(CH_3)_2$ $C_6H_4$, $R^2$ = $C_6H_4$) od. auch solche aus Bisphenolen und Phosgen gehören.

Zusätzlich zu den bisher genannten gesättigten Polyestern lassen sich auch ungesättigte Polyester aus ungesättigten. Dicarbonsäuren herstellen, die als Polyesterharze, insbes. als ungesättigte Polyesterharze *(UP-Harze),* techn.

Bedeutung erlangt haben.

Polyester findet man auch in der Natur, wo sie aus Hydroxycarbonsäuren gebildet werden *(Beisp.:* Depside und Depsipeptide). Vielen Bakterien dient Poly(β-hydroxybuttersäure) als Speicherstoff. Erdbienen produzieren Polyester aus 18-Hydroxyoctadecan- und 20-Hydroxyeicosansäure zur Auskleidung ihrer Nester.

Die PNB-Esterasen zeichnen sich dadurch aus, dass sie eine besonders guten Schutz von Textilfasern vor Pilling bieten.

Geeignete para-Nitrobenzylesterasen (p-Nitrobenzylesterasen, pNB-Esterasen, EST-B) im Sinne der Erfindung sind insbesondere solche Enyzme, wie sie in den Patentanmeldungen US5,468,632, US 5,906930, US5,945325, EP0549264 beschrieben sind.

Weiterhin besonders bevorzugt ist die Verwendung solcher Paranitrobenzylesterasen, mit einer Aminosäuresequenz, die zu einer der in SEQ ID NO. 1,2,4, 6, 11-14 oder 20-25 angegebenen Aminosäuresequenzen zu mindestens 50 %, bevorzugt zu mindestens 60 %, insbesondere zu mindestens 70 %, zu mindestens 80, zu mindestens 85%, zu mindestens 86%, zu mindestens 87%, zu mindestens 88%, zu mindestens 89%, zu mindestens 90 %, zu mindestens 91 %, zu mindestens 92 %, zu mindestens 93 %, zu mindestens 94 %, zu mindestens 95 %, zu mindestens 96 %, zu mindestens 97%, zu mindestens 98 %, zu mindestens 99 % oder 100 % identisch und/oder zu mindestens 80, zu mindestens 85%, zu mindestens 86%, zu mindestens 87%, zu mindestens 88%, zu mindestens 89%, zu mindestens 90 %, zu mindestens 91 %, zu mindestens 92 %, zu mindestens 93 %, zu mindestens 94 %, zu mindestens 95 %, zu mindestens 96 %, zu mindestens 97%, zu mindestens 98 %, zu mindestens 99 % oder 100 % homolog sind.

Besonders bevorzugt sind solche Para-Nitrobenzylesterasen, die zu den angegebenen Aminosäuresequenzen (1,2,4,6,11-14, 20-25) zu 95, besonders bevorzugt 98, insbesondere 100 % identisch sind.

Besonders bevorzugt sind hierbei solche Esterasen, die gemäß der in den Beispielen unter 2.4 genannten Methode, eine spezifische Aktivität gegenüber dem Substrat Bis-(p-methylbenzoesäure)-ethylenglykolester von 0,1 bis 30, bevorzugt 0,6 bis 20, insbesondere 0,7 bis 15, ganz besonders bevorzugt 0,9 bis 10, noch stärker bevorzugt 1 bis 5, insbesondere 1,1 bis 4, ganz besonders bevorzugt 1,5 bis 3 μmol freigesetzte Säure/min*mg Enzym aufweisen. Diese Esterasen haben sich als besonders vorteilhaft für den Einsatz in den erfindungsgemäßen Mitteln bzw. Verwendungen und Verfahren erwiesen.

Insbesondere bevorzugt sind solche Esterasen, die zu der unter Seq. ID Nr. 12 angegebenen Proteinsequenz zu mindestens 50 %, mindestens 55 %, insbesondere mindestens 60 %, bevorzugt mindestens 65 %, insbesondere bevorzugt mindestens 70 %, vorzugsweise mindestens 75 %, ganz besonders bevorzugt mindestens 80 %, insbesondere bevorzugt mindestens 85 %, mindestens 90 %, mindestens 95 %, mindestens 99 % , insbesondere 100 % homolog sind.

Ein weiterer Gegenstand der Erfindung ist die Verwendung von p-Nitrobenzylesterasen zur Spaltung von Polyalkylenterephtalaten, insbesondere Polyethylenterephtalaten (Abk.: PET oder PETE).

Ebenfalls können die Esterasen zur Spaltung bzw. zum Abbau von Kunststoffen, insbesondere Polyestern und/oder Weichmachern in Kunststoffen eingesetzt werden. Als Weichmacher in Kunststoffen werden häufig Phthalate eingesetzt, um die Eigenschaften der Kunststoffe für die Herstellung oder den Gebrauch zu verbessern.

Die Erfindung betrifft den partiellen oder vollständigen Abbau von Formkörpern, Flächengebilden, Beschichtungen, Verklebungen oder Schäumen aus biologisch abbaubaren Polymeren mit Enzymen. Insbesondere betrifft sie den enzymatischen Abbau von Polyestern. Das Verfahren zum Abbau von Polymeren kann auf verschiedene Weise durchgeführt werden:

Das Polymer wird der wässrigen Enzym-enthaltenden Lösung zugesetzt. Das biologisch abbaubare Polymer kann als Film, Folie oder Granulat zugesetzt werden. Formkörper können als Ganzes oder zerkleinert zugesetzt werden. Beschichtete oder verklebte Materialien oder Materialien, bei denen mit biologisch abbaubaren Polymeren Beschichtungen aufgetragen wurden oder Verklebungen erzeugt wurden, wie beispielsweise Papier oder Pappe sowie beschichtetes Papier oder beschichtete Pappe, können als Ganzes oder zerkleinert der enzymhaltigen Lösung zugesetzt werden.

Weiter kann man die wässrige enzymhaltige Lösung durch Aufsprühen auf die abzubauende Beschichtung oder den abzubauenden Formkörper auftragen oder aufsprühen.

Das beschriebene Verfahren des enzymatischen Abbaus von biologisch und enzymatisch abbaubaren Polymeren sowie daraus hergestellten Blends kann erfindungsgemäß beispielsweise eingesetzt werden zum Einschluß von Chemikalien, Wirkstoffen, Hormonen, Hilfsmitteln, Enzymen, Mikroorganismen, Pflanzensamen in (z.B. Kapseln und Mikrokapseln) und deren gezielter Freisetzung durch den Zusatz von Enzymen

Somit kann die Umwelt durch die Verwendung des erfindungsgemäßen Verfahrens, z.B. in Müllaufbereitungsanlagen, von biologisch und/oder abbaubaren Polymeren oder deren Gemischen schneller befreit werden.

Besonders bevorzugt sind hierbei solche Esterasen, die gemäß der in den Beispielen unter 2.4 genannten Methode, eine spezifische Aktivität gegenüber dem Substrat Bis-(p-methylbenzoesäure)-ethylenglykolester von 0,1 bis 30, bevorzugt 0,6 bis 20, insbesondere 0,7 bis 15, ganz besonders bevorzugt 0,9 bis 10, noch stärker bevorzugt 1 bis 5, insbesondere 1,1 bis 4, ganz besonders bevorzugt 1,5 bis 3 μmol freigesetzte Säure/min*mg Enzym aufweisen. Diese Esterasen haben sich als besonders vorteilhaft für den Einsatz in den erfindungsgemäßen Mitteln bzw. Verwendungen und Verfahren erwiesen.

Insbesondere bevorzugt sind solche Esterasen, die zu der unter Seq. ID Nr. 12 angegebenen Proteinsequenz zu mindestens 50 %, mindestens 55 %, insbesondere mindestens 60 %, bevorzugt mindestens 65 %, insbesondere bevorzugt mindestens 70 %, vorzugsweise mindestens 75 %, ganz besonders bevorzugt mindestens 80 %, insbesondere bevorzugt mindestens 85 %, mindestens 90 %, mindestens 95 %, mindestens 99 %, insbesondere 100 % homolog sind. Insbesondere bevorzugt sind ebenfalls Mutationen des unter SEQ ID NO. 12 angegebenen Enzyms, die eine noch verbesserte erfindungsgemäße Wirkung aufweisen.

Die Nukleotidsequenz einer erfindungsgemäß einsetzbaren Esterase aus *Bacillus subtilis (17A1)* ist im Sequenzprotokoll der vorliegenden Anmeldung unter SEQ ID NO. 3 angegeben. Sie umfasst 1470 bp. Die hiervon abgeleitete Aminosäuresequenz wird in SEQ ID NO. 1 angegeben. Sie umfasst 489 Aminosäuren, gefolgt von einem Stop-Codon.

Die Nukleotidsequenz einer erfindungsgemäß einsetzbaren Esterase aus *Bacillus licheniformis* (*19C5*) ist im Sequenzprotokoll der vorliegenden Anmeldung unter SEQ ID NO. 4 angegeben. Sie umfassen jeweils 1470 bp. Die hiervon abgeleitete Aminosäuresequenz wird in SEQ ID NO. 2 angegeben. Sie umfasst 489 Aminosäuren, gefolgt von einem Stop-Codon.

Aufgrund der zu erkennenden Übereinstimmungen und der Verwandtschaft zu den anderen angegebenen Esterasen sind die Esterasen als p-Nitrobenzylesterasen anzusehen.

Ein Gegenstand der vorliegenden Erfindung ist somit insbesondere die erfindungsgemäße Verwendung einer Esterase mit einer Aminosäuresequenz, die zu den in SEQ ID NO. 1, 2, 5 oder 6 angegebenen Aminosäuresequenzen mindestens zu 70% identisch ist.

Zunehmend bevorzugt ist hierbei die Verwendung solcher Esterasen, deren Aminosäuresequenz zu der in SEQ ID NO. 1, 2, 5 oder 6, bevorzugt 1 oder 2, angegebenen Aminosäuresequenz mindestens zu jeweils 72%, 74%, 76%, 78%, 80%, 82%, 84%, 85%, 86%, 88%, 90%, 91%, 92%, 93%, 94%, 95%, 96%, 97%, 98%, 99% und zu 100% identisch ist. Es ist zu erwarten, dass deren Eigenschaften denen der gefundenen zunehmend ähnlich sind.

Die am meisten bevorzugte Ausführungsform dieses Erfindungsgegenstands ist die Verwendung einer Esterase, deren Aminosäuresequenz mit den in SEQ ID NO. 1, 2, 5 oder 6 angegebenen Aminosäuresequenzen insgesamt identisch ist.

Denn um eine solche handelt es sich bei der neu gefundenen und mit der vorliegenden Anmeldung zur Verfügung gestellten Esterase aus *Bacillus* subtilis oder licheniformis.

Diese sind im Stand der Technik noch nicht bekannte Esterasen. Sie sind, wie in den Beispielen angegeben isolierbar, herstellbar und einsetzbar. Sie zeichnen sich zusätzlich dadurch aus, dass sie bei Verwendung in einem entsprechenden Mittel den für diesen Zweck etablierten Enzymen in der Leistung nahekommen, beziehungsweise sie sogar übertreffen.

Für die Entwicklung von technischen, insbesondere in Waschmitteln einsetzbaren Esterasen kann sie als ein natürlicherweise, mikrobiell gebildetes Enzym als Ausgangspunkt dienen, um über an sich bekannte Mutagenese-Verfahren, beispielsweise Punktmutagenese, Fragmentierung, Deletion, Insertion oder Fusion mit anderen Proteinen oder Proteinteilen oder über sonstige Modifikationen für den gewünschten Einsatz optimiert zu werden. Derartige Optimierungen können beispielsweise Anpassungen an Temperatur-Einflüsse, pH-Wert-Schwankungen, Redox-Verhältnissen und/oder sonstigen Einflüsse, sein, die für die technischen Einsatzgebiete relevant sind. Gewünscht sind beispielsweise eine Verbesserung der Oxidationsbeständigkeit, der Stabilität gegenüber denaturierenden Agentien oder proteolytischem Abbau, gegenüber hohen Temperaturen, sauren oder stark alkalischen Bedingungen, eine Senkung der Immunogenität oder der allergenen Wirkung.

Die Mutagenese-Verfahren beruhen auf einer der zugehörigen Nukleotidsequenz, die in SEQ ID NO. 3, 4, 7 oder 8 angegeben sind, beziehungsweise den hierzu hinreichend ähnlichen Nukleotidsequenzen. Entsprechende molekularbiologische Methoden sind im Stand der Technik beschrieben, so beispielsweise in Handbüchern wie dem von Fritsch, Sambrook und Maniatis "Molecular cloning: a laboratory manual", Cold Spring Harbour Laboratory Press, New York, 1989. Eine bevorzugte Ausführungsform stellt die Verwendung all solcher bislang ausgeführter Proteine dar, die dadurch gekennzeichnet sind, dass sie zusätzlich derivatisiert sind.

Unter Derivaten werden solche Proteine verstanden, die sich über eine zusätzliche Modifikation von den ausgeführten Proteinen ableiten. Derartige Modifikationen können beispielsweise die Stabilität, Substratspezifität oder die Bindungsstärke an das Substrat oder die enzymatische Aktivität beeinflussen. Sie können auch dazu dienen, um die Allergenizität und/oder Immunogenizität des Proteins herabzusetzen und damit beispielsweise dessen Hautverträglichkeit zu erhöhen.

Solche Derivatisierungen können beispielsweise biologisch erfolgen, etwa im Zusammenhang mit der Proteinbiosynthese durch den produzierenden Wirtsorganismus. Hier sind Kopplungen niedrigmolekularer Verbindungen wie von Lipiden oder Oligosacchariden besonders hervorzuheben.

Derivatisierungen können aber auch chemisch durchgeführt werden, etwa durch die chemische Umwandlung einer Seitenkette oder durch kovalente Bindung einer anderen, beispielsweise makromolekularen Verbindung an das Protein. Beispielsweise ist die Kopplung von Aminen an Carboxylgruppen eines Enzyms zur Veränderung des isoelektrischen Punkts möglich. Es können beispielsweise Makromoleküle wie Proteine, etwa über bifunktionelle chemische Verbindungen an erfindungsgemäße Proteine gebunden werden. So ist es beispielsweise möglich, ein erfindungsgemäßes Protein über einen Linker mit einer spezifischen Bindungsdomäne zu versehen. Solche Derivate eignen sich besonders für den Einsatz in Wasch- oder Reinigungsmitteln. Analog WO 00/01831 können auch Esterase-Inhibitoren über Linker, insbesondere Aminosäure-Linker an die erfindungsgemäßen Proteine gebunden werden. Kopplungen mit sonstigen makromolekularen Verbindungen, wie etwa Polyethylenglykol verbessern das Molekül hinsichtlich weiterer Eigenschaften wie Stabilität oder Hautverträglichkeit.

Unter Derivaten erfindungsgemäßer Proteine können im weitesten Sinne auch Präparationen dieser Enzyme verstanden werden. Je nach Gewinnung, Aufarbeitung oder Präparation kann ein Protein mit diversen anderen Stoffen vergesellschaftet sein, beispielsweise aus der Kultur der produzierenden Mikroorganismen. Ein Protein kann auch, beispielsweise zur Erhöhung seiner Lagerstabilität, mit bestimmten anderen Stoffen gezielt versetzt worden sein. Erfindungsgemäß sind deshalb auch alle Präparationen eines erfindungsgemäßen Proteins. Das ist auch unabhängig davon, ob es in einer bestimmten Präparation tatsächlich diese enzymatische Aktivität entfaltet oder nicht. Denn es kann gewünscht sein, dass es bei der Lagerung keine oder nur geringe Aktivität besitzt, und erst zum Zeitpunkt der Verwendung seine esterolytische Funktion entfaltet. Dies kann beispielsweise über entsprechende Begleitstoffe gesteuert werden.

Eine bevorzugte Ausführungsform stellt die Verwendung all solcher bislang ausgeführter Proteine dar, die dadurch gekennzeichnet sind, dass sie zusätzlich stabilisiert sind.

Dadurch wird ihre Stabilität bei der Lagerung und/oder während ihres Einsatzes, beispielsweise beim Waschprozess erhöht, so dass ihre Aktivität länger anhält und damit verstärkt wird. Die Stabilität erfindungsgemäßer Esterasen kann beispielsweise durch Kopplung an Polymere erhöht werden. Sie erfordert, dass die Proteine vor ihrem Einsatz in entsprechenden Mitteln über einen chemischen Kopplungsschritt mit derartigen Polymeren verbunden werden.

Bevorzugt sind Stabilisierungen, die über Punktmutagenese des Moleküls selbst möglich sind. Denn diese erfordern im Anschluss an die Proteingewinnung keine weiteren Arbeitsschritte. Einige hierfür geeigneten Punktmutationen sind an sich aus dem Stand der Technik bekannt.

Andere Möglichkeiten sind beispielsweise:

- Der Austausch bestimmter Aminosäurereste gegen Prolin;
- die Einführung polarerer oder geladener Gruppen auf der Oberfläche des Moleküls;

Eine andere Möglichkeit zur Stabilisierung gegenüber erhöhter Temperatur und dem Einwirken von Tensiden kann in der Stabilisierung über den Austausch von Aminosäuren, die nahe dem N-Terminus liegen, gegen solche, die über nichtkovalente Wechselwirkungen mit dem Rest des Moleküls in Kontakt treten und somit einen Beitrag zur Aufrechterhaltung der globulären Struktur leisten.

Bevorzugte Ausführungsformen sind solche, bei denen das Molekül auf mehrere Arten stabilisiert wird. Es kann davon ausgegangen werden, dass mehrere stabilisierende Mutationen additiv wirken.

In einer bevorzugten Ausführungsform werden solche bislang ausgeführten Proteine eingesetzt, die dadurch gekennzeichnet sind, dass sie aus einer natürlichen Quelle, insbesondere aus einem Mikroorganismus erhältlich sind.

Dies können beispielsweise einzellige Pilze oder Bakterien sein. Denn sie lassen sich zumeist einfacher gewinnen und handhaben als vielzellige Organismen oder die von Vielzellern abgeleiteten Zellkulturen; obgleich diese für spezielle Ausführungsformen sinnvolle Optionen darstellen können und somit nicht grundsätzlich vom Erfindungsgegenstand ausgeschlossen sind.

Es ist möglich, dass natürlich vorkommende Produzenten zwar ein erfindungsgemäßes Enzym herstellen können, dieses aber unter den zunächst ermittelten Bedingungen nur in geringem Maße exprimieren und/oder in das umgebende Medium abgeben. Das schließt aber nicht aus, dass geeignete Umweltbedingungen oder sonstige Faktoren experimentell ermittelt werden können, unter deren Einwirken sie zu einer wirtschaftlich sinnvollen Produktion des erfindungsgemäßen Proteins angeregt werden können. Solch ein Regulationsmechanismus kann für die biotechnologische Produktion gezielt eingesetzt werden. Sollte auch dies nicht möglich sein, können sie immer noch der Isolierung des zugehörigen Gens dienen.

Besonders bevorzugt sind hierunter solche aus gram-positiven Bakterien. Denn diese besitzen keine äußere Membran und geben sekretierte Proteine somit unmittelbar ins umgebende Medium ab.

Ganz besonders bevorzugt sind solche aus gram-positiven Bakterien der Gattung Bacillus.

Bacillus-Esterasen weisen von vornherein günstige Eigenschaften für diverse technische Einsatzmöglichkeiten auf. Dazu gehören eine gewisse Stabilität gegenüber erhöhter Temperatur, oxidierenden oder denaturierenden Agentien. Zudem hat man mit mikrobiellen Enzymen die größten Erfahrungen hinsichtlich ihrer biotechnologischen Produktion, was beispielsweise die Konstruktion günstiger Klonierungsvektoren, die Auswahl von Wirtszellen und Wachstumsbedingungen oder die Abschätzung von Risiken, wie beispielsweise die Allergenizität, angeht. Bacilli sind zudem als Produktionsorganismen mit einer besonders hohen Produktionsleistung in technischen Prozessen etabliert. Der Erfahrungsschatz, den man für die Herstellung und den Einsatz dieser Enzyme erworben hat, kommt zudem erfindungsgemäßen Weiterentwicklungen dieser Enzyme zugute. Dies betrifft beispielsweise ihre Kompatibilität mit anderen chemischen Verbindungen, wie beispielsweise die Inhaltsstoffe von Wasch- oder Reinigungsmitteln.

Unter denen aus den Bacillus-Species, wiederum, sind solche aus der Spezies *Bacillus subtilis oder licheniformis* bevorzugt.

Denn aus diesen wurden die Ausführungsformen der erfindungsgemäßen Enzyme ursprünglich erhalten. Die zugehörigen Sequenzen sind im Sequenzprotokoll angegeben. Von diesem oder von verwandten Stämmen können die oben beschriebenen Varianten insbesondere unter Anwendung molekularbiologischer Standardmethoden, wie beispielsweise der PCR und/oder an sich bekannten Punktmutagenese-Verfahren hergestellt werden.

Als Wirtszellen zur Proteinexpression erfindungsgemäß einsetzbarer Esterasen eignen sich prinzipiell alle Organismen außer Mensch, das heißt Prokaryonten, Eukaryonten oder Cyanophyta. Bevorzugt sind solche Wirtszellen, die sich genetisch gut handhaben lassen, was beispielsweise die Transformation mit dem Expressionsvektor, dessen stabile Etablierung und die Regulation der Expression angeht, beispielsweise einzellige Pilze oder Bakterien. Zudem zeichnen sich bevorzugte Wirtszellen durch eine gute mikrobiologische und biotechnologische Handhabbarkeit aus. Das betrifft beispielsweise leichte Kultivierbarkeit, hohe Wachstumsraten, geringe Anforderungen an Fermentationsmedien und gute Produktions- und Sekretionsraten für Fremdproteine. Vorzugsweise werden Laborstämme gewählt, die auf die Expression ausgerichtet sind. Solche sind kommerziell oder über allgemein zugängliche Stammsammlungen erhältlich. Jedes erfindungsgemäße Protein kann auf diese Weise theoretisch aus einer Vielzahl von Wirtsorganismen gewonnen werden. Aus der Fülle an verschiedenen nach dem Stand der Technik zur Verfügung stehenden Systeme müssen die optimalen Expressionssysteme für den Einzelfall experimentell ermitteln werden. Als Wirtszellen bevorzugte Bakterien sind solche, die dadurch gekennzeichnet sind, dass sie gram-positive Bakterien sind, insbesondere dass sie der Gattung Bacillus angehören, ganz besonders der Species *Bacillus lentus, Bacillus licheniformis, Bacillus amyloliquefaciens, Bacillus subtilis* oder *Bacillus alcalophilus.*

Beispiele für geeignete Eukaryonten sind Pilze wie Actinomyceten oder Hefen wie *Saccharomyces* oder *Kluyveromyces,* sowie thermophile pilzliche Expressionssysteme. Solche eignen sich besonders zur Expression temperaturbeständiger Varianten. Zu den Modifikationen, die eukaryontische Systeme besonders im Zusammenhang mit der Proteinsynthese durchführen, gehören beispielsweise die Bindung niedermolekularer Verbindungen wie Membrananker oder Oligosaccharide. Derartige Oligosaccharid-Modifikationen können beispielsweise zur Senkung der Allergenizität wünschenswert sein. Auch eine Coexpression mit den natürlicherweise von derartigen Zellen gebildeten Enzymen, wie beispielsweise Cellulasen, kann vorteilhaft sein.

Einen eigenständigen Erfindungsgegenstand stellen Verfahren zur Herstellung eines erfindungsgemäßen Proteins dar.

Beansprucht wird somit jedes Verfahren zur Herstellung eines oben beschriebenen, erfindungsgemäßen Proteins, Proteinfragments, Fusionsproteins oder Derivats unter Einsatz einer oben beschriebenen, erfindungsgemäßen Nukleinsäure und/oder unter Einsatz eines oben beschriebenen, erfindungsgemäßen Vektors und/oder unter Einsatz einer der oben beschriebenen, erfindungsgemäßen Zellen.

Hierzu gehören beispielsweise chemische Syntheseverfahren, die insbesondere für kürzere Fragmente wirtschaftlich sinnvoll sind.

Demgegenüber bevorzugt sind jedoch alle im Stand der Technik etablierten, oben in einzelnen Aspekten bereits angesprochenen molekularbiologischen, mikrobiologischen, beziehungsweise biotechnologischen Herstellverfahren. Demnach können beispielsweise anhand der oben bezeichneten DNA- und Aminosäuresequenzen, wie sie beispielsweise auch aus dem Sequenzprotokoll abgeleitet werden können, vorzugsweise anhand derer aus SEQ ID NO. 1, 2, 5 und 6 selbst, entsprechende Oligonukleotide und Oligopeptide bis hin zu den vollständigen Genen und Proteinen nach an sich bekannten molekularbiologischen Methoden synthetisiert werden.

Einen eigenen Erfindungsgegenstand stellen Wasch- oder Reinigungsmittel dar, die dadurch gekennzeichnet sind, dass sie ein oben beschriebenes, erfindungsgemäßes Protein enthalten.

Alle Arten von Wasch- oder Reinigungsmitteln, insbesondere Gemische, Rezepturen, Lösungen etc., deren Einsetzbarkeit durch Zugabe eines oben beschriebenen, erfindungsgemäßen Proteins verbessert wird, werden hiermit in den Schutzbereich der vorliegenden Erfindung eingeschlossen. Es kann sich dabei je nach Einsatzgebiet beispielsweise um feste Gemische, beispielsweise Pulver mit gefriergetrockeneten oder verkapselten Proteinen, oder um gelförmige oder flüssige Mittel handeln. Bevorzugte Rezepturen enthalten beispielsweise Puffersubstanzen, Stabilisatoren und/oder Reaktionspartner der Esterasen und/oder andere mit den Esterasen synergistische Inhaltsstoffe.

Zu diesem Erfindungsgegenstand zählen alle denkbaren Reinigungsmittelarten, sowohl Konzentrate als auch unverdünnt anzuwendende Mittel, zum Einsatz im kommerziellen Maßstab, in der Waschmaschine oder bei der Hand-Wäsche, beziehungsweise -Reinigung. Dazu gehören beispielsweise Waschmittel für Textilien, Teppiche, oder Naturfasern, für die nach der vorliegenden Erfindung die Bezeichnung Waschmittel verwendet wird. Dazu gehören beispielsweise auch Geschirrspülmittel für Geschirrspülmaschinen oder manuelle Geschirrspülmittel oder Reiniger für harte Oberflächen wie Metall, Glas, Porzellan, Keramik, Kacheln, Stein, lackierte Oberflächen, Kunststoffe, Holz oder Leder; für solche wird nach der vorliegenden Erfindung die Bezeichnung Reinigungsmittel verwendet. Jegliche Wasch- oder Reinigungsmittelart stellt eine Ausführungsform der vorliegenden Erfindung dar, sofern sie um ein erfindungsgemäßes Protein, Proteinfragment, Fusionsprotein oder Derivat bereichert ist.

Ausführungsformen der vorliegenden Erfindung umfassen alle nach den Stand der Technik etablierten und/oder alle zweckmäßigen Darreichungsformen der erfindungsgemäßen Wasch- oder Reinigungsmittel. Dazu zählen beispielsweise feste, pulverförmige, flüssige, gelförmige oder pastöse Mittel, gegebenenfalls auch aus mehreren Phasen, komprimiert oder nicht komprimiert; ferner gehören beispielsweise dazu: Extrudate, Granulate, Tabletten oder Pouches, sowohl in Großgebinden als auch portionsweise abgepackt.

In einer bevorzugten Ausführungsform enthalten die erfindungsgemäßen Wasch-oder Reinigungsmittel die oben beschriebenen, erfindungsgemäßen oder erfindungsgemäß zu verwendenden Proteine in einer Menge von 0,0001 μg bis 480 mg, vorzugsweise von 0,005 μg bis 420 mg, besonders bevorzugt von 0,02 μg bis 360 mg, ganz besonders bevorzugt von 0,05 μg bis 240 mg pro Gramm des Mittels.

Neben einem erfindungsgemäßen Protein enthält ein erfindungsgemäßes Wasch-oder Reinigungsmittel gegebenenfalls weitere Inhaltsstoffe wie Enzymstabilisatoren, Tenside, z. B. nichtionische, anionische und/oder amphotere Tenside, und/oder Bleichmittel, und/oder Builder, sowie gegebenenfalls weitere übliche Inhaltsstoffe, die im folgenden ausgeführt werden.

Zu den für Wasch- und Reinigungsmitteln üblichen Inhaltsstoffen werden im allgemeinen auch wasch-, beziehungsweise reinigungsaktive Enzyme gerechnet.

Somit stellen Wasch- oder Reinigungsmittel, die über ein oben beschriebenes, erfindungsgemäßes Protein hinaus durch zusätzlich weitere Enzyme gekennzeichnet sind, bevorzugte Ausführungsformen der vorliegenden Erfindung dar. Dazu gehören insbesondere andere Esterasen, Proteasen Amylasen, Cellulasen, Hemicellulasen wie beispielsweise β-Glucanasen, Oxidoreductasen wie beispielsweise Laccasen, Cutinasen und/oder Lipasen, aber auch Esterasen und alle anderen Enzyme, die aus dem Stand der Technik für dieses Einsatzgebiet beschrieben sind.

Enzyme wie Proteasen, Amylasen, Lipasen oder Cellulasen werden seit Jahrzehnten als aktive Komponenten in Wasch- und Reinigungsmitteln verwendet. Ihr jeweiliger Beitrag zur Wasch-, beziehungsweise Reinigungsleistung des betreffenden Mittels ist im Fall von Proteasen die Fähigkeit zum Abbau proteinhaltiger Verunreinigungen, im Fall von Amylase der Abbau von stärkehaltigen Verunreinigungen und im Fall von Lipase die fettspaltende Aktivität. Cellulasen werden über ihre schmutzentfernende, das heißt primäre Wasch-, beziehungsweise Reinigungsleistung hinaus, insbesondere wegen ihres Beitrags zur Sekundärwaschleistung eines Waschmittels und wegen ihrer Faserwirkung auf Textilien bevorzugt in Waschmitteln eingesetzt. Die jeweiligen Hydrolyseprodukte werden von den übrigen Wasch- oder Reinigungsmittel-Bestandteilen angegriffen, gelöst, emulgiert oder suspendiert oder aufgrund ihrer höheren Löslichkeit mit der Waschflotte ausgeschwemmt, so dass es günstigenfalls zu Synergieeffekten zwischen den Enzymen und den übrigen Bestandteilen kommt.

Einen dem Beitrag zur Sekundärwaschleistung eines Waschmittels durch Cellulase vergleichbaren Effekt können Proteasen auf natürliche Fasern, insbesondere auf Wolle oder Seide ausüben. Durch ihre Wirkung auf die Oberflächenstruktur solcher Gewebe können sie einen glättenden Einfluss auf das Material ausüben und damit dem Verfilzen entgegenwirken.

Weitere Enzyme erweitern die Reinigungsleistung entsprechender Mittel um ihre jeweils spezifische enzymatische Leistung. Dazu gehören beispielsweise Hemicellulasen wie beispielsweise β-Glucanasen, Oxidoreduktasen wie zum Beispiel Laccasen oder Pektin-lösende Enzyme, die insbesondere in Spezialwaschmitteln zum Einsatz kommen.

Zur Verwendung in erfindungsgemäßen Wasch- oder Reinigungsmitteln kommen in erster Linie aus Mikroorganismen, wie Bakterien oder Pilzen, gewonnene Enzyme in Frage. Sie werden in bekannter Weise durch Fermentationsprozesse aus geeigneten Mikroorganismen gewonnen.

Ein erfindungsgemäßes Protein und/oder andere enthaltene Proteine können besonders während der Lagerung durch Stabilisatoren beispielsweise vor Denaturierung, Zerfall oder Inaktivierung, etwa durch physikalische Einflüsse, Oxidation oder proteolytische Spaltung geschützt werden. Dies gilt für alle erfindungsgemäßen Mittel, insbesondere Wasch- und Reinigungsmittel.

Eine Gruppe von Stabilisatoren sind reversible Proteaseinhibitoren, die bei Verdünnung des Mittels in der Waschflotte abdissozieren. Benzamidin-Hydrochlorid und Leupeptin sind für diesen Zweck etabliert. Häufig werden Borax, Borsäuren, Boronsäuren oder deren Salze oder Ester verwendet, darunter vor allem Derivate mit aromatischen Gruppen, ortho-substituierte, meta-substituierte und para-substituierte Phenylboronsäuren, beziehungsweise deren Salze oder Ester. Peptidaldehyde, das heißt Oligopeptide mit reduziertem C-Terminus, und zwar solche aus 2 - 50 Monomeren, werden zur reversiblen Inhibierung von Wasch- und Reinigungsmittelproteasen eingesetzt. Zu den peptidischen reversiblen Proteaseinhibitoren gehört unter anderem Ovomucoid. Beispielsweise können spezifische, reversible Peptid-Inhibitoren für die Protease Subtilisin zur Verwendung in Protease-haltigen Mitteln und entsprechende Fusionsproteine aus Protease und Inhibitor eingesetzt werden.

Weitere Enzymstabilisatoren sind Aminoalkohole wie Mono-, Di-, Triethanol- und -propanolamin und deren Mischungen, aliphatische Carbonsäuren bis zu $C_{12}$, wie Bernsteinsäure, andere Dicarbonsäuren oder Salze der genannten Säuren. Im Stand der Technik werden für diesen Zweck endgruppenverschlossene Fettsäureamidalkoxylate offenbart. Bestimmte als Builder eingesetzte organische Säuren vermögen, zusätzlich ein enthaltenes Enzym zu stabilisieren.

Niedere aliphatische Alkohole, vor allem aber Polyole, wie beispielsweise Glycerin, Ethylenglykol, Propylenglykol oder Sorbit sind weitere häufig eingesetzte Enzymstabilisatoren. Ebenso werden Calciumsalze verwendet, wie beispielsweise Calciumacetat oder Calcium-Formiat, und Magnesiumsalze.

Polyamid-Oligomere oder polymere Verbindungen wie Lignin, wasserlösliche Vinyl-Copolymere oder Cellulose-Ether, Acryl-Polymere und/oder Polyamide stabilisieren die Enzym-Präparation unter anderem gegenüber physikalischen Einflüssen oder pH-Wert-Schwankungen. Polyamin-N-Oxid-enthaltende Polymere wirken gleichzeitig als Enzymstabilisatoren und als Farbübertragungsinhibitoren. Andere polymere Stabilisatoren sind die linearen $C_8$-$C_{18}$ Polyoxyalkylene. Alkylpolyglycoside könnten die enzymatischen Komponenten des erfindungsgemäßen Mittels stabilisieren und sogar in ihrer Leistung steigern. Vernetzte N-haltige Verbindungen erfüllen eine Doppelfunktion als Soil-release-Agentien und als Enzym-Stabilisatoren. Hydrophobes, nichtionisches Polymer wirkt in einer Mischung mit anderen Stabilisatoren stabilisierend auf eine Cellulase, so dass sich solche oder ähnliche Bestandteile auch für das erfindungswesentliche Enzym eignen könnten.

Reduktionsmittel und Antioxidantien erhöhen die Stabilität der Enzyme gegenüber oxidativem Zerfall. Schwefelhaltige Reduktionsmittel sind bekannt. Andere Beispiele sind Natrium-Sulfit und reduzierende Zucker.

Vielfach werden auch Kombinatonen von Stabilisatoren verwendet, beispielsweise aus Polyolen, Borsäure und/oder Borax, die Kombination von Borsäure oder Borat, reduzierenden Salzen und Bernsteinsäure oder anderen Dicarbonsäuren oder die Kombination von Borsäure oder Borat mit Polyolen oder Polyaminoverbindungen und mit reduzierenden Salzen offenbart. Die Wirkung von Peptid-Aldehyd-Stabilisatoren wird durch die Kombination mit Borsäure und/oder Borsäurederivaten und Polyolen gesteigert und durch die zusätzliche Verwendung von Calcium-Ionen noch weiter verstärkt.

Mittel mit stabilisierten Enzymaktivitäten stellen bevorzugte Ausführungsformen der vorliegenden Erfindung dar. Besonders bevorzugt sind solche mit Enzymen, die auf mehrere der dargestellten Weisen stabilisiert sind.

Da erfindungsgemäße Mittel in allen denkbaren Formen angeboten werden können, stellen erfindungsgemäße Enzyme, beziehungsweise Proteine in allen für die Zugabe zu den jeweiligen Mitteln zweckmäßigen Formulierungen jeweilige Ausführungsformen der vorliegenden Erfindung dar. Dazu gehören beispielsweise flüssige Formulierungen, feste Granulate oder Kapseln.

Die verkapselte Form bietet sich an, um die Enzyme oder andere Inhaltsstoffe vor anderen Bestandteilen, wie beispielsweise Bleichmitteln, zu schützen oder um eine kontrollierte Freisetzung (controlled release) zu ermöglichen. Je nach der Größe dieser Kapseln wird nach Milli-, Mikro- und Nanokapseln unterschieden, wobei Mikrokapseln für Enzyme besonders bevorzugt sind. Eine mögliche Verkapselungsmethode besteht darin, dass die Proteine, ausgehend von einer Mischung der Proteinlösung mit einer Lösung oder Suspension von Stärke oder einem Stärkederivat, in dieser Substanz verkapselt werden.

Im Fall fester Mittel können die Proteine beispielsweise in getrockneter, granulierter und/oder verkapselter Form eingesetzt werden. Sie können separat, das heißt als eigene Phase, oder mit anderen Bestandteilen zusammen in derselben Phase, mit oder ohne Kompaktierung zugesetzt werden. Sollen mikroverkapselte Enzyme in fester Form verarbeitet werden, so kann das Wasser mit aus dem Stand der Technik bekannten Verfahren aus den sich aus der Aufarbeitung ergebenden wäßrigen Lösungen entfernt werden, wie Sprühtrocknung, Abzentrifugieren oder durch Umsolubilisieren. Die auf diese Weise erhaltenen Teilchen haben üblicherweise eine Teilchengröße zwischen 50 und 200 $\mu$m.

Flüssigen, gelförmigen oder pastösen erfindungsgemäßen Mitteln können die Enzyme, und auch das erfindungsgemäße Protein ausgehend von einer nach dem Stand der Technik durchgeführten Proteingewinnung und Präparation in konzentrierter wäßriger oder nichtwäßriger Lösung, Suspension oder Emulsion zugesetzt werden, aber auch in Gelform oder verkapselt oder als getrocknetes Pulver. Derartige erfindungsgemäße Wasch- oder Reinigungsmittel werden in der Regel durch einfaches Mischen der Inhaltsstoffe hergestellt, die in Substanz oder als Lösung in einen automatischen Mischer gegeben werden können.

Neben der primären Waschleistung können die in Waschmitteln enthaltenen Esterasen ferner die Funktion erfüllen, andere Bestandteile durch esterolytische Spaltung zu aktivieren oder nach entsprechender Einwirkzeit zu inaktivieren. Vergleichbare regulatorische Funktionen sind auch über das erfindungsgemäße Protein möglich. Eine Ausführungsform der vorliegenden Erfindung sind ferner solche Mittel mit Kapseln aus Esterase-sensitivem Material, welche beispielsweise von erfindungsgemäßen Proteinen zu einem beabsichtigten Zeitpunkt hydrolysiert werden und ihren Inhalt freisetzen. Ein vergleichbarer Effekt kann auch bei anderen mehrphasigen Mitteln erzielt werden.

Eine weitere Ausführungsform stellen Wasch- oder Reinigungsmittel zur Behandlung von Textilrohstoffen oder zur Textilpflege dar, die dadurch gekennzeichnet sind, dass sie allein oder neben anderen aktiven Inhaltsstoffen eines der oben beschriebenen, erfindungsgemäßen Proteine enthalten, insbesondere für Fasern oder Textilien mit Kunstfaser-Bestandteilen und ganz besonders für solche mit Polyester.

Auch künstliche Fasern, wie beispielsweise Polyester, zeichnen sich durch eine charakteristische Oberflächenstruktur aus. Diese kann langfristig, insbesondere bei der Unterwerfung unter mehrere Waschprozesse, zu unerwünschten Effekten, wie etwa Verfilzung, dem sogenannten Pilling, neigen. Zur Vermeidung solcher Effekte werden entweder die Rohstoffe oder der fertige Stoff, das Textil bzw. die Faser, mit erfindungsgemäßen Mitteln behandelt, welche beispielsweise dazu beitragen, die Oberflächenstruktur zu glätten und damit einem Verfilzen entgegenwirken.

Bevorzugt werden die erfindungsgemäßen Mittel zur Verbesserung des Aussehens und/oder der Oberflächenstruktur bereits verfilzter Fasern, insbesondere von Polyesterfasern, verwendet. Die erfindungsgemäßen Enzyme sind in der Lage, die Esterbindungen der vefilzten Kunstfasern zu spalten, und damit das Verfilzen des Gewebes, der Faser bzw. des Textils rückgangig zu machen (Depilling), von vornherein zu verhindern, aufzuhalten, deutlich zu verlangsamen und/oder sogar vollständig zu stoppen.

In einer bevorzugten Ausführungsform ist das Mittel mit einer erfindungsgemäßen Esterase so konzipiert, dass es regelmäßig als Pflegemittel verwendet werden kann, beispielsweise indem es dem Waschprozess zugesetzt, nach dem Waschen angewendet oder unabhängig vor dem Waschen appliziert wird. Der gewünschte Effekt besteht darin, eine glatte Oberflächenstruktur des Textils über einen langen Zeitraum zu erhalten und/oder Schädigungen des Gewebes vorzubeugen und/oder zu verringern.

Einen eigenen Erfindungsgegenstand stellen Verfahren zur maschinellen Reinigung von Textilien oder von harten Oberflächen dar, die dadurch gekennzeichnet sind, dass in wenigstens einem der Verfahrensschritte ein oben beschriebenes, erfindungsgemäßes Protein, Proteinfragment, Fusionsprotein oder Derivat aktiv wird, insbesondere in einer Menge von 0,01 $\mu$g bis 96 g, vorzugsweise von 0,05 $\mu$g bis 72 g, besonders bevorzugt von 0,1 $\mu$g bis 48 g und ganz besonders bevorzugt von 0,5 $\mu$g bis 24 g pro Anwendung.

Hierunter fallen sowohl manuelle als auch maschinelle Verfahren, wobei maschinelle Verfahren aufgrund ihrer präziseren Steuerbarkeit, was beispielsweise die eingesetzten Mengen und Einwirkzeiten angeht, bevorzugt sind.

Verfahren zur Reinigung von Textilien zeichnen sich im allgemeinen dadurch aus, dass in mehreren Verfahrensschritten verschiedene reinigungsaktive Substanzen auf das Reinigungsgut aufgebracht und nach der Einwirkzeit abgewaschen werden, oder dass das Reinigungsgut in sonstiger Weise mit einem Waschmittel oder einer Lösung dieses Mittels behandelt wird. Das gleiche gilt für Verfahren zur Reinigung von allen anderen Materialien als Textilien, welche unter dem Begriff harte Oberflächen zusammengefasst werden. Alle denkbaren Wasch- oder Reinigungsverfahren können in wenigstens einem der Verfahrensschritte um erfindungsgemäße Proteine bereichert werden, und stellen dann Ausführungsformen der vorliegenden Erfindung dar.

Ein einzelner Teilschritt eines solchen Verfahrens zur maschinellen Reinigung von Textilien kann darin bestehen, dass gewünschtenfalls neben stabilisierenden Verbindungen, Salzen oder Puffersubstanzen als einzige aktive Komponente ein erfindungsgemäßes Enzym aufgebracht wird. Dies stellt eine besonders bevorzugte Ausführungsform der vorliegenden Erfindung dar.

In einer weiteren bevorzugten Ausführungsform solcher Verfahren werden die betreffenden erfindungsgemäßen Enzyme im Rahmen einer der oben ausgeführten Rezepturen für erfindungsgemäße Wasch- beziehungsweise Reinigungsmittel bereitgestellt.

Bevorzugte Ausführungsformen dieses Erfindungsgegenstand stellen Verfahren zur Behandlung von Textilrohstoffen oder zur Textilpflege dar, die dadurch gekennzeichnet sind, dass in wenigstens einem der Verfahrensschritte ein oben beschriebenes, erfindungsgemäßes Protein aktiv wird, insbesondere für Textilrohstoffe, Fasern oder Textilien mit künstlichen Bestandteilen und ganz besonders für solche mit Kunstfasern, insbesondere Polyester.

Es kann sich dabei beispielsweise um Verfahren handeln, in denen Materialien zur Verarbeitung in Textilien vorbereitet werden, etwa zur Antifilzausrüstung, oder beispielsweise um Verfahren, welche die Reinigung getragener Textilien um eine pflegende Komponente bereichern. Es handelt sich in bevorzugten Ausführungsformen um Verfahren zur Behandlung von Textilrohstoffen, Fasern oder Textilien mit künstlichen Bestandteilen, insbesondere mit Kunstfasern, bevorzugt Polyester.

Einen eigenen Erfindungsgegenstand stellt die Verwendung eines oben beschriebenen, erfindungsgemäßen Proteins zur Reinigung von Textilien oder von harten Oberflächen dar.

Vorzugsweise gelten für diese Verwendung die oben ausgeführten Konzentrationsbereiche.

Denn erfindungsgemäße Proteine können, insbesondere entsprechend den oben beschriebenen Eigenschaften und den oben beschriebenen Verfahren dazu verwendet werden, um Textilien von störenden Knötchen oder Verfilzungen zu befreien (Depilling). Ausführungsformen stellen beispielsweise die Handwäsche oder die manuelle Entfernung von Flecken von Textilien oder von harten Oberflächen oder die Verwendung im Zusammenhang mit einem maschinellen Verfahren.

In einer bevorzugten Ausführungsform dieser Verwendung werden die betreffenden erfindungsgemäßen Enzyme im Rahmen einer der oben ausgeführten Rezepturen für erfindungsgemäße Mittel, vorzugsweise Wasch-, beziehungsweise Reinigungsmittel bereitgestellt.

Die Verwendung der erfindungsgemäßen Proteine in Wasch- und Reinigungsmitteln kann weiterhin den satinähnlichen Glanz reduzieren, der bei Polyesterfasern zu beobachten ist und von den Kunden als eher billig angesehen wird. Weiterhin kann die Entstehung von glänzenden Stellen auf Kunstfasern beim Waschen reduziert oder sogar im Wesentlichen verhindert werden. Bereits durch starke Beanspruchung der Fasern, z.B. Reibung oder Scheuern, entstandene glänzende Stellen werden durch die Verwendung von Wasch- und Reinigungsmittel, welche die erfindungsgemäßen Enzyme erhalten, reduziert.

Weiterhin können die erfindungsgemäßen Enzyme verwendet werden, um die Redeposition, d.h. die Anhaftung von Schmutz aus der Waschflotte während des Waschprozesses zu vermindern und/oder ganz zu verhindern.

Darüber hinaus ermöglicht es die Verwendung von erfindungsgemäßen Enzymen in Wasch- und Reinigungsmitteln eine Verbesserung der Reinigungsleistung insgesamt zu erreichen.

Weiterhin können die para-Nitrobenzylesterasen zur chemischen Synthese oder Aufreinigung bei einer chemischen Synthese eingesetzt werden. Da Enzyme in der Regel stereoselektive katalytische Zentren besitzen, ist es möglich, die genannten Enzyme zur Trennung von Racematen bzw. zur stereoselektiven Synthese einzusetzen.

Eine weitere Ausführungsform dieses Erfindungsgegenstands stellt die Verwendung eines oben beschriebenen, erfindungsgemäßen Proteins zur Behandlung von natürlichen oder künstlichen Rohstoffen dar, insbesondere zur Oberflächenbehandlung.

Vorzugsweise erfolgt diese Verwendung im Rahmen entsprechender Mittel oder Verfahren. Sie ist beispielsweise dann erforderlich, wenn Rohstoffe von bestimmten Verunreinigungen befreit werden sollen. Hierunter sind in erster Linie zu erhaltende künstliche Rohstoffe, wie Polyesterverbindungen, aber auch biotechnologisch über Fermentationen hergestellte Stoffe wie beispielsweise Antibiotika zu verstehen.

Die Verwendung von p-Nitrobenzylesterasen, bevorzugt erhältlich aus Bakterien der Gattung *Bacillus,* in Verfahren zur Herstellung und/oder Aufreinigung von Polyestern, insbesondere Polyalkylenterephthalaten, stellt ein weiterer bevorzugter Erfindungsgegenstand dar. Bei der Herstellung von Polyalkylenterephthalaten, insbesondere Polyethylenterephthalaten (PET), wird die Ausbeute an Polymer durch eine Nebenreaktion beeinflusst, die zu einer Bildung von zyklischen Oligomeren führt. Durch die erfindungsgemäße Verwendung ist es möglich, die gebildeten zyklischen Oligomere wieder rückgängig zu machen und die Monomere für die weitere Reaktion zur Verfügung zu stellen. Die Produkte der Nebenreaktion werden somit zurückgedrängt und eine höhere Ausbeute an gewünschtem Polymer erzielt.

Eine weitere Ausführungsform dieses Erfindungsgegenstands stellt die Verwendung eines oben beschriebenen, erfindungsgemäßen Proteins zur Gewinnung oder Behandlung von Rohmaterialien oder Zwischenprodukten in der Textilherstellung, insbesondere zum Entfernen von Schutzschichten auf Geweben dar.

Vorzugsweise erfolgt diese Verwendung im Rahmen entsprechender Mittel oder Verfahren dar. Ein Beispiel für die Gewinnung oder Behandlung von Rohmaterialien oder Zwischenprodukten in der Textilherstellung ist die Aufarbeitung von Kunstfasern, Enzymatische Verfahren, beziehungsweise Verwendungen sind vergleichbaren chemischen Verfahren besonders hinsichtlich ihrer Umweltverträglickeit überlegen.

In einer bevorzugten Ausführungsform werden erfindungsgemäße Proteine dazu verwendet, um Schutzschichten von Textilien, insbesondere Zwischenprodukten oder Werkstoffen zu entfernen oder ihre Oberfläche zu glätten, bevor sie in einem nachfolgenden Verarbeitungsschritt weiterbearbeitet werden.

Eine weitere Ausführungsform dieses Erfindungsgegenstands stellt die Verwendung eines oben beschriebenen, erfindungsgemäßen Proteins zur Behandlung von

Textilrohstoffen oder zur Textilpflege dar, insbesondere zur Behandlung von Kunstfasern, insbesondere Polyestern, oder kunstfaserhaltigen Mischtextilien.

Vorzugsweise erfolgt diese Verwendung im Rahmen entsprechender Mittel oder Verfahren. Entsprechend dem oben Gesagten werden die betreffenden Textilrohstoffe durch die Esterase von Verunreinigungen befreit; außerdem kommen einem wenigstens zum Teil aus Protein bestehenden Material die oberflächenglättenden und pflegenden Eigenschaften des esterolytischen Enzyms zugute. Aus diesem Grunde ist auch die Verwendung zur Pflege der betreffenden Materialien umfasst. Insbesondere wird deshalb die Oberflächenbehandlung von Kunstfasern, insbesondere Polyestern, oder kunstfaserhaltigen Mischtextilien beansprucht. Dies gilt sowohl für die Herstellung für derartige Textilien, als auch für die Pflege während des Gebrauchs, beispielsweise im Zusammenhang mit der Textilreinigung (siehe oben).

Beispiele:

**1. Sequenzen der erfindungsgemäßen Esterasen**

Tabelle 1: Zuordnung der Sequenz-ID Nummern

| SEQ-ID No. | Organismus | Protein/DNA/RNA | Nr. des Datenbankeintrags NCBI | Esterase/Lipase |
|---|---|---|---|---|
| 1 | Bacillus subtilis | Protein | - | PNBE |

(fortgesetzt)

| SEQ-ID No. | Organismus | Protein/DNA/RNA | Nr. des Datenbankeintrags NCBI | Esterase/Lipase |
|---|---|---|---|---|
| 2 | Bacillus licheniformis | Protein | - | PNBE |
| 3 | Bacillus subtilis | DNA/RNA | - | PNBE |
| 4 | Bacillus licheniformis | DNA/RNA | - | PNBE |
| 5 | Bacillus subtilis | Präprotein | - | PNBE |
| 6 | Bacillus licheniformis | Präprotein | - | PNBE |
| 7 | Bacillus subtilis | DNA/RNA | - | PNBE |
| 8 | Bacillus subtilis | DNA/RNA | - | PNBE |
| 9 | Artificial | Signalpeptid | - | PNBE |
| 10 | Artificial | DNA/RNA | - | PNBE |
| 11 | Bacillus subtilis | Protein | gi\|1762126 | PNBE |
| 12 | Bacillus licheniformis DSM13 | Protein | gil52346943 | PNBE |
| 13 | Bacillus subtilis | Protein | gi\|7546321 | PNBE |
| 14 | Bacillus subtilis | Protein | gi\|468046 | PNBE |
| 15 | Bacillus subtilis | Protein | gil1762126 | PNBE |
| 16 | Bacillus subtilis | Protein | gi\|1495277 | PNBE |
| 17 | Bacillus subtilis | Protein | gi\|1945688 | PNBE |
| 18 | Bacillus subtilis | Protein | gi\|2635952 | PNBE |
| 19 | Bacillus licheniformis ATCC 14580 | Protein | gi\|52002286 | PNBE |
| 20 | Staphylococcus epidermidis ATCC 12228 | Protein | gi\|27316488 | PNBE |
| 21 | Staphylococcus aureus | Protein | gi\|57286454 | PNBE |
| 22 | Streptomyces avermitilis MA-4680 | Protein | gi\|29611030 | PNBE |
| 23 | Caulobacterium crescentus CB15 | Protein | gi\|13422044 | PNBE |
| 24 | Clostridium acetobutylicum ATCC824 | Protein | gi\|14994366 | PNBE |
| 25 | Artificial | Protein | gi\|7546320 | PNBE |
| 26 | Burkholderia cepacia | Protein | gi\|67464317 | Lipase |
| 27 | Bacillus licheniformis DSM13 | DNA | _ | PNBE |

## 2. Bestimmung der Enzymaktivitäten

### 2.1 Esterase-Aktivitätsbestimmung mit *para*-Nitrophenyl-Estern

Der Nachweis der Esterase-Aktivität wurde standardmäßig mit *para*-Nitrophenyl-Acetat (pNPA) bestimmt. Dazu wurde eine 400 mM Stammlösung mit DMSO angesetzt. 980 µL eines 50 mM Tris-HCl-Puffer (pH 8,0) wurden mit 10 µL in DMSO gelösten *para*-Nitrophenyl-Acetat vermengt. Die Reaktion wurde - wenn nicht anders beschrieben - bei Raumtemperatur durchgeführt und durch Zugabe von 10 µL Enzymlösung gestartet. Anschließend wurde die Absorptionszunahme bei 410 nm vermessen (UV $MC^2$, Fa. SAFAS, Monaco). Die Enzymaktivität wurde mit einem Molaren Extinktionskoeffizienten von 17100 $[M^{-1} \cdot cm^{-1}]$ errechnet, wobei 1 Unit als die Enzymmenge definiert wurde, die 1 μmol *p*-Nitrophenol pro Minute freisetzte.

**2.2 Esterase-Aktivitätsbestimmung mit Phenolrot**

Für die biochemische Reinigung der Dialkylphthalat-Hydrolase wurden esterolytische Aktivitätsbestimmungen gegenüber Diethylterepthtalat (DET) als Substrat mit einem photometrisches Messverfahren angewendet, welches die Ansäuerung des Testsystems mittels pH-Indikator anzeigt. Grundlage dieses Testsystems ist die gleiche Protonenaffinität des eingesetzten Puffers (EPPS-Puffer; pKa = 8,0) und des eingesetzten pH-Indikators (Phenolrot; $pK_a = 8{,}0$), die einen linearen Verlauf der Absorptionsabnahme bei 560nm ermöglichen. Zur Errechnung der Aktivität wurden die folgenden Formeln 2,1 und 2.2 mit Q="Pufferfaktor": $Cp_{uffer}$=Endkonzentration an Puffer; $C_{indikator}$=Endkonzentration an Indikator; $\Delta\varepsilon_{560nm}$: Differenz von deprotonierter Form und protonierter Form des Phenolrots; A: Aktivität der Esterase; dE/dt: Extinktionsabnahme gegenüber eines Zeitintervalls; $V_{Reaktion}$: Reaktionsvolumen verwendet:

$$Q = \frac{c_{Puffer}}{c_{Indikator}} * \frac{1}{\Delta\varepsilon_{560nm} * l} \qquad (2.1)$$

$$A = \left(\mu mol * min^{-1}\right) = \frac{dE}{dt} * Q * V_{Reaktion} * 10^{-6} \qquad (2.2)$$

Die Enzymaktivität wurde mit einem empirisch ermittelten Molaren Extinktionskoeffizienten von 58000 $[M^{-1} \cdot cm^{-1}]$ für die deprotonierte und von 100 $[M^{-1} \cdot cm^{-1}]$ für die protonierte Form errechnet, wobei 1 Unit als die Enzymmenge definiert wurde, die 1 μmol Säure pro Minute freisetzte.

**2.3 Bestimmung der Esterase-Aktivität mittels Titration**

Enzymaktivitäten der Esterasen gegenüber verschiedenen Substraten der Phthalat-Familie (Abb. 4) wurden mit dem Titrationsapparat 702 $S_{TAT}$ (Fa. $M_{ETROHM}$ LDT., Schweiz) bestimmt. Die Phthalate wurden mit 0,5g Triton X100 in Diethylether gelöst. Der Diethylether wurde anschließend unter Stickstoffbegasung entfernt. Es wurde die 50 ml Puffer (2 mM Tris/HCl, pH 9) zugegeben, mittels Ultraschall homogenisiert und in unterschiedlichen Endkonzentrationen (0,3 mM bis 100 mM) in einem Gesamtreaktionsvolumen von 2 mL (einschließlich Enzym) eingesetzt. Der Reaktionspuffer wurde für mindestens 10 Minuten bei 30°C vorinkubiert. Nach Zugabe des Enzyms wurde der NaOH-Verbrauch (Konzentrationsbestimmung durch dreimalige Titerbestimmung mit Oxalsäure) bei einer Reaktionstemperatur von 30°C aufgezeichnet. Eine Unit wurde als die Enzymaktivität definiert, die 1 μmol Säure pro Minute freisetzte.

**2.4 Bestimmung der Esterase-Aktivität durch Hydrolyse von Bis-(p-methylbenzoesäure)-Ethylengykolester)**

**2.4.1 Synthese von (Bis-(*p*-methylbenzoesäure)-Ethylenglykolester)**

Bis-(*p*-methylbenzoesäure)-ethylenglykolester wurde durch die Veresterung von 4-Methylbenzoylchlorid und Ethylenglykol synthetisiert (vgl. Abb. 1). Ethylenglykol und Pyridin werden dabei im Rundkolben mit Rückflusskühler vorgelegt und 4-Methylbenzoylchlorid unter Eiskühlung zugetropft. Nach Stehen lassen des Ansatzes für 16 Stunden wurde der erhaltene feste Ester abfiltriert und in 70% Ethanol 30% Wasser rekristallisiert. Anschließend wurde der Feststoff mit 70% Ethanol gewaschen und für 24 Stunden lyophilisiert.

**2.4.2 Bestimmung der enzymatischen Aktivität**

Die von den erfindungsgemäßen Enzymen katalysierte Spaltung des Bis-(*p*-Methylbenzoesäure)-ethylenglykolesters ergibt 2 Äquivalente 4-Methylbenzoesäure und Ethylenglykol (vgl. Abb. 2).

Für die Präparation des Enzym-Assays wird die gewünschte Menge an Substrat mit 0,5 g Triton X100 versetzt und in 50mL Ethanol (99%ig, vergällt) unter Erwärmung gelöst. Sobald alles Substrat gelöst ist, wird die Lösung in 50 mL Puffer (2 mM Tris/HCl-Puffer; pH 9,0) unter starken Rühren mittels Ultra-Turrax hinein gegeben. Anschließend wurde diese Lösung für mindestens 12 Stunden bei Raumtemperatur gerührt, um einen Großteil des eingesetzten Ethanols zu entfernen. Die so hergestellte Substratlösung wurde direkt titrimetrisch vermessen. Der Reaktionspuffer wurde für mindestens 10 Minuten bei 30°C vorinkubiert.

Es wurden unterschiedliche Endkonzentrationen (0,67 mM bis 50 mM) in einem Gesamtreaktionsvolumen von 2 mL einschließlich Enzym eingesetzt. Nach Zugabe des Enzyms wurde der NaOH-Verbrauch (Konzentrationsbestimmung durch dreimalige Titerbestimmung mit Oxalsäure mit Hilfe des Titrationsapparats 702 $S_{TAT}$ (Fa. $M_{ETROHM}$ LDT., Schweiz)) bei einer Reaktionstemperatur von 30°C aufgezeichnet. Eine Unit wurde als die Enzymaktivität definiert, die 1 μmol Säure pro Minute freisetzte.

Bei diesem und den weiteren Beispielen werden folgende Abkürzungen für die getesteten Esterasen verwendet: Enzym gemäß SeqID No. 12 (pNB-Est13), Enzym gemäß SeqID No, 2 (pNB-Est19), Enzym gemäß SeqID No. 1 (pNB-Est17) Die für die genannten Esterasen erhaltenen Daten in Tabelle 2 wurden durch titrimetrische Messungen ermittelt und stellen Mittelwerte aus drei unabhängig voneinander durchgeführten Messungen mit einer relativen Standardabweichung von unter 5% dar.

Tabelle 2: Kinetische Daten der Hydrolyse des Bis-(*p*-Methylbenzoesäure)-ethylenglykolesters durch *para*-Nitrobenzyl-Esterasen *p*NB-Est17, *p*NB-Est19 und *p*NB-Est13

| Bis-(*p*-Methylbenzoesäure)-ethylenglykolester | **Vmax [μmol/min]** | **$k_M$ [mM]** | **$k_{cat}$ (1/s]** | **$k_{cat}$ / $k_M$ [1/mM*s]** | **Spezifische Aktivität [umol/min*mg]** |
|---|---|---|---|---|---|
| *Enzym gemäß SeqID No. 1* (*p*NB-Est17) | 0,642 | 9,83 | 5177 | 527 | 1,60 |
| *Enzym gemäß SeqID No, 2* (*p*NB-Est19) | 0,385 | 3,59 | 3838 | 1070 | 1,19 |
| *Enzym gemäß SeqID No. 12* (*p*NB-Est13) | 1,801 | 11,21 | 9358 | 835 | 2,87 |

**3. Untersuchung der Substratpezifität gegenüber *para*-Nitrophenylestern und Triglyceriden mit unterschiedlichen Kettenlängen**

Zur Bestimmung von Kettenlängen-Spezifitäten der untersuchten Esterasen wurden *p*-Nitrophenyl-Ester mit unterschiedlicher Kettenlänge als Substrat für die spektroskopischen Aktivitätsmessungen verwendet. Dazu wurden die *p*-Nitrophenylester in 10 mL Isopropanol gelöst und mit 90 mL Phosphat-Puffer (pH 8, Dinatriumhydrogenphosphat und Kaliumdihydrogenphosphat) versetzt. Die Endkonzentration der Substrate lag bei 0,8 mM. Jeweils 990 μL des Puffer-Substrat-Gemisches wurde mit 10 μL der zu vermessenden Enzymprobe vermengt und eine Minute bei Raumtemperatur inkubiert. Anschließend wurde die Absorptionszunahme bei 410 nm vermessen (UV $Mc^2$, Fa. SAFAS, Monaco). Die Enzymaktivität wurde mit einem Molaren Extinktionskoeffizienten von 17100 [$M^{-1} \cdot cm^{-1}$] errechnet, wobei 1 Unit als die Enzymmenge definiert wurde, die 1 μmol *p*-Nitrophenol pro Minute freisetzte.

Die in Tabelle 3 dargestellten Werte sind Mittelwerte von drei unabhängig durchgeführten Messungen mit einer relativen Standardabweichung von unter 10%.

Tabelle 3: Substratspezifitäten der *p*NB-Est13, *p*NB-Est17 und *p*NB-Est19 bei unterschiedlichen Kettenlängen der *para*-Nitrophenylester beschriebenen Bedingungen.

| **Substrat** | **spezifische Aktivtät [U/mg]** | | |
|---|---|---|---|
| | ***p*NB-Est13** | ***p*NB-Est17** | ***p*NB-Est19** |
| ***p*-NP-Butyrat** | 22,4 | 196,3 | 234,8 |
| ***p*-NP-Caproat** | 50,5 | 102,7 | 249,6 |
| ***p*-NP-Caprylat** | 13,0 | 73,2 | 83,6 |
| ***p*-NP-Caprat** | 4,4 | 10,2 | 3,9 |
| ***p*-NP-Laurat** | 1,3 | 3,4 | 4,0 |
| ***p*-NP-Myristat** | < 1 | < 1 | 1,3 |
| ***p*-NP-Palmitat** | < 1 | < 1 | < 1 |
| ***p*-NP-Stearat** | < 1 | < 1 | < 1 |

**4. Analytischer Nachweis der Hydrolyseprodukte von verschiedenen Dialkylphthalaten nach Inkubation mit den *para*-Nitrobenzyl-Esterasen *p*NB-Est17, *p*NB-Est19 und *p*NB-Est13**

Die esterolytische Aktivität der untersuchten *p*NB-Esterasen gegenüber unterschiedlichen Dialkylphthalaten wurde durch den Nachweis der Spaltprodukte verifiziert. Der Nachweis erfolgte sowohl über Dünnschichtchromatographie als auch durch gekoppelte Gaschromatographie / Massenspektroskopie (GC/MS). Die $R_F$-Werte der Reaktionsedukte und Produkte nach einer durchgeführten Dünnschichtchromatographie sind in der nachfolgenden Tabelle 3 aufgeführt. Die Detektion eines bzw. zweier neuer spots deutete bereits auf eine Hydrolyse hin. Die jeweils mit durchgeführten Blindproben wiesen keine zusätzlichen *spots* auf.

Als Laufmittel in vorgesättigter DC-Kammer wurde 90% Dichlormethan / 10% Methanol verwendet; die Trennung der Substanzen erfolgte auf mit Silica-Gel 60 $F_{254}$ beschichteten Folien; die Detektion der einzelnen Spots wurde unter UV-Licht vorgenommen; die Substratkonzentration im Ansatz betrug 6,67 mM, die Inkubationsdauer lag bei 2 h, Raumtemperatur

Tabelle 4: Charakteristischen Lauflängen ($R_F$-Werte) der Edukte und Produkte ermittelt durch dünnschichtchromatographische Analysen der Reaktionsansätze mit *p*NB-Esterasen.

| Substrat | $R_F$-Wert Substrat | $R_F$-Wert Produkt 1 | $R_F$-Wert Produkt 2 |
|---|---|---|---|
| Dimethylterephthalat | 0,78 | 0,18 | - |
| Diethylterephthalat | 0,75 | 0,21 | - |
| Dimethylisophthalat | 0,78 | 0,23 | - |
| Dimethylphthalat | 0,70 | 0,07 | - |
| Diethylphthalat | 0,73 | 0,13 | - |
| Dibutylphthalate | 0,74 | 0,13 | - |
| Phenylbenzoat | 0,76 | 0,45 | 0,25 |

Nach der GC/MS-Analyse der Reaktionsansätze erfolgte die Zuordnung der Massenpeaks der Spaltprodukte durch Vergleich mit der WILEY-Datenbank und bestätigte die Hydrolyse, die schon in den dünnschichtchromatographischen Analysen beobachtet wurde. In der Abbildung 4 sind alle beobachteten Reaktionen schematisch dargestellt. Für jede enzymatische Reaktion wurden Blindproben vermessen, die aus Reaktionsansatz ohne Enzym bzw. Reaktionsansatz ohne Substrat bestanden. Es zeigte sich, dass bei allen untersuchten Phthalaten und Terephthalaten nur eine von zwei möglichen funktionellen Estergruppen hydrolysiert wird. Die freie Phthalsäure bzw. Terephthalsäure oder Isophthalsäure wurde in keinem Reaktionsansatz detektiert. Das jeweilige Reaktionsprodukt ist der Monoester der Ausgangsverbindung, sowie der jeweilige Alkanol. Als Substrat, welches nicht zu den Phthalaten gehört, wurde die Hydrolyse von Phenylbenzoat untersucht. Als Hydrolyseprodukte wurden Phenol und Benzoesäure detektiert.

**5. Ermittlung von Vmax, $k_M$, $k_{cat}$ und $k_{cat}/k_M$ der *para*-Nitrobenzyl-Esterasen *p*NB-Est17, *p*NB-Est19 und *p*NB-Est13 gegenüber unterschiedlichen Phthalaten und Terephthalaten**

Zur Bestimmung der kinetischen Konstanten wie $k_M$, $v_{max}$, $k_{cat}$ und $k_{cat}/k_M$ der drei *para*-Nitrobenzyl-Esterasen *p*NB-Est17, *p*NB-Est19 und *p*NB-Est13 gegenüber unterschiedlichen Phthalaten und Terephthalaten wurden titrimetrische Messungen durchgeführt. Bei unterschiedlichen Substratkonzentrationen wurden die jeweiligen Enzymaktivitäten bestimmt und nach MICHAELIS-MENTEN ausgewertet (s. Abb. 5 bis 7). Die ermittelten $k_M$- sowie vmax-Werte und die eingesetzten Enzymkonzentrationen wurden für die Berechnung der kinetischen Konstante ($k_{cat}$ bzw. $k_2$) und der katalytischen Effizienz ($k_{cat}/k_M$) herangezogen - unter der Annahme das die eingesetzte Enzymkonzentration gleich der Konzentration an Aktiv-Enzym ist. Die erhaltenen Werte sind jeweils tabellarisch zusammengefasst.

Die in Abbildung 5 bis 7 bzw. Tabelle 5 bis 7 dargestellten Werte sind Mittelwerte aus drei unabhängig voneinander durchgeführten titrimetrischen Messungen mit einer relativen Standardabweichung von unter 5%. Der Errechnung von $k_{cat}$ und $k_{cat}/k_M$ wurde aufgrund der Vorrausetzung vorgenommen, dass die eingesetzte Ezymkonzentration gleich der Konzentration an Aktiv-Enzym ist.

Bei allen drei untersuchten *Para*-Nitrobenzyl-Esterasen konnte der Monoester der Ausgangsverbindung mit dem jeweiligen Alkohol nachgewiesen werden. In keinem Fall konnte die freie Phthalsaäure bzw. Terephthalsäure oder Isophthalsäure nachgewiesen werden.

**Hydrolyse von Dimethylphthalsäureester mit unterschiedlicher Stellung (*ortho*, *meta*, *para*)**

Tabelle 5: $k_M$- und $V_{mac}$-Werte sowie $k_{cat}$ und $k_{cat}/_{Km}$ der Hydrolyse von Dimethylphthalsäure in *ortho-, meta* und *para*-Stellung, DMP=Dimethylphthalsäure; DMIP=Dimethylisophthalsäure; DMT=Dimethylterephthalsäure durch *p*NB-Est17, *p*NB-Est19 und *p*NB-Est13.

| **Substrat DMP** | ***p*NB-Est17** | ***p*NB-Est19** | ***p*NB-Est13** |
|---|---|---|---|
| $v_{Max}$ [U/mg] | 14,09 | 15,76 | 9,11 |
| $k_M$ [mM] | 27,70 | 20,35 | 8,68 |
| $k_{cat}$ [1/s] | 45672 | 50970 | 29735 |
| $K_{cat}$/km [1/mM*S] | 1649 | 2505 | 3427 |
| **Substrat DMIP** | ***p*NB-Est17** | ***p*NB-Est19** | ***p*NB-Est13** |
| $v_{Max}$[U/mg] | 11,99 | 8,55 | 27,67 |
| $k_M$ [mM] | 3,67 | 2,44 | 2,93 |
| $k_{cat}$ [1/s] | 38880 | 27637 | 90267 |
| $k_{cat}/k_M$ [1/mM*s] | 10594 | 11327 | 30808 |
| **Substrat DMT** | ***p*NB-Est17** | ***p*NB-Est19** | ***p*NB-Estl3** |
| $V_{max}$ [U/mg] | 18,52 | 15,69 | 4,53 |
| $k_M$ [mM] | 5,99 | 13,56 | 6,87 |
| $k_{cat}$ [1/s] | 60028 | 50718 | 14784 |
| $k_{cat}/k_M$ [1/mM*$_S$] | 10021 | 3741 | 2152 |

**Hydrolyse von Phthalsäureester mit unterschiedlicher Kettenlänge (*ortho*)**

Tabelle 6: $k_M$- und $V_{max}$-Werte sowie $k_{cat}$ und $k_{cat}/k_m$ der Hydrolyse von Phthalsäureestern in ortho-Stellung mit unterschiedlicher Alkylkettenlänge (DMP = Dimethylphthalsäure: DEP = Diethylphthalsäure: DBP = Dibutylphthalsäure) durch *p*NB-Est17. *p*NB-Est19 und *p*NB-Est13.

| **Substrat DMP** | ***p*NB-Est17** | ***p*NB-Est19** | ***p*NB-Est13** |
|---|---|---|---|
| $V_{Max}$ [U/mg] | 14,09 | 15,76 | 9,11 |
| $k_M$ [mM] | 27,70 | 20,35 | 8,68 |
| $k_{cat}$ [1/s] | 45672 | 50970 | 29735 |
| $K_{cat}/k_m$ [1/mM*s] | 1649 | 2505 | 3427 |
| **Substrat DEP** | ***p*NB-Est17** | ***p*NB-Est19** | ***p*NB-Est13** |
| $V_{Max}$ [U/mg] | 9,57 | 7,20 | 3,21 |
| $k_M$ [mM] | 5,20 | 3,55 | 4,14 |
| $k_{cat}$ [1/s) | 31065 | 23265 | 10468 |
| $k_{cat}/k_M$ [1/mM*s] | 5974 | 6554 | 2528 |
| **Substrat DBP** | ***p*NB-Est17** | ***p*NB-Est19** | ***p*NB-Est13** |
| $V_{Max}$ [U/mg] | 48,03 | 40,69 | 2,87 |
| $k_M$ [mM] | 1,24 | 0,70 | 1,16 |
| $k_{cat}$ [1/s] | 155676 | 23265 | 9356 |
| $k_{cat}/k_M$ [1/mM*S] | 125545 | 187329 | 8066 |

**Hydrolyse von Phthalsäureester mit unterschiedlicher Kettenlänge (*para*)**

Tabelle 7: $k_M$- und $V_{max}$-Werte sowie $k_{cat}$ und $k_{cat}/k_m$ der Hydrolyse von Dimethylterephtalat (DMT) Diethylterephthalat (DET) durch *p*NB-Est17, *p*NB-Est19 und *p*NB-Est13.

| **Substrat DMT** | ***p*NB-Est17** | ***p*NB-Est19** | ***p*NB-Est13** |
|---|---|---|---|
| $V_{max}$ [U/mg] | 18,52 | 15,69 | 4,53 |
| $k_M$ [mM] | 5,99 | 13,56 | 6,87 |
| $k_{cat}$ [1/s] | 60028 | 50718 | 14784 |
| $k_{ca}/k_M$ [1/mM*s] | 10021 | 3741 | 2152 |
| **Substrat DET** | ***p*NB-Est17** | ***p*NB-Est19** | ***p*NB-Est13** |
| $V_{Max}$ [U/mg] | 28,06 | 30,79 | 3,57 |
| $k_M$ [mM] | 1,06 | 0,97 | 1,21 |
| $k_{cat}$ [1/S] | 90998 | 99411 | 12574 |
| $k_{cat}/k_M$ [1/mM*s] | 85847 | 102486 | 10392 |

Erläuterung der Abbildungen Abb. 1: Synthese von Bis-(*p*-methylbenzoesäure)-Ethylenglykolester durch Veresterung von 4-Methylbenzoesäure und Ethylenglykol Abb. 2: Schema der enzymatischen Hydrolyse von Bis-(*p*-methylbenzoesäure)-Ethylenglykolester Abb. 3: Nachweis der enzymatischen Hydrolyse und Identifizierung der Hydrolyseprodukte von Bis-(*p*-methylbenzoesäure)-Ethylenglykolester durch *para*-Nitrobenzyl-Esterasen Abb. 4: Schematische Darstellung der enzym-katalysierten Reaktionen von Dimethylphthalat, Diethyl-phthalat, Dimethylphthalat, Diethylphthalat, Dibutylphthalat, Dimethylisophthalat und Phenylbenzoat.

Abb 5: MICHAELIS-MENTEN-Kinetiken der *para*-Nitrobenzyl-Esterasen *p*NB-Est17 (o), *p*NB-Est19 (Δ) und *p*NB-Est13 (□) gegenüber Dimethylphthalsäure (DMP), Dimethylisophthalsäure (DMIP) und Dimethylterephthalsäure (DMT) Abb. 6: MICHAELIS-MENTEN-Kinetiken der *para*-Nitrobenzyl-Esterasen *p*NB-Est17 (o), *p*NB-Est19 (Δ) und *p*NB-Est13 (□) gegenüber ortho-Phthalsäureestern: Dimethylphthalsäure (DMP), Diethylphthalsäure (DEP) und Dibutylphthalsäure (DBP).

Abb. 7: MICHAELIS-MENTEN-Kinetiken der *para*-Nitrobenzyl-Esterasen *p*NB-Est17 (o), *p*NB-Est19 (Δ) und *p*NB-Est13 (□) gegenüber *para*-Pthalsäureestern: Dimethylterephtalat (DMT) und Diethylterephthalat (DET)

## Patentansprüche

1. Wasch- oder Reinigungsmittel, **dadurch gekennzeichnet, dass** es para-Nitrobenzylesterasen (PNB-Esterasen) enthält.

**2.** Mittel gemäß Anspruch 1, **dadurch gekennzeichnet, dass** die Esterase ausgewählt ist aus Esterasen mit einer Aminosäuresequenz, die zu einer der in SEQ ID NO. 1,2, 5, 6 oder 11-26, bevorzugt 1,2,5, 6 oder 12, angegebenen Aminosäuresequenzen mindestens zu 50 %, bevorzugt 60 %, inbesondere 70 %, bevorzugt mindestens zu 80 %, bevorzugt 90 %, insbesondere 95%, ganz besonders bevorzugt 100 % identisch oder zu mindestens zu 80%, bevorzugt 90 %, insbesondere 95%, ganz besonders bevorzugt 100 % homolog ist.

**3.** Mittel gemäß Anspruch 1 oder 2, **dadurch gekennzeichnet, dass** die Esterasen bei 30 °C eine spezifische Aktivität gegenüber dem Substrat Bis-(p-methylbenzoesäure)-ethylenglykolester von 0,1 bis 30, bevorzugt 0,6 bis 20, insbesondere 0,7 bis 15, ganz besonders bevorzugt 0,9 bis 10, noch stärker bevorzugt 1 bis 5, insbesondere 1,1 bis 4, ganz besonders bevorzugt 1,5 bis 3 μmol freigesetzte Säure/min*mg Enzym aufweisen.

**4.** Mittel gemäß einem der Ansprüche 1 bis 3, **dadurch gekennzeichnet, dass** die Esterasen aus Mikroorganismen, insbesondere aus Mikroorganismen der Gattung Bacillus stammen.

**5.** Mittel gemäß einem der Ansprüche 1 bis 4, **dadurch gekennzeichnet, dass** es mindestens ein oder mehrere Tenside enthält.

**6.** Mittel gemäß einem der Ansprüche 1 bis 5, **dadurch gekennzeichnet, dass** es zusätzlich weitere Enzyme, insbesondere Proteasen, Amylasen, Cellulasen, Hemicellulasen, Oxidoreduktasen und/oder Lipasen enthält.

**7.** Mittel gemäß einem der vorstehenden Ansprüche, **dadurch gekennzeichnet, dass** es eine oder mehrere para-Nitrobenzyl-Esterasen in einer Menge von 0,0001 μg bis 480 mg, vorzugsweise von 0,005 μg bis 420 mg, besonders bevorzugt von 0,02 μg bis 360 mg, ganz besonders bevorzugt von 0,050 μg bis 240 mg pro g des Mittels enthält.

**8.** Verwendung einer para-Nitrobenzyl-Esterase oder eines Mittels gemäß einem der Ansprüche 1 bis 7 zur Ausrüstung von Textilien.

**9.** Verwendung einer para-Nitrobenzyl-Esterase oder eines Mittels gemäß einem der Ansprüche 1 bis 7 zum Schutz von Fasern, insbesondere Kunstfasern, oder zur Verminderung von Pilling auf solchen.

**10.** Verwendung einer para-Nitrobenzyl-Esterase oder eines Mittels gemäß einem der Ansprüche 1 bis 7 zur Benetzung von Textilien vor der Färbung.

**11.** Verwendung einer para-Nitrobenzyl-Esterase oder eines Mittels gemäß einem der Ansprüche 1 bis 7 zur Spaltung bzw. zum Abbau von Kunststoffen, insbesondere Polyestern und/oder Weichmachern in Kunststoffen.

**12.** Verwendung einer para-Nitrobenzyl-Esterase oder eines Mittels gemäß einem der Ansprüche 1 bis 7 bei der chemischen Synthese von Polymeren, insbesondere Polyestern, bevorzugt Polyalkylenterephthalaten.

**13.** Verwendung einer para-Nitrobenzyl-Esterase und/oder eines Mittels gemäß einem der Ansprüche 1 bis 7 zur Reinigung von Textilien oder von harten Oberflächen, insbesondere in einer Menge von 0,04 μg bis 96 g, vorzugsweise von 0,05 μg bis 72 g, besonders bevorzugt von 1 μg bis 48 g und ganz besonders bevorzugt von 2 μg bis 24 g pro Anwendung.

**14.** Verwendung einer para-Nitrobenzyl-Esterase und/oder eines Mittels gemäß einem der Ansprüche 1 bis 7 zur Gewinnung oder Behandlung von Rohmaterialien oder Zwischenprodukten in der Textilherstellung, insbesondere zum Entfernen von Schutzschichten auf Geweben.

**15.** Verwendung einer para-Nitrobenzyl-Esterase, und/oder eines Mittels gemäß einem der Ansprüche 1 bis 7 zur Behandlung von Textilrohstoffen oder zur Textilpflege, insbesondere zur Behandlung von Kunstfasern oder kunstfaserhaltigen, insbesondere polyesterhaltigen Mischtextilien.

**16.** Verfahren zur maschinellen Reinigung von Textilien oder von harten Oberflächen, **dadurch gekennzeichnet, dass** in wenigstens einem der Verfahrensschritte eine para-Nitrobenzyl-Esterase und/oder ein Mittel gemäß Ansprüchen 1 bis 7 aktiv wird, insbesondere in einer Menge von 0,01 μg bis 96 g, vorzugsweise von 0,05 μg bis 72 g, besonders bevorzugt von 0,1 μg bis 48 g und ganz besonders bevorzugt von 0,5 μg bis 24 g pro Anwendung.

**17.** Verfahren zur Behandlung von Textilrohstoffen oder zur Textilpflege, **dadurch gekennzeichnet, dass** in wenigstens einem der Verfahrensschritte eine para-Nitrobenzyl-Esterase und/oder ein Mittel gemäß Ansprüchen 1 bis 7 aktiv wird, insbesondere für Textilrohstoffe, Fasern oder Textilien mit künstlichen Bestandteilen und ganz besonders für solche mit Kunstfasern, insbesondere Polyestern.

## Claims

**1.** A laundry detergent or cleaning agent **characterised in that** it comprises para-nitrobanzyl esterases (PNB-esterases).

**2.** The agent according to claim 1, **characterised in that** the esterase is selected from esterases with an amino acid sequence that is identical to one of the amino acid sequences listed in SEQ ID NO. 1, 2, 5, 6 or 11-26, preferably 1, 2, 5, 6 or 12, to at least 50 %, preferably 60 %, particularly 70 %, preferably to at least 80 %, preferably 90 %, particularly 95 %, quite particularly preferably 100 %, or is homologous to at least 80 %, preferably 90 %, particularly 95 %, quite particularly preferably 100%.

**3.** The agent according to claim 1 or 2, **characterised in that** the esterases exhibit a specific activity at 30 °C towards the substrate bis-(p-methylbenzoic acid) ethylene glycol ester of 0.1 to 30, preferably 0.6 to 20, particularly 0.7 to 15, quite particularly preferably 0.9 to 10, even more preferably 1 to 5, particularly 1.1 to 4, quite particularly preferably 1.5 to 3 μmol released acid/min*mg enzyme.

**4.** The agent according to one of claims 1 to 3, **characterised in that** the esterases originate from microorganisms, in particular from microorganisms of the genus Bacillus.

**5.** The agent according to one of claims 1 to 4, **characterised in that** it comprises at least one or more surfactants.

**6.** The agent according to one of claims 1 to 5 additionally **characterised in that** it comprises further enzymes, in particular proteases, amylases, cellulases, hemicellulases, further oxidoreductases and/or lipases.

**7.** The agent according to one of the previous claims, **characterised in that** it comprises one or more para-nitrobenzyl esterases in an amount of 0.0001 μg to 480 mg, preferably 0.005 μg to 420 mg, particularly preferably 0.02 μg to 360 mg, quite particularly preferably 0.050 μg to 240 mg per gram of the agent.

**8.** Use of a para-nitrobenzyl esterase or an agent according to one of claims 1 to 7 for the finishing of textiles.

**9.** Use of a para-nitrobenzyl esterase or an agent according to one of claims 1 to 7 for the protection of fibres, particularly synthetic fibres, or for the reduction of pilling on such fibres.

**10.** Use of a para-nitrobenzyl esterase or an agent according to one of claims 1 to 7 for wetting textiles prior to dyeing.

**11.** Use of a para-nitrobenzyl esterase or an agent according to one of claims 1 to 7 for the cleavage or for the degradation of plastics, particularly polyesters and/or plasticizers in plastics.

**12.** Use of a para-nitrobenzyl esterase or an agent according to one of claims 1 to 7 in the chemical synthesis of polymers, particularly polyesters, preferably polyalkylene terephthalates.

**13.** Use of a para-nitrobenzyl esterase, and/or an agent according to one of claims 1 to 7 for cleaning textiles or hard surfaces, in particular in a quantity of 0.04 μg to 96 g, advantageously 0.05 μg to 72 g, particularly preferably 1 μg to 48 g and quite particularly preferably 2 μg to 24 g per application.

**14.** Use of a para-nitrobenzyl esterase, and/or an agent according to one of claims 1 to 7 for the production or treatment of raw materials or intermediates in textile manufacture, in particular for the removal of protective layers on fabrics.

**15.** Use of para-nitrobenzyl esterase according to one of claims 1 to 7 for the treatment of textile raw materials or for fabric care, in particular for treating synthetic fibres or mixed textiles containing synthetic fibres, particularly mixed textiles containing polyester.

**16.** A process for the automatic cleaning of textiles or hard surfaces, **characterised in that** a para-nitrobenzyl esterase, and/or an agent according to one of claims 1 to 7 is active in at least one of the process steps, in particular in a quantity of 0.01 µg to 96 g, advantageously 0.05 µg to 72 g, particularly preferably 0.1 µg to 48 g and quite particularly preferably 0.5 µg to 24 g per application.

17. A process for the treatment of textile raw materials or for fabric care, **characterised in that** a para-nitrobenzyl esterase, and/or an agent according to one of claims 1 to 7 is active in at least one of the process steps, in particular for textile raw materials, fibres or textiles containing synthetic components and quite particularly for those containing synthetic fibres, particularly polyesters.

## Revendications

1. Agent de lavage ou de nettoyage, **caractérisé en ce qu'**il contient des para-nitrobenzylestérases (PNB-estérases).

2. Agent selon la revendication 1, **caractérisé en ce que** l'estérase est choisie parmi des estérases comprenant une séquence d'acides aminés qui présente une identité, par rapport à une des séquences d'acides aminés indiquée dans la SEQ ID NO: 1, 2, 5, 6 ou 11-26, de préférence 1, 2, 5, 6 ou 12, au moins à concurrence de 50 %, de préférence à concurrence de 60 %, en particulier à concurrence de 70 %, de préférence au moins à concurrence de 80 %, de manière préférée à concurrence de 90 %, en particulier à concurrence de 95 %, de manière tout particulièrement préférée à concurrence de 100 %, ou une homologie à concurrence d'au moins 80 %, de préférence à concurrence de 90 %, en particulier à concurrence de 95 %, de manière tout particulièrement préférée à concurrence de 100 %.

3. Agent selon la revendication 1 ou 2, **caractérisé en ce que** les estérases présentent, à 30 °C, une activité spécifique vis-à-vis de l'ester d'éthylèneglycol de l'acide bis-(p-méthylbenzoïque) faisant office de substrat, s'élevant de 0,1 à 30, de préférence de 0,6 à 20, en particulier de 0,7 à 15, de manière tout particulièrement préférée de 0,9 à 10, de manière encore plus préférée de 1 à 5, en particulier de 1,1 à 4, de manière tout particulièrement préférée de 1,5 à 3 µmol d'acide libéré/min*mg d'enzyme.

4. Agent selon l'une quelconque des revendications 1 à 3, **caractérisé en ce que** les estérases proviennent de micro-organismes, en particulier de micro-organismes du genre Bacillus.

5. Agent selon l'une quelconque des revendications 1 à 4, **caractérisé en ce qu'**il contient au moins un ou plusieurs agents tensioactifs.

6. Agent selon l'une quelconque des revendications 1 à 5, **caractérisé en ce qu'**il contient en outre des enzymes supplémentaires en particulier des protéases, des amylases, des cellulases, des hémicellulases, des oxydoréductases et/ou des lipases.

7. Agent selon l'une quelconque des revendications précédentes, **caractérisé en ce qu'**il contient une ou plusieurs para-nitrobenzylestérases en une quantité de 0,0001 µg à 480 mg, de préférence de 0,005 µg à 420 mg, de manière particulièrement préférée de 0,02 µg à 360 mg, de manière tout particulièrement préférée de 0,050 µg à 240 mg par g de l'agent.

8. Utilisation d'une para-nitrobenzylestérase ou d'un agent selon l'une quelconque des revendications 1 à 7, pour l'apprêtage de textiles.

9. Utilisation d'une para-nitrobenzylestérase ou d'un agent selon l'une quelconque des revendications 1 à 7, pour la protection de fibres, en particulier de fibres synthétiques, ou bien pour empêcher le boulochage sur ces dernières.

10. Utilisation d'une para-nitrobenzylestérase ou d'un agent selon l'une quelconque des revendications 1 à 7, pour le mouillage de textiles avant la teinture.

11. Utilisation d'une para-nitrobenzylestérase ou d'un agent selon l'une quelconque des revendications 1 à 7, pour la désintégration, respectivement la décomposition de matières synthétiques, en particulier de polyesters et/ou de plastifiants dans des matières synthétiques.

12. Utilisation d'une para-nitrobenzylestérase ou d'un agent selon l'une quelconque des revendications 1 à 7, lors de la synthèse chimique de polymères, en particulier de polyesters, de préférence de polyalkylènatétéphtalates.

**13.** Utilisation d'une para-nitrobenzylestérase ou d'un agent selon l'une quelconque des revendications 1 à 7, pour le nettoyage de textiles ou de surfaces dures, en particulier en une quantité de 0,04 µg à 96 g, de préférence de 0,05 µg à 72 g, de manière particulièrement préférée de 1 µg à 48 g, et de manière tout particulièrement préférée de 2 µg à 24 g, par application.

**14.** Utilisation d'une para-nitrobenzylestérase ou d'un agent selon l'une quelconque des revendications 1 à 7, pour l'obtention ou le traitement de matières premières ou de produits intermédiaires dans la fabrication de textiles, en particulier pour éliminer des couches de protection sur des tissus.

**15.** Utilisation d'une para-nitrobenzylestérase ou d'un agent selon l'une quelconque des revendications 1 à 7, pour le traitement de matières premières textiles ou pour le soin des textiles, en particulier pour le traitement de fibres synthétiques ou de textiles mixtes contenant des fibres synthétiques, en particulier contenant des polyesters.

**16.** Procédé pour le nettoyage mécanique de textiles ou de surfaces dures, **caractérisé en ce que**, dans au moins une des étapes opératoires, on active une para-nitrobenzylestérase et/ou un agent selon les revendications 1 à 7, en particulier en une quantité de 0,01 µg à 96 g, de préférence de 0,05 µg à 72 g, de manière particulièrement préférée de 0,1 µg à 48 g, et de manière tout particulièrement préférée de 0,5 µg à 24 g, par application.

**17.** Procédé pour le traitement de matières premières textiles ou pour le soin des textiles, **caractérisé en ce que**, dans au moins une des étapes opératoires, on active une para-nitrobenzylestérase et/ou un agent selon les revendications 1 à 7, en particulier pour des matières premières textiles, des fibres ou des textiles comprenant des constituants synthétiques, et tout particulièrement pour des textiles de ce type comprenant des fibres synthétiques, en particulier des polyesters.

Abb. 1:

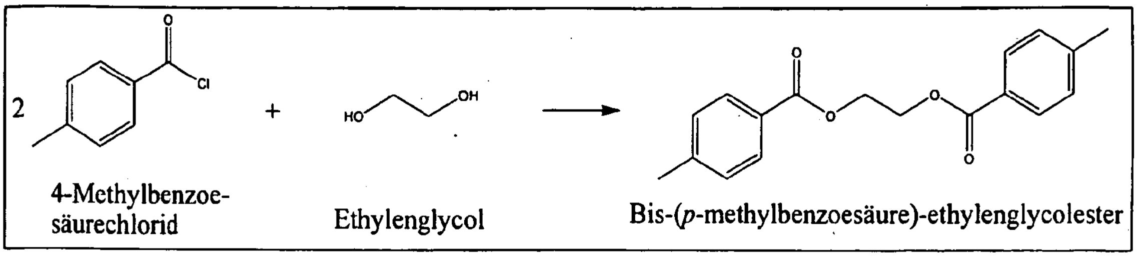

4-Methylbenzoesäurechlorid

Ethylenglycol

Bis-(*p*-methylbenzoesäure)-ethylenglycolester

Abb. 2:

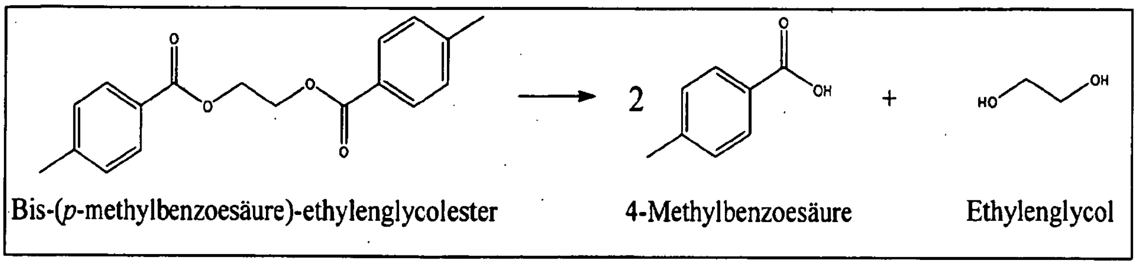

Bis-(*p*-methylbenzoesäure)-ethylenglycolester

4-Methylbenzoesäure

Ethylenglycol

Abb. 3:

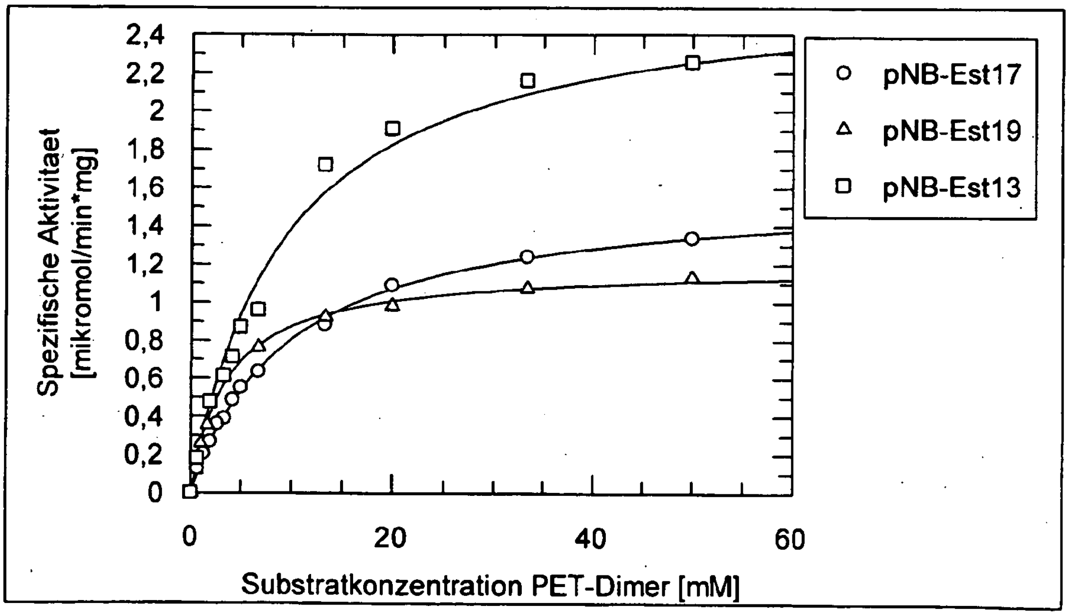

Abb. 4:

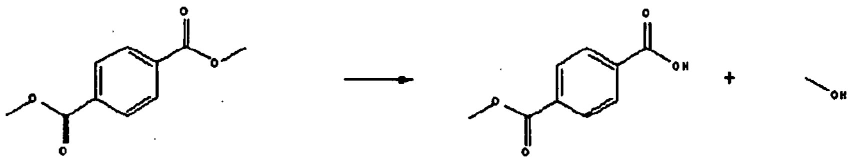

Dimethylterepthalat
Monomethylterepthalat
Methanol

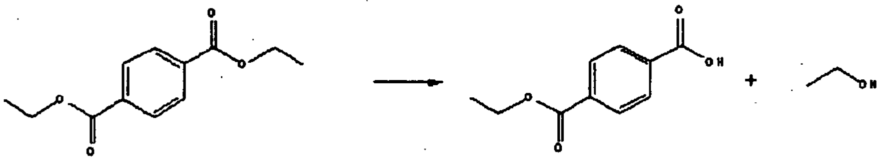

Diethylterepthalat
Monoethylterepthalat
Ethanol

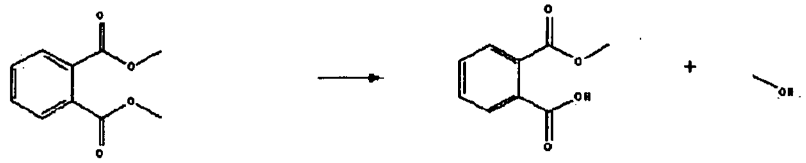

Dimethylpthalat
Monomethylpthalat
Methanol

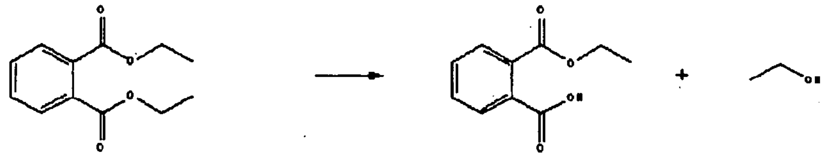

Diethylpthalat
Monoethylpthalat
Ethanol

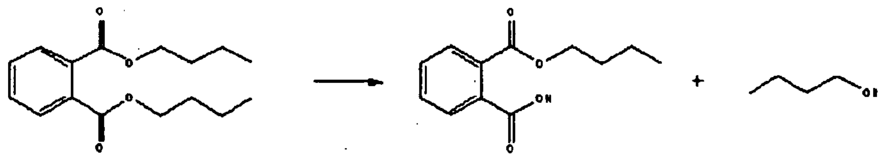

Dibuthylpthalat
Monobuylpthalat
Butanol

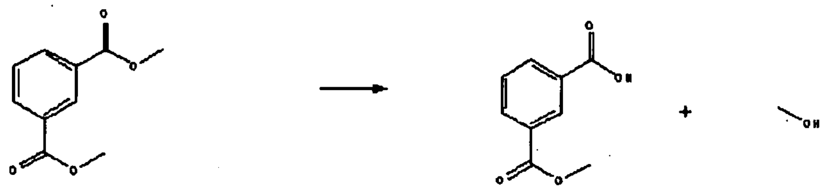

Dimethylisopthalat
Monomethylisopthalat
Methanol

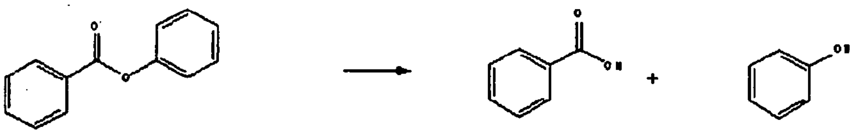

Phenylbenzoat
Benzoesäure
Phenol

Abb. 5(a):

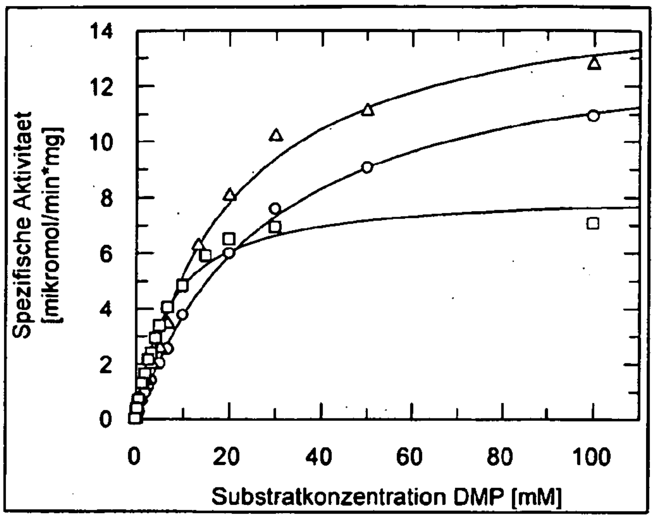


Abb. 5(b):

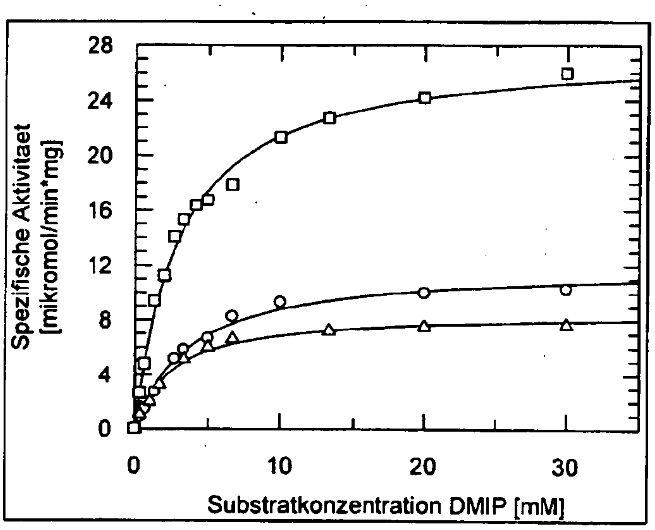


Abb. 5(c):

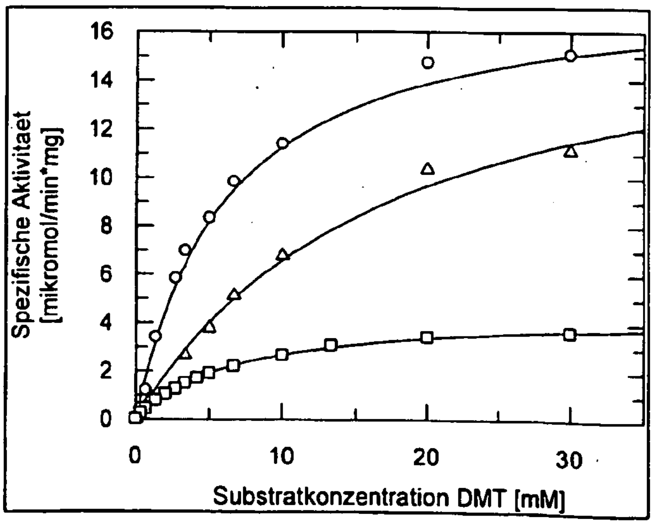

Abb. 6(a):

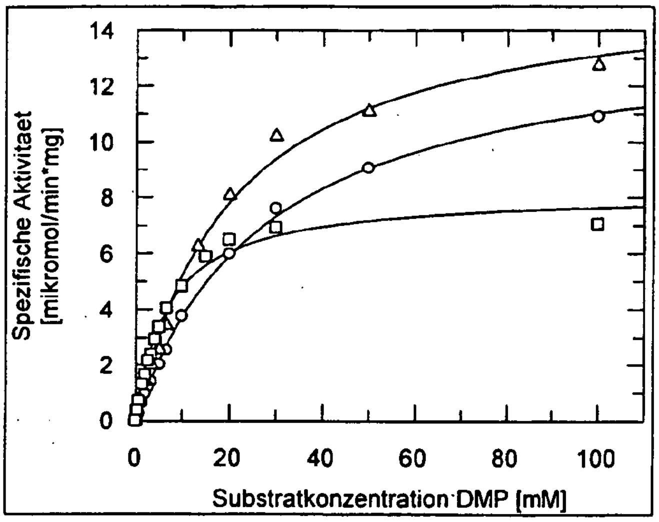


Abb. 6(b):

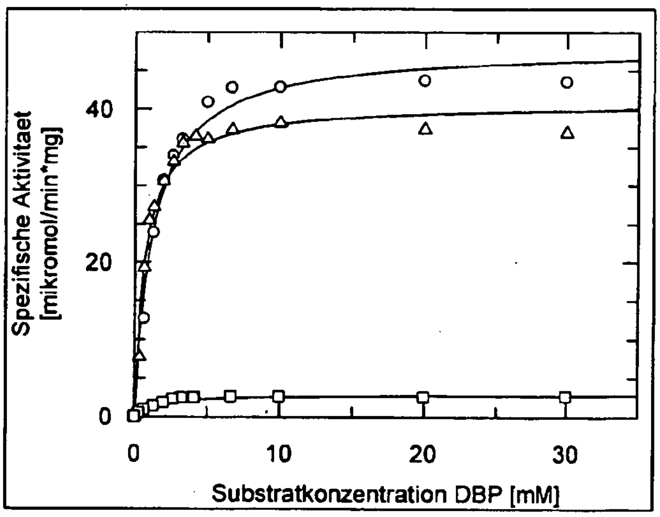


Abb. 6(c):

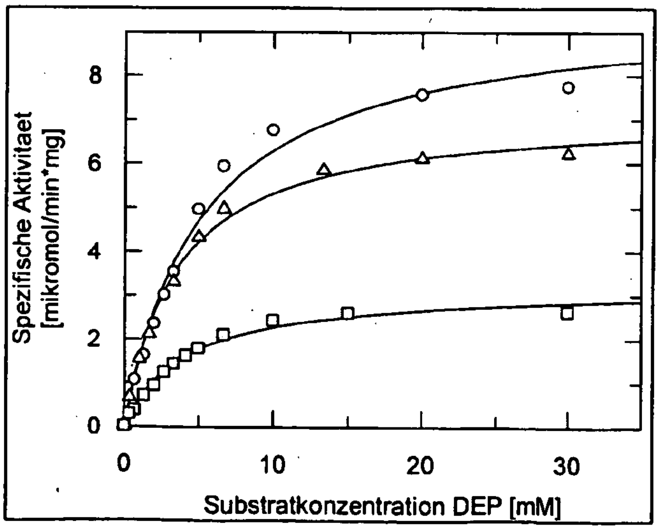

Abb. 7(a):

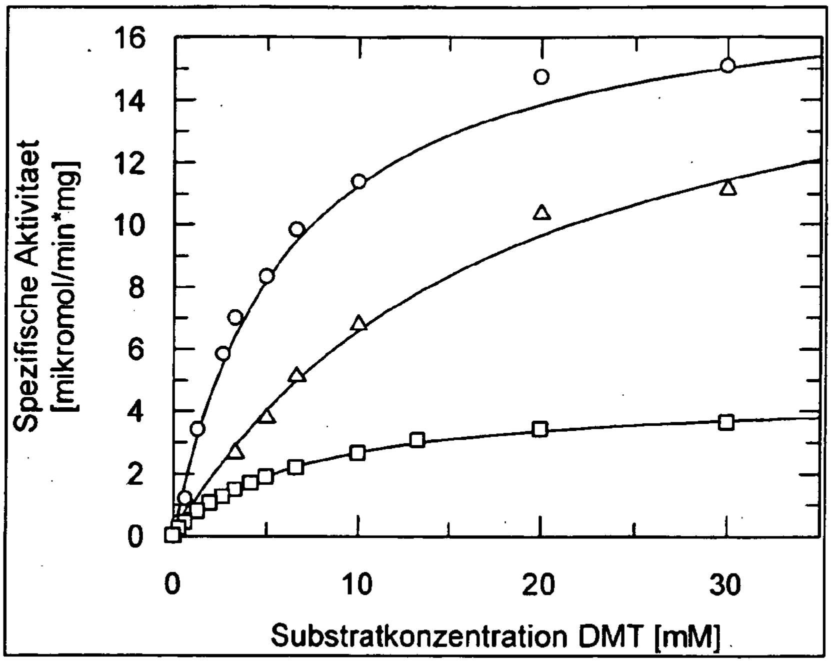
Spezifische Aktivitaet [mikromol/min*mg]
0
2
4
6
8
10
12
14
16
0
10
20
30
Substratkonzentration DMT [mM]

Abb. 7(b):

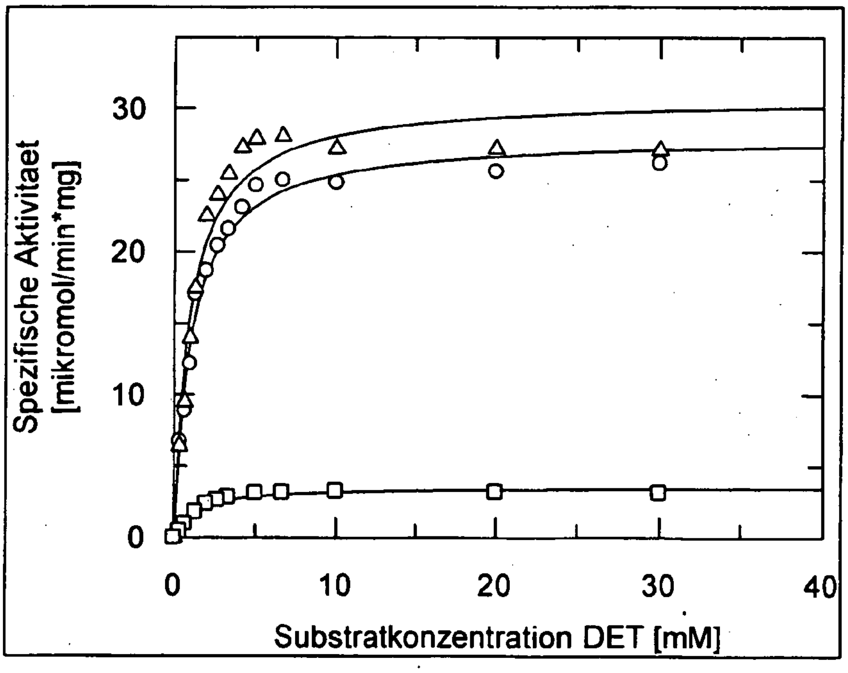
Spezifische Aktivitaet [mikromol/min*mg]
0
10
20
30
0
10
20
30
40
Substratkonzentration DET [mM]

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 5468632 A
- US 5906930 A
- US 5945325 A
- EP 0549264 A
- WO 0001831 A
- DE 102005037659


### In der Beschreibung aufgeführte Nicht-Patentliteratur


- **Arpigny, Jäger.** *Biochem. J.,* 1999, vol. 343, 177-183
- **D. J. Lipman ; W. R. Pearson.** *Science,* 1985, vol. 227, 1435-1441
- **Fritsch ; Sambrook ; Maniatis.** Molecular cloning: a laboratory manual. Cold Spring Harbour Laboratory Press, 1989